# EUROPEAN PATENT SPECIFICATION

(11) **EP 1 119 529 B1**
(45) Date of publication and mention of the grant of the patent: **17.09.2003**
(21) Application number: 99947750.8
(22) Date of filing: 05.10.1999
(51) Int. Cl.: C07B 61/00, C07D 239/38, C07C 271/20, C07C 311/18, C07C 237/18

(54) **CHEMICAL CONSTRUCTS**
CHEMISCHE KONSTRUKTIONEN
PRODUITS DE SYNTHESE CHIMIQUES

(30) Priority: 05.10.1998 GB 9821655
(43) Date of publication of application: 01.08.2001
(73) Proprietor: GLAXO GROUP LIMITED, Greenford, Middlesex UB6 ONN (GB)
(72) Inventor: CARR, Robin, Arthur, Ellis c/o Glaxo Wellcome plc, Stevenage Hertfordshire SG1 2NY (GB); GEHANNE, Sylvie Glaxo Wellcome SpA, I-37100 Verona (IT); KAY, Corinne c/o Glaxo Wellcome Chemistry Lab., Lensfield Road Cambridge CB2 1EW (GB); McKEOWN, Stephen, Carl Glaxo Wellcome plc, Stevenage Hertfordshire SG1 2NY (GB); MURRAY, Peter, John OSI Pharmaceuticals, Aston Science Park Birmingham B7 4EJ (GB); PAIO, Alfredo Glaxo Wellcome SpA, I-37100 Verona (IT); SCICINSKI, Jan c/o Glaxo Wellcome Chemistry Lab., Lensfield Road Cambridge CB2 1EW (GB); WATSON, Stephen, Paul Glaxo Wellcome plc, Stevenage Hertfordshire SG1 2NY (GB); WILLIAMS, G. c/o Glaxo Wellcome Chemistry Lab., Lensfield Road Cambridge CB2 1EW (GB); ZARAMELLA, Alessio Glaxo Wellcome SpA, I-37100 Verona (IT)
(74) Representative: Stott, Michael John
(86) International application number: GB9903286
(87) International publication number: WO00020357

(56) References cited:
- WO-A-95/28640
- WO-A-97/37953
- CARRASCO M R ET AL: "Direct Monitoring of Organic Reactions on Polymeric Supports" TETRAHEDRON LETTERS,NL,ELSEVIER SCIENCE PUBLISHERS, AMSTERDAM, vol. 38, no. 36, 8 September 1997 (1997-09-08), pages 6331-6334, XP004087929 ISSN: 0040-4039 cited in the application
- GEYSEN H M ET AL: "ISOTOPE OR MASS ENCODING OF COMBINATORIAL LIBRARIES" CHEMISTRY AND BIOLOGY,GB,CURRENT BIOLOGY, LONDON, vol. 3, no. 8, 1 August 1996 (1996-08-01), pages 679-688, XP002035873 ISSN: 1074-5521 cited in the application

## Description

### Field of the Invention

This invention relates to chemical constructs for use in solid phase synthesis, and to methods of detecting and/or characterising the products of solid phase synthesis using the constructs.

### Background of the Invention

Solid phase synthesis has been known for many years in the field of peptide synthesis and more recently has also been used increasingly for the synthesis of non-peptides.

Solid phase synthesis has found particular application in the field of combinatorial chemistry and the preparation of chemical libraries as potential sources of new leads for drug discovery, see for example Anthony W. Czarnik, *Analytical Chemistry News and Features*, June 1, 1998, pp 378A-386A, and *The Combinatorial Index*, Barry A. Bunin, Academic Press, San Diego 1998. A feature of combinatorial chemistry methods is that they enable very large numbers of different compounds to be prepared from a relatively limited number of molecular building blocks in a relatively small number of reactions. Combinatorial chemistry makes use of the "split and pool" approach in which a suspension of chemical starting material tethered to a solid support is split into N portions, each of which is reacted with a different reagent. The products of the N reactions are then pooled and mixed thoroughly, the resulting pool is split into N' portions and again each portion is reacted with a different reagent. This procedure can be repeated as many times as there are steps in the reaction sequence. Thus, for a three step reaction sequence, if the reaction mixture is divided into ten portions at each stage, each of the pools being reacted with a different reagent before recombining with the other portions, the total number of compounds formed by the process will be 10³ = 1000. Thus it can be seen that by using the split and pool technique, a large number of different molecules can be synthesised using a minimal number of reactions (thirty in the case mentioned above).

Each of the solid supports (e.g. a resin bead) will have a single product molecule tethered to it and hence in principle each of the reaction products can be separated and analysed or subjected to biological testing simply by isolating each single solid support and cleaving the product from the support. However, the large numbers of compounds generated by combinatorial methods means that it can be impracticable to identify and characterise each compound. Consequently, the compounds usually are first tested, either on the solid support or after cleaving from the support, and only those compounds, which show some biological activity, are subsequently identified. In order to minimise the number of biological tests carried out, compounds can be tested in pools containing a predetermined number of compounds, the inactive pools being discarded and the active pools being subject to further investigation. The biological activities of the compounds can be analysed using high throughput automated assay techniques permitting large numbers of compounds to be analysed in a short time.

The resin beads typically used in the synthesis of combinatorial libraries are derived from cross-linked polystyrene resin and are usually of the order of 90µm and 250µm in diameter (i.e. just visible to the naked eye). The number of beads in a given mass or volume of resin will depend on the average bead size but, for example, with a bead size of 150µm diameter, there will be approximately 500 beads per milligram of bulk resin. Typical compound loadings on the beads are of the order of 0.1 to 0.4 mmol g⁻¹ and hence beads of the dimensions given above will tend to have individual compound loadings of about 1 nanomole of compound per bead. It will be appreciated therefore that one of the problems facing the chemist working with solid phase constructs is how to identify and characterise the various compounds in a combinatorial library since each compound may be present in the library in very small amounts, and there will usually be insufficient compound present on a given solid support to allow for both biological testing and identification of the compound.

One known approach to the problem of how to identify the compounds in a combinatorial library is to provide each solid support with a coding tag from which the identity of the compound can be determined. Thus, for example, a coding tag can be built up in sequential steps on the solid support in parallel with the construction of the desired target compound, the coding tag reflecting the synthesis history of the product compound and being unique for each product compound. The coding tag is usually built up on the support using chemical reactions of a type which are orthogonal to the chemistry used to build up the product compound, thereby ensuring that the coding units and product compounds do not become confused. Once the product compound has been tested, and its biological activity confirmed, the coding tag can then be decoded to allow identification of the compound.

In an alternative approach, as described in Geysen *et al*., Chemistry & Biology, 1996, Vol. 3, No. 8, pp 679-688, and in WO 97/37953, coding tags such as isotopic labels can be incorporated into *inter alia* a linker group between the solid support and the substrate. This approach has the advantage that it does not require the formation of a separate coding tag using orthogonal chemistry and thereby reduces the overall number of synthesis steps involved.

A further problem facing the solid phase chemist is the difficulty in quantifying the products of a given reaction sequence. In any solid phase synthesis procedure, whether or not part of a combinatorial procedure, it is important to be able to determine the optimal conditions for a given reaction step in a series of steps. It is also important to be able to monitor the progress of a reaction so that it can be determined whether a particular reaction has gone to completion. This is particularly important in a multistage solid phase synthesis where the failure of a given stage to proceed to completion can lead to the formation of side products thereby complicating what is otherwise a relatively straightforward separation procedure. Where multiple products are formed in a given reaction step or sequence of steps, or where a given reaction step has not proceeded to completion, it can be extremely useful to have means of quantifying the reaction products, either in absolute terms, or in terms of the relative concentrations with respect to other reaction products. Where the products are produced in milligram quantities, quantitation can be carried out using a variety of instrumental techniques, for example nuclear magnetic resonance (nmr) spectroscopy. However, quantitation is much more difficult where the amount of product available for analysis may be as little as 1 nanomole.

Although both destructive and non-destructive analytical methods are known for determining the products of solid phase synthesis (see *The Combinatorial Index, idem),* one of the recognised problems with solid phase synthesis is that it is generally more difficult to monitor reactions carried out on solid supports than it is to monitor conventional solution phase reactions; see for example the Czarnik paper referred to above. This is particularly true with the products of combinatorial chemistry methods where there is a need to use rapid high throughput techniques to analyse the large numbers of compounds generated by such methods. Techniques such as mass spectrometry are potentially ideally suited as a means of providing high throughput analyses, but a substantial problem is that not all compounds produce a guaranteed response under mass spectrometric conditions. Indeed, many compounds are "invisible" to the so-called "soft" methods of mass spectrometry such as Matrix-Assisted Laser-Desorption Ionisation (MALDI) and Electrospray mass spectrometry which are intended to detect molecular ions without causing significant fragmentation of the molecule. In this context, the term "soft " means mass spectral methods that give molecular ions and not merely fragments. One of the reasons for this is that under MALDI and electrospray conditions, many compounds, particularly peptides, do not ionise sufficiently well to give a detectable mass spectral response.

*Geysen et al. (idem)* address this problem by suggesting the introduction of a readily ionisable basic centre such as lysine into the linker, and further suggest that the basic centre could be quaternised by alkylation during work up in order to give a charged group which would sensitise the construct to mass spectrometric analysis. However, one drawback of this approach is the need to protect the amine group during the various synthesis steps and the consequent need to deprotect the amine group prior to analysis.

A development of the Geysen approach is disclosed in Carrasco *et al. Tetrahedron Letters*, 1997, 38, No. 36, pp 6331-6334. Carrasco's method involves the formation of a construct comprising a resin bead linked via a first linker group to a group, which the authors refer to as an "ionisation tag". The "ionisation tag" in turn is linked via a second linker group to a substrate. The first and second linker groups are orthogonally cleavable; i.e. they can be selectively cleaved using different chemistries.

In the specific example disclosed in Carrasco *et al.,* the first linker group is photochemically cleavable whilst the second linker group is chemically cleavable. The "ionisation tag" used by Carrasco is a tetrapeptide chain having the sequence (reading from the C-terminus) Gly-Phe-Lys-Ala, and having an N-(2-trimethylammonium)-acetyl group linked to the lysine. The purpose of the ionisation tag is twofold. Firstly, it provides an already ionised "sensitiser group" which enables the construct to be detected by matrix-assisted laser-desorption ionisation (MALDI) mass spectrometry, and secondly, it adds mass to the construct thereby enabling substrate molecules to be detected without being swamped or masked by low molecular weight peaks in the mass spectrum.

However, there are several significant potential problems with the Carrasco constructs and in particular the "ionisation tag" used in the construct. More particularly, it is envisaged that the quaternary ammonium sensitiser group will be incompatible with many of the types of chemistry that the chemist might wish to perform on the constructs. Thus, for example, the trimethylammonium group is potentially chemically reactive and, furthermore, the cationic group may function as an ion exchange centre and complex with anions.

### Summary of the Invention

It is an object of the present invention to provide a means of monitoring chemical reactions on solid supports which avoids problems inherent in known methods and which provides a means of detecting and identifying the products of solid phase synthetic techniques using mass spectroscopy.

Accordingly, in a first aspect, the invention provides a chemical construct for use in solid phase synthesis comprising a solid support Q having linked thereto via a connecting group Y a substrate R; the connecting group Y having first and second cleavage sites which are orthogonally and selectively cleavable; the second cleavage site being selectively cleavable to release the substrate; and the first cleavage site being located at a position between the second cleavage site and the solid support and being selectively cleavable to release a fragment Fr comprising the substrate and at least a portion of the connecting group Y; characterised in that cleavage of the skeleton of the construct at the first cleavage site forms or introduces on the chemical fragment Fr at the first cleavage site a moiety comprising a sensitising group G which sensitises the chemical fragment Fr to instrumental, e.g. mass spectroscopic analysis.

In the constructs of the invention, the sensitising group G is formed or introduced by cleavage of the "skeleton" of the construct. This is in contrast with known constructs (see for example Carrasco *et al., idem*), where the sensitising group is present throughout the synthesis of the substrate, or where the sensitising group is revealed by cleavage of a side chain or removal of a protecting group from a pendant sensitising group.

The sensitising group G renders the fragment Fr, and hence indirectly the substrate R, more sensitive to analysis by a given analytical technique, and in particular a mass spectrometric technique. Thus the sensitising group can be a group which is readily ionisable under the conditions encountered in a mass spectrometer, and in particular electrospray mass spectrometry, to afford a strong signal.

The ionisable sensitising group formed during the selective cleavage from the solid support of the chemical fragment Fr serves to ensure that the fragment is ionised sufficiently in the mass spectrometer to give a strong response. This overcomes a problem inherent in many molecules synthesised by solid phase methods where a suitable ionising group is not present and analysis by high throughput mass spectral techniques is problematical.

The ionisable group can be for example a basic amino group or a carboxylate group but preferably it is a basic amino group. It will be appreciated that the term "basic amino group" as used herein refers in particular to an amino group that is readily protonated.

The basic amino group can be a primary amino group, a secondary amino group, or a tertiary amino group. Where the basic amino group is a tertiary amino group, it can be for example, a cyclic amino group such as piperidino, piperazino, pyrrolidino, or morpholino, piperidino or piperazino (e.g. N-methylpiperazino) being presently preferred.

In accordance with the invention, a moiety comprising the sensitising group G, such as a basic amino group, is formed or introduced at the first cleavage site as the chemical fragment Fr (hereinafter referred to as the analytical fragment) is cleaved from the solid support. The advantage of forming the sensitising group G in this way is that it avoids the potential problem of a pre-existing or pre-formed sensitising group interfering with the chemistry of the construct. Moreover, it avoids the need to have the sensitising group independently protected thereby obviating the additional problem of de-protection, a requirement which could limit the types of chemistry available to the chemist for cleavage at the first and second cleavage sites. Thus, for example, if a protected pendant sensitising group were present, and for example the conditions required for de-protection involved the use of an acid such as trifluoroacetic acid, then this would effectively prevent the use of an acid labile cleavage site as the second cleavage site linking the substrate to the rest of the construct. The constructs of the invention avoid such problems.

The atoms or functional group making up the sensitising group G can be present in a masked form in the construct before cleavage of the fragment Fr from the resin, the cleavage conditions merely serving to unmask the sensitising group G. Alternatively, the atoms or functional group making up or containing the sensitising group G can be introduced at the cleavage site during the cleavage reaction. For example, where the sensitising group is a basic amino group, the nitrogen atom of the amino group can be present in the construct before cleavage, or it can be introduced during the cleavage reaction.

When the sensitising group G is introduced from an external source during the cleavage reaction, it can form part of a larger chemical moiety. For example, the group G can be introduced as part of a group X-G wherein X is the residue of a nucleophile or electrophile, for example a nitrogen or sulphur-based nucleophilic group, e.g. a group of the formula G-Alk-Nuc wherein Alk is an alkylene group (e.g. a C₂₋₂₀ and preferably C₂₋₆ alkylene group) optionally interrupted by one or more heteroatoms selected from oxygen, nitrogen and sulphur, and Nuc is a nucleophile, such as an amine (e.g. NH or NR' where R' is a C₁₋₆ alkyl group) or a thiolate group., and Nuc is a nucleophile, such as an amine or a thiolate group.

It is preferred that the chemical fragment Fr contains a means for imparting a characteristic signature to the mass spectrum of the fragment. The signature can advantageously be provided by incorporating into the fragment a "peak splitting" isotopic label. The peak splitting isotopic label comprises at least one atom that exists in a number of stable isotopic forms. By isotopic labelling of one or more particular atoms in the fragment Fr such that a given atom is labelled with a mixture of isotopes, the mass spectra of the molecular ions will appear as a characteristic pattern, the precise pattern depending on the relative amounts of the individual isotopes. Thus, for example, if a given atom in the fragment Fr is labelled such that 50% of the atoms are of one isotopic form and 50% are of another isotopic form, the mass spectrum will show the molecular ion as a characteristic doublet in which the peaks are of approximately equal height.

The purpose of the peak splitting atom(s) is to provide a characteristic pattern that will characterise any peak in the mass spectrum originating from the analytical fragment Fr, thereby distinguishing such peaks from those due to extraneous materials.

Examples of atoms that can be used as isotopic peak splitting labels include ¹H/²H (D), ⁷⁹Br/⁸¹Br, ¹²C/¹³C, ¹⁴N/¹⁵N and ¹⁶O/¹⁸O.

The fragment Fr can contain a single peak splitting isotopic label or more than one such label. For example, the isotopic label can be a single bromine atom in which case the peak for the molecular ion of the analytical fragment Fr liberated following cleavage from the solid support will appear as a doublet. By introducing a second or subsequent peak splitting label(s), a more complex peak pattern will be produced for the molecular ion.

The isotopic peak splitting label(s) preferably is/are located between the first and second cleavage sites.

The first and second cleavage sites can be defined by first and second linker groups L¹ and L². A "spacer group" A can be interposed between the two linker groups L¹ and L², the spacer group A typically containing an isotopic peak splitting label. Accordingly, in one preferred embodiment of the invention, there is provided a chemical construct as hereinbefore defined wherein the connecting group Y has the formula L¹-A-L²; wherein L¹ is a first linker group defining the first cleavage site; A is a chemical group (the spacer group) containing a peak splitting isotopic label; and L² is a second linker group defining the second cleavage site.

The first and second cleavage sites are orthogonally and selectively cleavable; i.e. the conditions used to effect cleavage at one of the cleavage sites will not cleave the other. A wide variety of different types of cleavage reaction can be used, examples being reactions selected from acid catalysed cleavage, base catalysed cleavage, metal catalysed cleavage, oxidative cleavage, reductive cleavage, nucleophilic displacement, cleavage by 1,2 *bis* nucleophiles, electrophilic displacement, and thermal, photochemical and enzymatic cleavage.

Examples of linker groups that can be selectively cleaved under photochemical conditions to reveal or introduce a sensitising group G (e.g. an amine group) include carbamate groups such as o-nitrobenzylcarbamate groups in which initial cleavage takes place between the benzylic methylene group and the adjacent oxygen atom, followed by loss of carbon dioxide to give a basic amino group.

Examples of linker groups that can be cleaved by nucleophilic displacement (e.g. to reveal or introduce a sensitising group G) include sulphonamide linker groups in which the aryl (e.g. phenyl) ring of the sulphonamide contains an electron withdrawing group such as a nitro group, preferably in the *ortho* position of the aryl ring (see for example Kay *et al*. Tetrahedron Letters - 1997, 38(39)). Cleavage of the linker between the amino and sulphonyl moieties can be effected by a nucleophile such as a thiol or thiolate nucleophile in the presence of a base such as 1,8-diazobicyco[5.4.0]undec-7-ene (DBU), morpholine or sodium carbonate. Further examples of groups that can be cleaved by nucleophilic displacement include mercaptopyrimidine-based "safety catch" linkers such as 5-carboxy-2-mercaptopyrimidine, where cleavage can be effected by reacting under oxidising conditions to generate a sulphoxide or sulphone linkage, followed by reaction with a nucleophilic amino group to form a 2-aminopyrimidine. Such linkers can be cleaved, for example, by reaction with a cyclic amine such as piperidine or an N-substituted piperazine (e.g. N-methylpiperazine), or an amino group-containing thiolate nucleophile (e.g. dimethylaminoethylthiolate) after first oxidising with an oxidising agent, preferably a relatively mild oxidising agent such as a per-acid or an inorganic per-salt such as potassium peroxymonosulphate (e.g. "Oxone").

Either the first or second cleavage sites/linkers, or both, but preferably the first, can be of the "safety catch" variety; i.e. the cleavage site or linker group must be chemically modified in a first step before it can be subjected to cleavage in a second step (for example to reveal or introduce the sensitising group G). An advantage of such an arrangement is that it prevents or significantly reduces the possibility of cleavage taking place inadvertently. One example of a "safety catch" mechanism involves oxidation of a functional group (e.g. the mercaptopyrimidine-based "safety catch" linker described above) in a first step, the oxidation serving to make the functional group more amenable to displacement by a nucleophile in a subsequent cleavage step. The nucleophile can vary considerably in structure. For example, in one embodiment, the nucleophile can be an amino group-containing nucleophile), the amino group participating in the nucleophilic displacement action such that the amino group (sensitising group G) is attached directly to the cleavage site. Alternatively, in another embodiment, the amino group (or other sensitising group) can be present in a group (e.g. a dialkylaminoalkyl-thiolate anion) containing another nucleophile such as a sulphur nucleophile that becomes attached to the cleavage site.

Another class of linker groups suitable for use in the constructs of the present invention is the class of linker groups that are cleavable by 1,2 *bis* nucleophiles such as hydrazines and hydroxylamines. Such *bis* nucleophiles can be used to cleave the bond between the nitrogen atom and adjacent carbon atom in certain enamine systems, for example enamine systems in which the enamine is conjugated to a carbonyl group. Examples of such groups are groups based on oxo-substituted cycloalkylidene rings such as the 1-[4,4-dimethyl-2,6-dioxo-cyclohexylidene]-ethyl (dde) group coupled to an amino nitrogen atom. The constructs are preferably set up such that the enamine system defines the first cleavage site, breaking of the bond between the nitrogen atom and adjacent carbon atom as described above revealing an amine group that functions as a mass spectrometric sensitiser.

Either the first or second, but preferably the first, cleavage sites can be defined by linker groups that are cleavable under the action of transition metals, preferably palladium (0). Examples of such linker groups include allyloxy carbonyl amino groups which, when cleaved, release an amine group capable of acting as a mass spectrometric sensitiser.

Examples of linkers that can be selectively cleaved under acidic conditions include appropriately substituted benzyloxycarbonyl groups and appropriately substituted diphenyl-methylamino groups, both of which can be cleaved by the action of trifluoroacetic acid.

Particular examples of acid cleavable linker groups are set forth in *The Combinatorial* *Index*, Barry A. Bunin, Academic Press, San Diego 1998, the disclosure of which is incorporated herein by reference. Linkers of the "Rink" or "Knorr" type typically comprise an N-protected 1-amino-1,1-diphenylmethane moiety, the amino group when deprotected allowing attachment to a substrate, one of the phenyl rings being substituted for example with dimethoxy groups and the other having a carboxyalkyloxy substituent providing a second point of attachment. Cleavage with TFA gives rise to a substrate compound having a terminal carboxamido group. Linkers of the "Wang" type typically contain a substituted phenoxyacetyl group, the acetyl group providing one point of attachment, and a benzylic hydroxyl group on the phenyl ring forming a second point of attachment. Esters can be formed between a carboxyl group of a substrate and the benzylic hydroxyl group, the ester groups being subsequently cleavable with TFA to release a substrate compound having a terminal carboxylate group.

Examples of linkers that can be selectively cleaved under basic conditions include groups containing ester linkages.

In one embodiment, there is provided a chemical construct as hereinbefore defined wherein the first cleavage site is selectively cleavable to form or introduce a sensitising group G ( in particular an amino group) and the cleavage is effected by one type of chemistry selected from a group of chemistries consisting of cleavage under acid conditions, base catalysed cleavage, palladium (0) catalysed cleavage, oxidative cleavage, oxidation followed by nucleophilic displacement, reductive cleavage, nucleophilic displacement, cleavage by 1,2-*bis* nucleophiles, electrophilic displacement, and thermal, photochemical and enzymic cleavage, and the second cleavage site is selectively cleavable by a different type of chemistry selected from the said group.

Although groups that are cleavable under acidic conditions can be used to form the first cleavage site, it is currently preferred that the first cleavage site is stable to acidic conditions. However, the second cleavage site may advantageously be cleavable in acidic conditions.

In a preferred embodiment, the first cleavage site is cleavable to form or introduce the sensitising group G, the cleavage being effected by one type of chemistry selected from:
(i) photochemical cleavage, e.g. photochemical cleavage of a carbamate group such as a nitrobenzylcarbamate group;
(ii) oxidation followed by cleavage through nucleophilic displacement, for example oxidation of a thiopyrimidine followed by nucleophilic displacement by an amine (e.g. a secondary amine such as N-methyl piperazine);
(iii) cleavage of a sulphonamide by nucleophilic displacement, for example by a thiolate nucleophile (e.g. mercaptoethanol in the presence of a strong base such as DBU);
(iv) cleavage of enamine groups (particularly those containing an enamine moiety conjugated to a carbonyl group; e.g. as 1-[4,4-dimethyl-2,6-dioxo-cyclohexylidene]ethyl amino) with a 1,2-*bis* nucleophile such as hydrazine or hydroxylamine or derivatives thereof ; and
(v) transition metal catalysed cleavage of allyloxycarbonylamino groups, for example palladium (0) catalysed cleavage of allyloxycarbonylamino groups.

In the aforementioned preferred embodiment, the second cleavage site can be, for example, cleaved under acid conditions or by photolysis.

In one preferred embodiment, the first cleavage site is defined by a sulphonamide linker group, and the second cleavage site is optionally defined by a group, such as a Rink linker, which is cleavable under acidic conditions.

In another preferred embodiment, the first cleavage site is defined by a thiopyrimidine linker susceptible to cleavage by oxidation followed by nucleophilic displacement, and the second cleavage site is optionally defined by a group, such as a Rink linker, which is cleavable under acidic conditions.

In a further preferred embodiment, the first cleavage site is defined by a dde group as hereinbefore described, and the second cleavage site is optionally defined by a group, such as a Rink linker, which is cleavable under acidic conditions.

In a still further preferred embodiment, the first cleavage site is defined by a photochemically cleavable carbamate group as hereinbefore described, and the second cleavage site is optionally defined by a group, such as a Rink linker, which is cleavable under acidic conditions.

In yet another preferred embodiment, the first cleavage site is defined by a group such as allyloxycarbonylamino that can be cleaved by a transition metal such as palladium (0), and the second cleavage site is optionally defined by a group, such as a Rink linker, which is cleavable under acidic conditions

By making the first and second cleavage sites, e.g. as defined by the first and second linker groups L¹, L², orthogonally cleavable, it is possible selectively to separate from the construct either the chemical fragment Fr or the substrate R, simply by using different cleavage conditions. This means that during experiments designed to optimise the conditions for a particular reaction step, the chemist can subject the construct to conditions suitable for cleaving off the analytical fragment Fr thereby allowing analysis to be carried out to determine the outcome of each test reaction. Similarly, during a preparative reaction (e.g. preparation of a combinatorial library or subsequent preparative reactions such as scale-up reaction or commercial production), quality control (QC) can be carried out by removing one or more solid supports from the reaction vessel, cleaving the constructs at the first cleavage site and analysing the resulting fragment Fr to see whether a particular reaction step has worked. On the other hand, by cleaving at the second cleavage site e.g. on the second linker group, rather than the first, the reaction product R is released from the solid support. Thus, an advantage of the constructs of the present invention is that they can be used both at an experimental level to optimise a particular process step, and also at a preparative level without modifying the linker groups.

In one embodiment of the invention, the fragment Fr, and more preferably the spacer group A contains an alkylene diamine group or aminoalkoxy group. The precise size of the alkylene group and its degree of substitution is not currently considered to be important, but by way of example the chain could be from 2 to 30 carbon atoms in length, for example up to 20 carbon atoms, more typically less than 10 carbon atoms, particular examples being ethylene or propylene diamine or aminoalcohol groups which may be substituted or unsubstituted. The alkylene diamine group or aminoalcohol typically contains a peak splitting isotopic label as hereinbefore defined. The two amino groups can each be bonded to respectively the first and second linker groups. In order to increase the mass of the spacer group A, the alkylene diamine group can be substituted by an aryl group, or example an N-aryl group such as an N-benzyl group. The N-aryl group may optionally be substituted with one or more substituent groups.

Where, as is preferred, the spacer group contains one or more mass-spectral peak splitting isotopic labels, these can be located either in the alkylene chain or in a substituent group attached to the alkylene chain. Thus, for example, an N-benzyl group bonded to one of the two amino groups in an alkylenediamine can have a methylene group that is substituted with the peak splitting atom deuterium. Alternatively, an aryl ring (e.g. an N-benzyl group) present in a substituent on the alkylene diamine can be substituted with a peak splitting bromine atom, one particular example of an aryl group being an N-*o*-bromobenzyl group.

Although alkylene diamine and amino alcohol groups are given as specific examples of spacer groups, alternative spacer groups can be used. For example, the spacer groups can be formed from hydrocarbon chains containing up to thirty or more carbon atoms in the chain which can be optionally interrupted with one or more heteroatoms such as oxygen, nitrogen or sulphur. As a further alternative, the spacer can be for example a peptide chain containing one or more amino acids. The precise nature and length of the spacer is not currently considered to be important provided that the spacer does not interfere with the chemistry of the construct.

The chemical fragment Fr, and in particular the spacer group A, as well as providing a means of identification using mass spectrometry, can also be provided with one or more additional sensitisers to allow characterisation by other analytical techniques. For example, the spacer group can contain a chromophore to allow characterisation by ultra violet (u.v.) or fluorescence spectroscopy.

More particularly, in order to allow for quantitative analysis to be carried out to determine either the relative amount (e.g. relative to another substrate R) or absolute amount of substrate present in a reaction mixture, the connecting group Y and fragment Fr can contain a chromophore C^{u} which facilitates analysis of the fragment Fr by ultraviolet, visible or fluorescence spectrophotometry, and preferably by ultraviolet spectrophotometry.

Accordingly, in another aspect, the invention provides a method of analysing the fragment Fr of the present invention, which comprises subjecting the fragment Fr to ultra violet, visible or fluorescence spectrophotometric analysis to quantify the substrate R.

In accordance with the foregoing method aspect of the invention, the spectrophotometric analysis may be used to determine either the absolute amount of substrate R present in a given sample, or it may be used to determine the relative amount (e.g. in terms of a molar ratio) of substrate R present relative to another component (e.g. where more than one substrate R is present) in the sample. References to methods of quantifying the substrate R in this application include both absolute determination and relative determination, unless the context indicates otherwise.

In order to ensure that any chromophores present in the substrate R do not interfere with the analysis, the chromophore is typically selected such that it has a substantial absorption band that distinguishes it from the substrate. Thus, for example, the absorption band may be remote from any significant absorption bands in the substrate. Alternatively, the absorption band in the chromophore may be of such an intensity that it effectively swamps any overlapping band in the substrate. It is preferred that the chromophore C^{u} has a principal log Eₘₐₓ value of at least 2.5. It is further preferred that the principal log Eₘₐₓ value is at least 1.5 times greater than the principal log Eₘₐₓ of the substrate R.

The relationship between the absorption of visible or UV radiation by a compound in solution and the concentration of the compound is defined by the Beer-Lambert law which can be expressed by the formula *E* = *A*/*cl* where *A* is the absorbance or optical density of a sample solution, *c* is the molar concentration, *l* is the path length of the sample, and E is the molar absorptivity. The molar absorptivity E is constant for a given compound at a given wavelength and is usually expressed as Eₘₐₓ - the molar absorptivity at an absorption band maximum. The values for E or Eₘₐₓ are typically expressed in units of 10⁻² m² mol⁻¹ and, unless indicated otherwise, references to E or Eₘₐₓ values herein will refer to such units. Since E or Eₘₐₓ values can be very large, the logarithmic equivalent is often used and such logarithmic equivalents are referred to herein as log Eₘₐₓ values.

A given compound will often have significant absorption peaks or bands at several different wavelengths and hence can often be defined with reference to several Eₘₐₓ values. In the context of the present invention, the term "principal Eₘₐₓ " is used to denote the Eₘₐₓ at the wavelength at which the greatest absorbance occurs. Thus, in the constructs of the present invention, the chromophore C^{u} preferably has a principle log Eₘₐₓ value of at least 2.5 and typically has a principal log Eₘₐₓ value which is at least 1.5 times greater than the principal Eₘₐₓ of the substrate R. More typically however, the chromophore C^{u} has a principal log Eₘₐₓ that is at least 2, more usually 2.5, and preferably 3 times greater than the principal log Eₘₐₓ of the substrate R.

Although analysis of the compounds can be effected by measuring absorbance at a principal maximum, measurement of side bands or lesser maxima may be used instead to determine the amount of compound present. In general, the wavelength at which the measurement is taken will depend on the precise absorption characteristics of the substrates R and the chromophore C^{u}, and can be determined on a case by case basis.

The chromophore C^{u} may be distinguished from any chromophores in the substrate R in that it may possess an absorption band which is remote from any significant absorption bands. By this is meant that the absorption band of the chromophore is remote from, and does not overlap to any significant extent (e.g. less than 5% overlap with respect to the area under the curve (a.u.c.) of the respective absorption peaks) with any significant absorptions due to the substrate R. A significant absorption in the present context means an absorption having a log E value of 1 or more.

In one preferred embodiment of the invention, there is provided a chemical construct for use in solid phase synthesis comprising a solid support Q having linked thereto via a connecting group Y a substrate R wherein Q, Y and R are as hereinbefore defined; and the connecting group Y is orthogonally and selectively cleavable as hereinbefore defined; wherein the fragment Fr comprises the substrate and at least a portion of the connecting group Y, and the said portion contains a chromophore C^{u} which facilitates analysis of the fragment Fr^{u} by ultra violet, visible or fluorescence spectroscopy, the chromophore C^{u} having a principal log Eₘₐₓ value of at least 2.5 and wherein (i) the principal log Eₘₐₓ value is at least 1.5 times greater than the principal log Eₘₐₓ of the substrate R; or (ii), the chromophore C^{u} has an absorption peak at a wavelength remote from absorptions due to the substrate R.

The presence of a UV chromophore in the construct enables quantitation of the products of a synthesis to be carried out. Such quantitation can be (i) absolute quantitation or (ii) relative quantitation, or both. By absolute quantitation is meant that the absolute amounts of a substrate molecule or fragment or construct containing the substrate molecule can be determined, whereas the term " *relative quantitation*" is used herein to mean the determination of the amount of a substrate (or fragment or construct containing the substrate) relative to another component (such as a side product or starting material or another substrate) in a reaction mixture. The UV chromophore is typically selected such that it has a very strong absorption at a unique or characteristic wavelength, which is usually distinct from the wavelengths at which the maximum absorbances of a typical substrate molecule might be found. However, in cases where the absorption of the chromophore is very large relative to the substrate or substrate R, the overlapping of absorptions due to the substrate and the chromophore may have a minimal impact on the accuracy of the measurement of the quantity of substrate. In general, overlapping absorption bands should not prevent meaningful analyses to be carried out provided that any error introduced is no greater than 10% and preferably no greater than about 5%. Accordingly, as an alternative to defining the chromophore in terms of relative absorbances and absolute molar absorption values, the chromophore C, and its relationship with the absorption due to the substrate can be defined in terms of the degree of error arising from any overlap between the absorption bands of the substrate R and the chromophore.

Thus, in a further embodiment, the invention provides a chemical construct for use in solid phase synthesis comprising a solid support Q having linked thereto via a connecting group Y a substrate R wherein Q, Y and R are as hereinbefore defined; and the connecting group Y is orthogonally and selectively cleavable as hereinbefore defined; wherein the fragment Fr comprises the substrate and at least a portion of the connecting group Y, and the said portion contains a chromophore C^{u} which facilitates analysis of the fragment Fr^{u} by ultra violet, visible or fluorescence spectroscopy, wherein the absorption characteristics of the chromophore C" and the substrate R are such that at a given measurement wavelength, any errors in measurement of the quantity of substrate R (or any fragment or construct containing the fragment) arising from any overlap between absorption bands due to the chromophore and absorption bands due to the substrate R are less than 10%, preferably less than 5%.

Examples of chromophores are groups containing an aryl group, preferably a fused polycyclic aryl group, e.g. a C₆ - C₃₀ polycyclic aryl group in which one or more (e.g. 1, 2, or 3) ring carbon atoms are optionally replaced by a heteroatom such as nitrogen, sulphur or oxygen. Examples of polycyclic aryl groups are polycyclic hydrocarbons such as naphthyl, phenanthrenyl and anthracenyl groups, and polycyclic heteroaryl groups such as acridine, or phenanthroline. Such aryl groups or polycylclic groups can be optionally substituted, preferably with a substituent or substituents which is or are inert and/or non-interfering with regard to the reaction conditions employed in a given reaction scheme.

Examples of such substituent groups include alkyl and alkoxy (e.g. alkyl and alkoxy having from 1 to 20 carbon atoms, preferably from 1 to 6 carbons), each optionally containing one or more unsaturated linkages, and being optionally interrupted by one or more heteroatoms selected from oxygen, nitrogen and sulphur, amino (preferably protected amino or mono-or disubstituted amino, e.g. dimethylamino); halogen such as fluoro, chloro, bromo and iodo; alkylthio (e.g. C₁₋₂₀ alkylthio, preferably C₁₋₆), hydroxy, protected (e.g. acylated) hydroxy, thio, protected (e.g. acylated) thio, trifluoromethyl, nitro, alkylsulphonyl, aryl (e.g. optionally substituted phenyl, thienyl, furanyl), and arylalkyl (e.g. benzyl and phenethyl).

Other examples of chromophores include highly conjugated aryl or non-aryl systems, provided that such compounds are inert under the synthetic conditions employed in the solid phase chemistry, and so-called "push-pull" systems of the type G-J-M wherein G is an electron-donating group (e.g. amino or alkoxy), J is an conjugated array of multiple, e.g. double, bonds, and M is an electron withdrawing group (e.g. nitro or sulphonyl), the groups G and M being electronically linked through the conjugated array of multiple bonds. One example of such as chromophore is the dansyl (1-dimethylamino-5-naphthylsulphonyl) group.

The precise nature of the chromophore, its chemical properties and its absorbance characteristics, can be determined on a case by case basis depending upon the type of chemistry to be used in a given synthesis. It is most preferred however, that the chromophore is inert to the reactants and reaction conditions employed in the synthesis.

Presently preferred chromophores include anthracenyl and dansyl (5-dimethylamino-1-naphthylsulphonyl groups, anthracenyl being particularly preferred.

The quantitation of the substrate R can be either absolute quantitation or relative quantitation in which the relative amounts of the substrate R and another component in the synthesis mixture (e.g. another substrate, or starting material, or a side product for example) are determined. Relative quantitation makes use of the fact that the chromophore C^{u} confers on each substrate a common set of characteristic absorbances that can be used as the basis for the spectrophotometric analysis.

In one embodiment of the invention, the characterisation and/or analysis of the constructs of the invention is carried out on a single bead, or a small number of beads, for example from 1 to 20 beads, more usually less than ten beads. Such beads typically have an average diameter in the range from 90µm to 250µm and a compound loading of between 0.1 mmol g⁻¹ and 0.4 mmol g⁻¹ average loading of an individual bead is less than 10 nanomoles, often less than 5 nanomoles, for example approximately 1 nanomole.

A significant advantage of the use of the UV chromophore-containing constructs of the present invention is that they can be used to provide quantitative information on very small amounts of substrate compounds, for example amounts of the order of 1 nanomolar typically available from single resin beads of the size used in split and mix combinatorial methods. In combinatorial libraries formed by the split and mix method, each bead typically will contain only a single substrate compound, but a library will contain a plurality of beads bearing different substrates. The analysis of mixtures of beads bearing different substrates is problematical and hence it is generally necessary to carry out analyses on single beads. The constructs of the present invention enable analysis to carried out efficiently and accurately at the single bead level, and in particular with beads of a size of the order of 90µm to 250µm in diameter with loadings of the order of 0.1-0.4 mmol g-¹.

In constructs where the first and second cleavage sites are defined by first and second linker groups L¹ and L², and a "spacer group" A is interposed between the two linker groups L¹ and L², the spacer group A can contain or have linked thereto the UV chromophore C^{u}. Thus, in one embodiment of the invention, there is provided a chemical construct as hereinbefore defined wherein the connecting group Y has the formula L¹-A-L²; wherein L¹ is a first linker group defining the first cleavage site; A is a chemical group (the spacer group) containing or having linked thereto the UV chromophore C^{u}, and optionally containing a peak splitting isotopic label; and L² is a second linker group defining the second cleavage site.

The chromophore C^{u} may be formed as an integral part of the backbone or skeleton of the fragment Fr^{u} (e.g. as part of the spacer group A), or it can be a pendant group. Thus, for example, when it is a pendant group, it can be linked to A either directly or via an alkylene chain (e.g. of 1 to 6 carbon atoms) optionally interrupted by one or more oxygen, nitrogen or sulphur atoms, or by means of an ether, thioether, amide, sulphonamide or ester linkage. Where the spacer group A contains an alkylene diamine group or aminoalkoxy group, the chromophore C^{u} can, for example, be linked (directly or via an optionally interrupted alkylene chain as hereinbefore defined) to the nitrogen atom of the, or one the, amino groups. For example, the chromophore C^{u} can be linked to the nitrogen atom by means of an ethylene or propylene chain or by a sulphonyl group.

In compounds where a substituent such as a benzyl group is attached to one of the amino groups of an alkylene diamine spacer group A, the chromophore C^{u} can be linked, directly or indirectly, to the other of the amino groups. In one currently preferred embodiment, the chromophore C^{u} is a polycyclic aryl group such as an anthracenyl or phenanthrenyl group linked via a propylene chain to a nitrogen atom at one end of the diamine spacer group.

The solid support Q can be any type of solid support suitable for use in solid phase synthesis, and in particular combinatorial chemistry. Thus, purely by way of example, the solid support can take the form of beads, a solid surface, solid substrates, particles, pellets, discs, capillaries, hollow fibres, needles, solid fibres, or organic or inorganic gels such as silica gels, and insoluble organic particles such as particles formed from fullerenes.

Examples of beads are polymeric beads such as cellulose beads or resin beads, particular examples of materials from which resin beads can be prepared including functionalised polymer resins such as polystyrene resins, polyacrylamide resins and dimethylacrylamide resins. Examples of suitable supports are listed in *The Combinatorial Index* by Barry A. Bunin, referred to above. Examples of functionalised resins include cross-linked polystyrene resins modified to provide amino groups or hydroxyl groups at the surfaces thereof: see for example *Combinatorial Chemistry* by Nicholas K. Terrett, Oxford University Press, 1998.

In order to enable to simplify determination of the synthesis history of a solid support, and to provide a means of controlling the mixing of solid supports in split-and-pool combinatorial syntheses, the solid supports (e.g. groups of beads) can be confined within permeable labelled containers throughout a reaction sequence, whereby synthesis takes place as a result of the reactants and solvents flowing through the permeations in the container. A given reaction mixture may contain a plurality of containers and, at the end of each reaction step, the containers can be removed and identified by the label which can be, for example, a radio frequency label. Examples of such containers are the polypropylene mesh "Kan" ™ containers available from Irori of La Jolla, California, USA which contain miniaturised radio frequency tags.

In another aspect, the invention provides a method of analysing the constructs hereinbefore defined; the method comprising cleaving the construct at the first cleavage site to release the chemical fragment Fr, the cleavage reaction generating on the chemical fragment Fr a mass spectrometric sensitising group (e.g. a group which is ionisable under mass spectroscopic conditions), and then subjecting the chemical fragment to mass spectrometry, e.g. electrospray mass spectrometry.

The analysis of the fragment Fr provides information on the reaction history of the construct. Thus, by mass spectrometric analysis, it can readily be determined whether or not the desired substrate has been formed in a given reaction sequence. Analysis of the fragment Fr can therefore be used not only to characterise the substrate or product of the solid phase reaction sequence, but also to follow the progress of the reactions.

The invention also provides a method of analysing the constructs hereinbefore defined; the method comprising cleaving the construct at the first cleavage site to release the chemical fragment F^{u} and subjecting the fragment to UV, visible or fluorescence spectrometric analysis, for example quantitative UV analysis.

The cleavage products can be subjected to chromatography, preferably a liquid chromatography technique such as HPLC, using a UV detector which can be set up to provide a means of determining concentrations of the various compounds as they elute from the chromatography column. The chromatography column can in turn be linked to another instrumental means of analysis for the purpose of identifying the substrate. A preferred means of instrumental analysis is mass spectroscopic analysis, and thus the method and constructs of the invention afford a means of means of achieving both identification and quantitation of the substrate in a single linked series of analytical procedures

In a further aspect, the invention provides an intermediate chemical construct for use preparing a chemical construct as hereinbefore defined, the intermediate construct having the formula Q-Y' wherein Y' is a reactive or protected form of the group Y, and Q and Y are as hereinbefore defined.

in a still further aspect, the invention provides an intermediate construct of the formula Q-L¹-A^{p} wherein Q and L¹ are as hereinbefore defined and A^{p} is a reactive or protected form of the spacer group A containing a splitting isotopic label and optionally a. chromophore C^{u}. In a particular embodiment, the intermediate construct has the general formula Q-L¹-N(Alk - C^{u})-Alk-NH-X¹ wherein Alk is an alkylene group and X¹ is hydrogen or an aralkyl group. The intermediate construct is preferably isotopically labelled with a peak splitting combination of atoms such as ¹H/H² (D), ⁷⁹Br/⁸¹Br, ¹²C/¹³C, ¹⁴N/¹⁵N and ¹⁶O/¹⁸O.

The invention further provides a method of identifying a pharmaceutically useful substrate comprising preparing a library containing a plurality of chemical constructs as hereinbefore defined and subjecting the library to biological testing to identify biologically active substrates. The method may include the further step of formulating a biologically active substrate thus identified with a pharmaceutically acceptable carrier to form a pharmaceutical composition.

### Brief Description of the Drawings

Figure 1 shows the mass spectrum of the photolysis product of the construct described in Example 1;
Figure 2 shows the mass spectrum of a fragment produced by cleavage of the thiopyrimidine linker group in the construct described in Example 2;

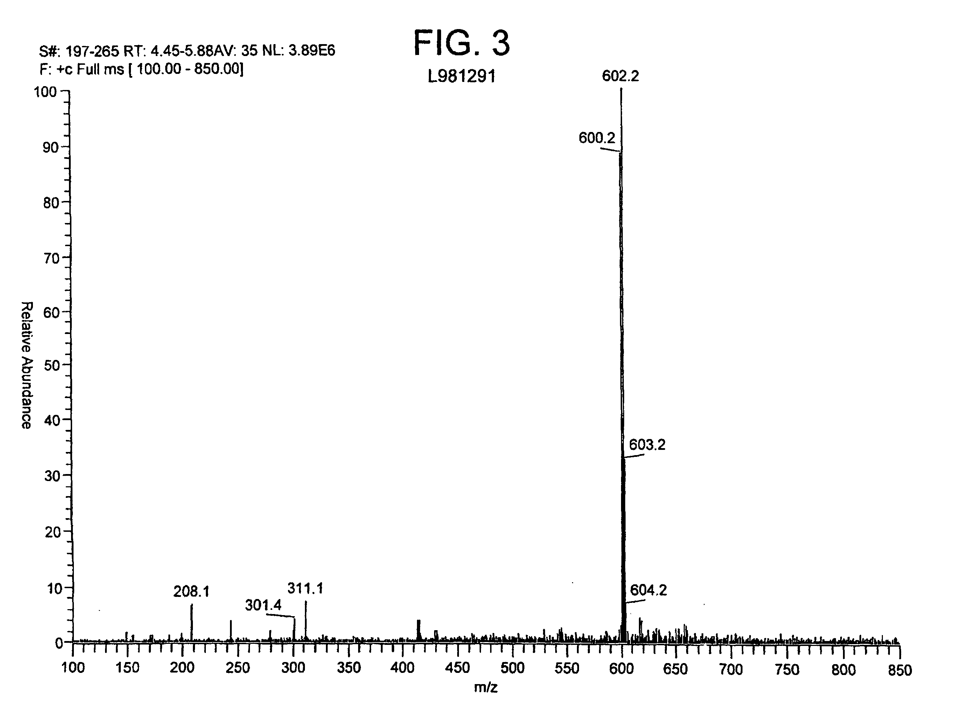

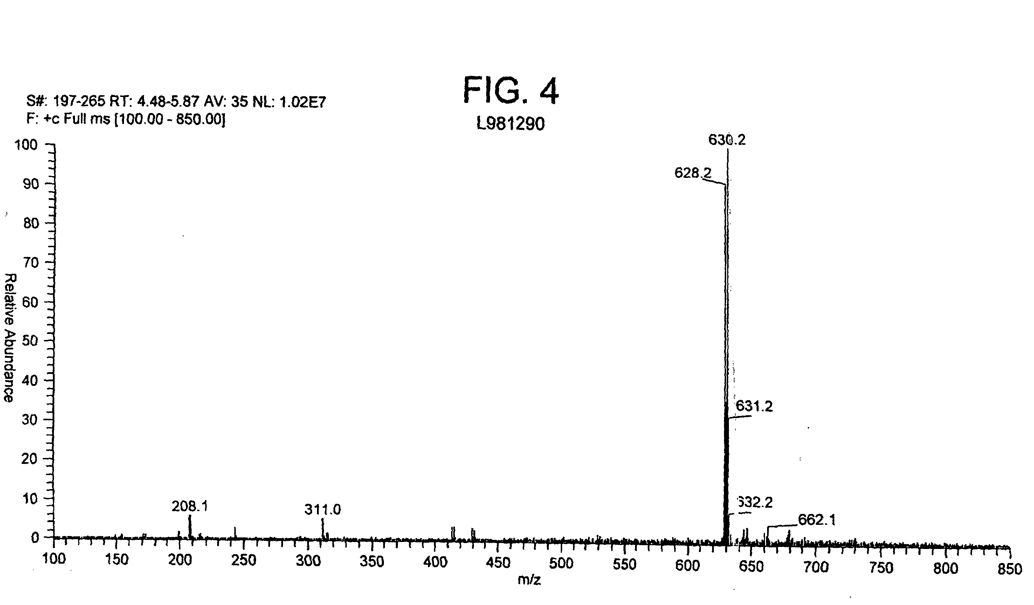

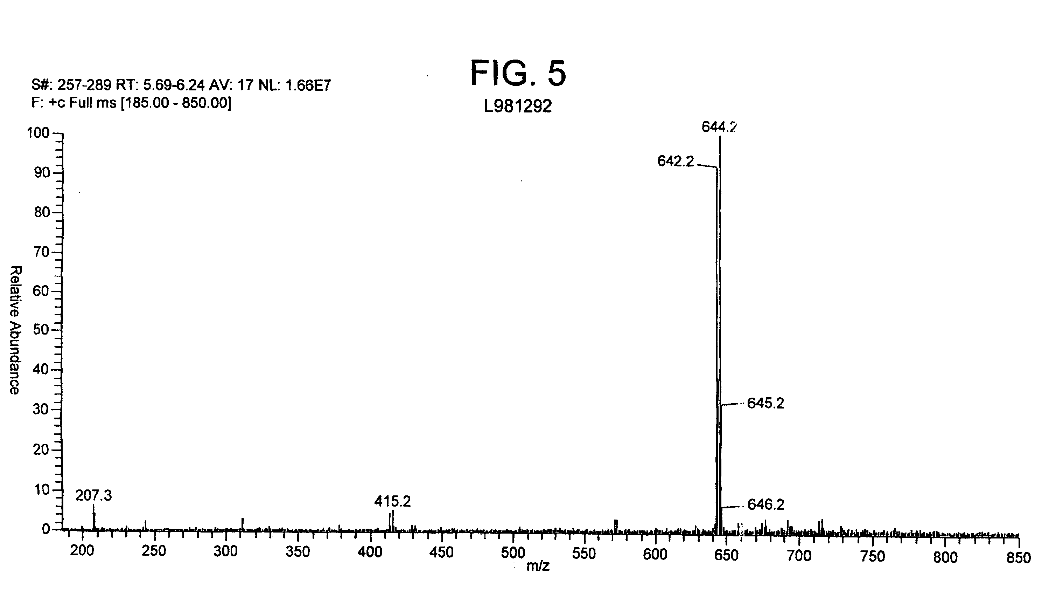

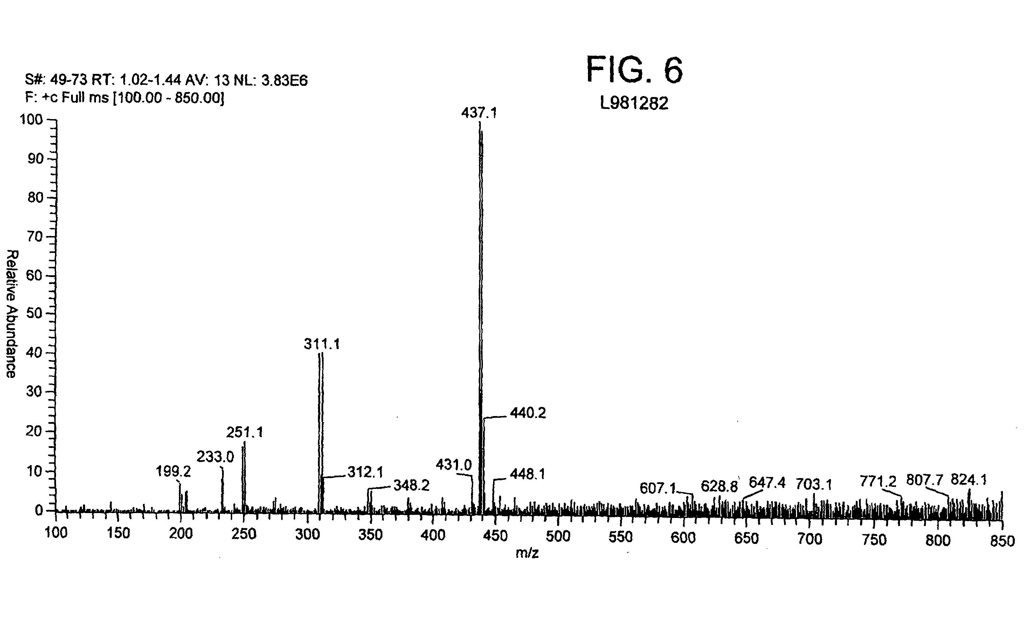

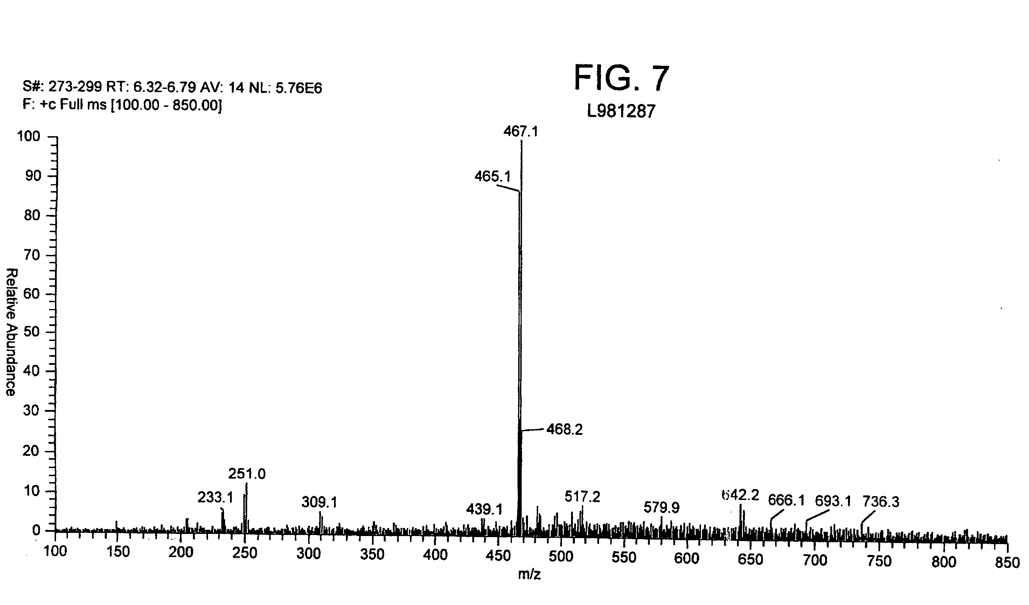

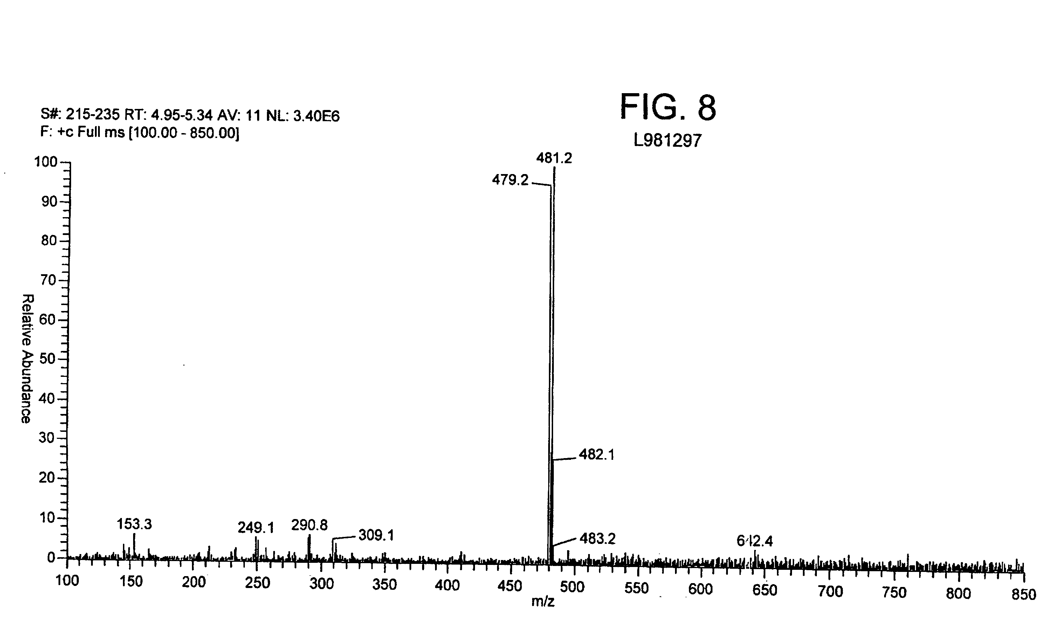

Figures 3 to 8 show the mass spectra of the photolysis products of the constructs described in Example 4;

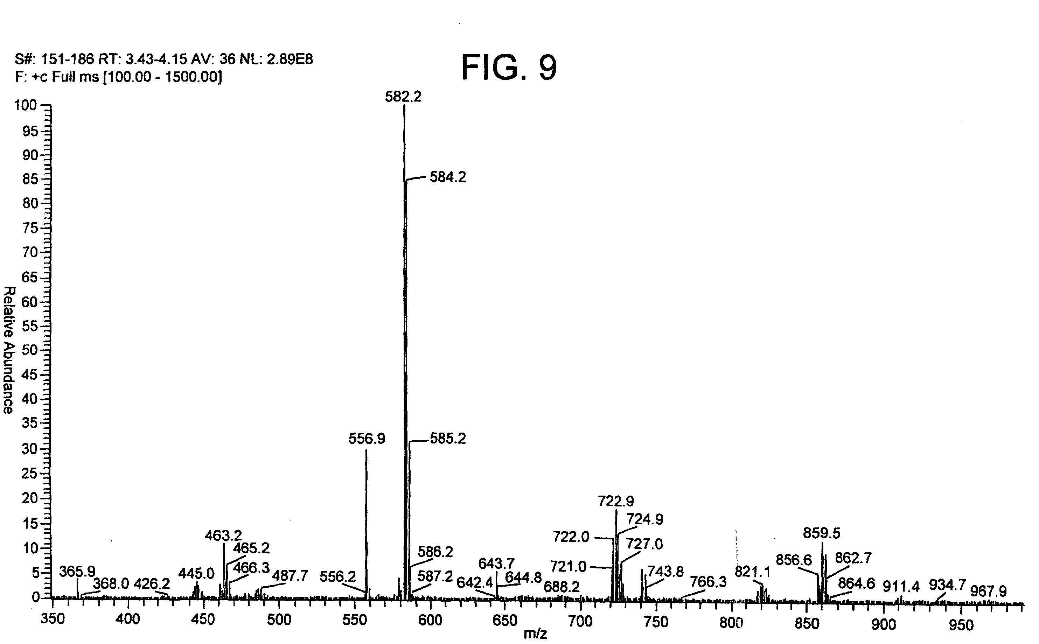

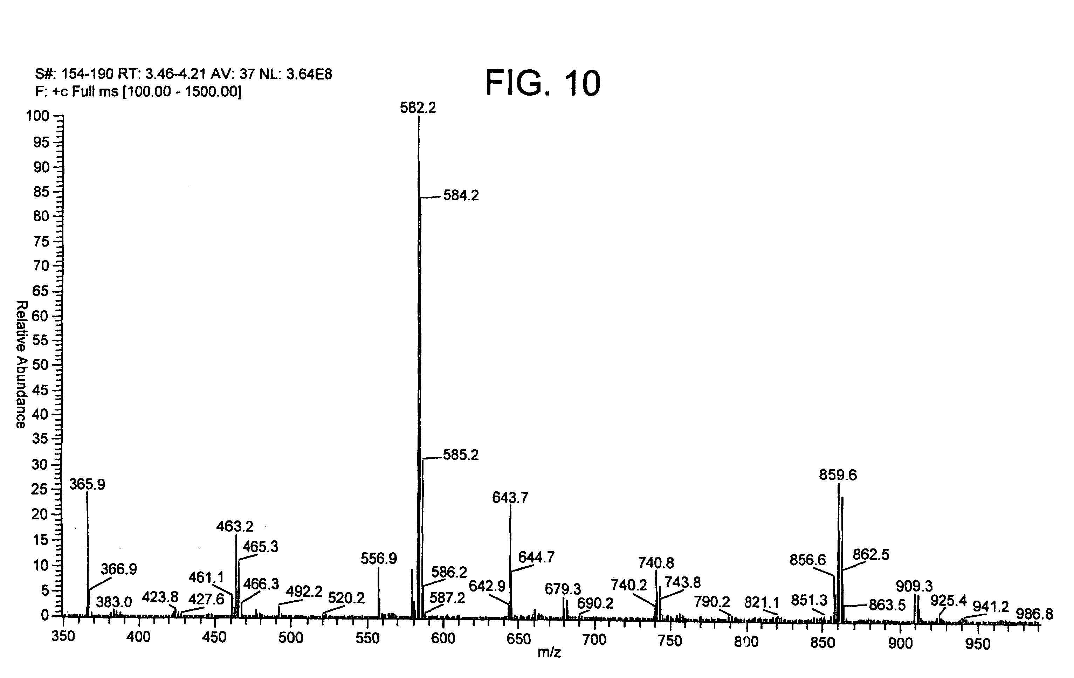

Figures 9 and 10 show the mass spectra of analytical fragments produced by palladium (0) cleavage of the "Alloc" - linker - containing constructs of Example 7; and
Figures 11 to 18 show the comparative mass spectra of either a substrate, or an analytical fragment containing the substrate, following cleavage at either the first or second cleavage sites for four of the members of the combinatorial library described in Example 6.

### Description of Illustrative Embodiments

The invention will now be illustrated but not limited by reference to the following examples.

In the Examples, the following abbreviations are used.

### Abbreviations

- AcOH: Acetic acid
- DMF: Dimethylformamide
- DCM: Dichloromethane
- DMAP: 4-Dimethylaminopyridine
- DIC: Diisopropylcarbodiimide
- TFA: Trifluoroacetic acid
- DIPEA: Diisopropylethylamine
- HATU: 2-(1H-9-Azabenzotriazole-1-yl)-1,1,3,3-tetramethyluronium hexafluorophosphate
- DMSO: Dimethylsulfoxide
- PyBOP®: Benzotriazole-1-yl-oxy-tris-pyrrolidino-phosphoniumhexafluorophosphate
- HOBT: N-Hydroxybenzotriazole
- AA: Amino acid

### EXAMPLE 1

### Preparation of a Construct containing a Photolytically Cleavable First Cleavage Site

A photocleavable construct was prepared following the reaction scheme shown in Scheme 1. In this example, the photolytic cleavage of a substituted nitrobenzyloxycarbonyl linker group attached to one amino group of an ethylene diamine chain liberates the free amino group so that it is capable of undergoing ionisation in a mass spectrometer thereby enabling a characteristic molecular ion to be formed. In this example, the ethylene diamine connecting group has an isotopically labelled benzyl group attached to the other amino group, the effect of which is to provide a characteristic split peak in the mass spectrum of the cleavage product to assist identification.

The compound numbers used in the Experimental for Example 1 refer to the compound numbers in Scheme 1. 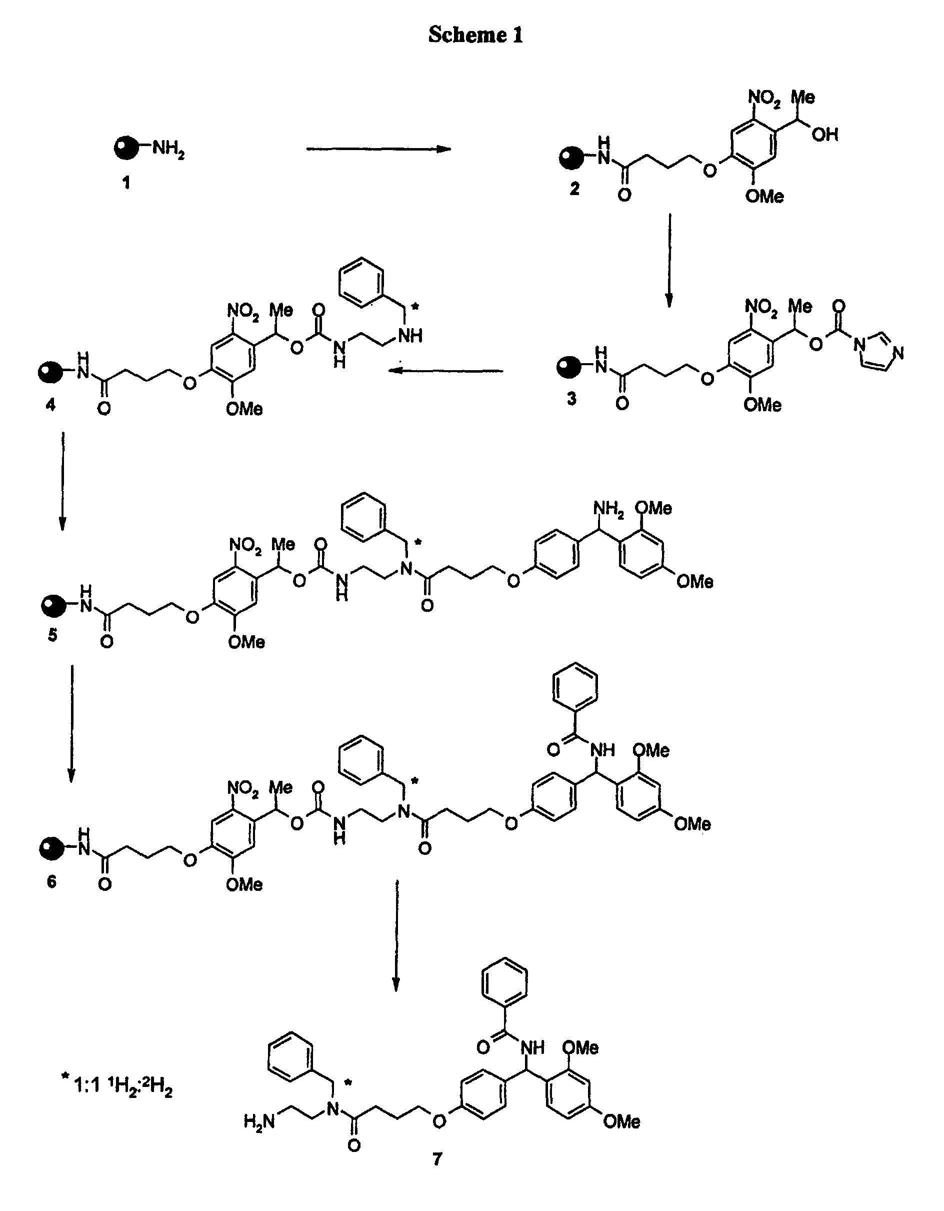 >

### Experimental For Scheme 1

TentaGel NH₂ (Rapp Polymere) (Resin 1) (30 g, 12.0 mmol), having a bead diameter in the range 130 µm to 160 µm and a loading of 0.4 mmol g⁻¹, was swelled with DMF and to this was added a solution of HOBT (9.3 g, 69.0 mmol), DIC (6.48 ml, 41.4 mmol) and 4-[4-(1-Hydroxyethyl)-2-methoxy-5-nitrophenoxy) butanoic acid (11.8 g, 39.4 mmol) in DMF (420 ml) and the mixture shaken for 16 h. The resin was then drained and washed with DMF followed by MeOH then DCM and finally MeOH. This was then dried *in vacuo* to give resin **2**.

Resin **2** (3 g, 11.9 mmol) was treated with a solution of carbonyldiimidazole (19.3 g, 119 mmol) in DCM (400 ml) and the whole heated at 50 °C with slow stirring for 5 h. The resin was then drained and washed with DCM followed by Et₂O, DCM, Et₂O and dried *in vacuo* to give resin **3**.

Resin **3** (15 g, 4.35 mmol) was swelled with DMF (200 ml) and to this was added a solution of l-benzyl-1-*tert*-butoxycarbonyl-1,2-diaminoethane (**17**) (2.71 g, 10.9 mmol) and 1-(dideuterobenzyl)-1-*tert*-butoxycarbonyl-1,2-diaminoethane (**16**) (2.71 g, 10.9 mmol) in DMF (20 ml) and the whole heated at 50 °C with slow stirring for 16 h. The resin was drained and washed with DMF followed by Et₂O, DCM, and Et₂O. The resin was then dried *in vacuo*. The resin was then treated with a 1:4 solution of aq.TFA (95%) and DCM and shaken for 30 min. The resin was then drained and washed with DCM followed by Et₂O, DCM, Et₂O and the resin was dried *in vacuo*. The resin was then shaken with 10% solution of DIPEA in DMF for 1h and then drained, the washing was then repeated as above to give resin **4**.

A solution of HATU (0.96 g,2.52 mmol), DIPEA (0.89 ml, 5.04 mmol) and p-[(R,S)-α-[1-(9H-fluoren-9-yl)-methoxyformamido]-2,4-dimethoxy-benzyl]-phenoxy-butanoic acid (1.42 g, 2.52 mmol) in DMF (30 ml) was added to Resin **4** (3 g, 0.84 mmol) and the whole shaken for 16 h. The resin was then drained and washed with DMF, and DCM. A solution of 20% piperidine in DMF (30 ml) was added to the resin and the whole shaken for 1h. The resin was then drained and washed with DMF, DCM followed by Et₂O, DCM and Et₂O and the resin was dried *in vacuo*.

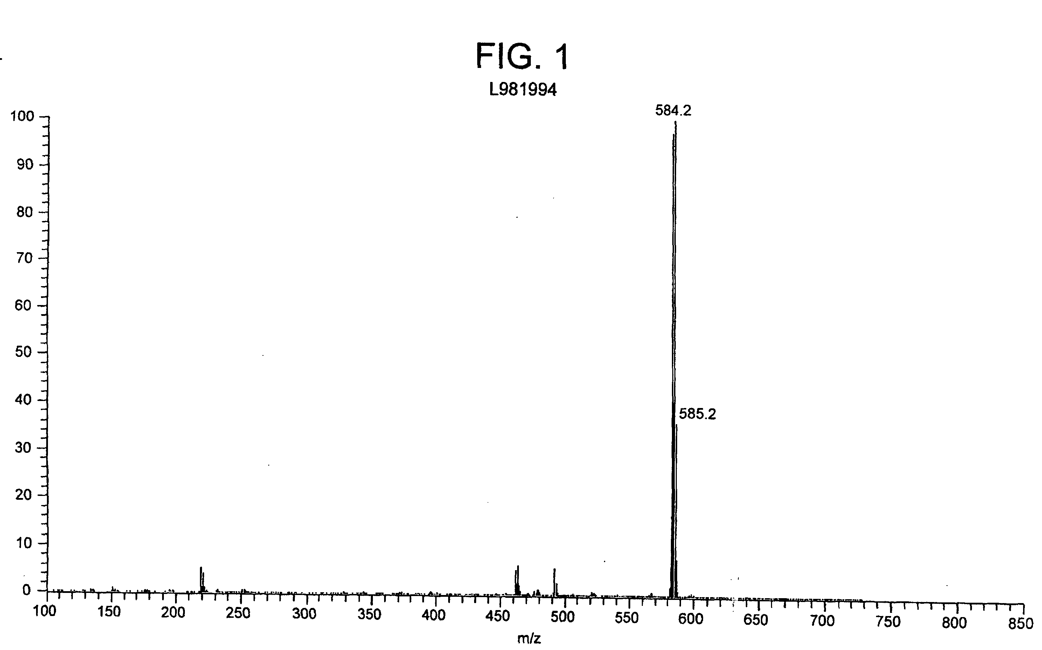

Resin **5** (20 mg, 0.004 mmol) was treated with a solution of HATU (15 mg, 0.04 mmol), DIPEA (14 µl, 0.08 mmol) and benzoic acid (5 mg, 0.04 mmol) in DMF (1 ml) and the whole was shaken for 6 h. The resin was then drained and washed with DMF, DCM, Et₂O, DCM and Et₂O and dried *in vacuo* to give resin **6**. Construct analysis of this resin by photocleavage followed by MS analysis is shown in figure **1** (see general cleavage conditions).

As can be seen from Figure 1, the presence of the sensitising amine group and peak splitter group in the analytical fragment cleaved from the resin to demonstrate a strong molecular ion with a characteristic doublet pattern.

### General method of Construct Analysis of Resins via the photocleavable linker.

A small sample of the resin (ca. 0.5 mg) is suspended in DMSO (50 µl) and exposed to UV light for 30 min. The solution is then analysed by mass spectrometry. (Alternatively DMSO containing 0.2% hydrazine hydrate can be used as the solvent).

The mass spectra were obtained on a Finnegan MAT LCQ ion trap Mass Spectrometer operating in positive electrospray mode. Scan range 100 -1500 a.m.u. Scan cycle time 1.4 seconds; ion time 200 microseconds

### EXAMPLE 2

### Preparation of a Construct Containing a "Safety Catch" Thiopyrimidine Linker Group at the First Cleavage Site

A construct containing a thiopyrimidine "Safety Catch" linker at the first cleavage site was prepared according to the reaction scheme shown in Scheme 2 below. The construct numbers used in the following experimental description refer to the numbers in Scheme 2.

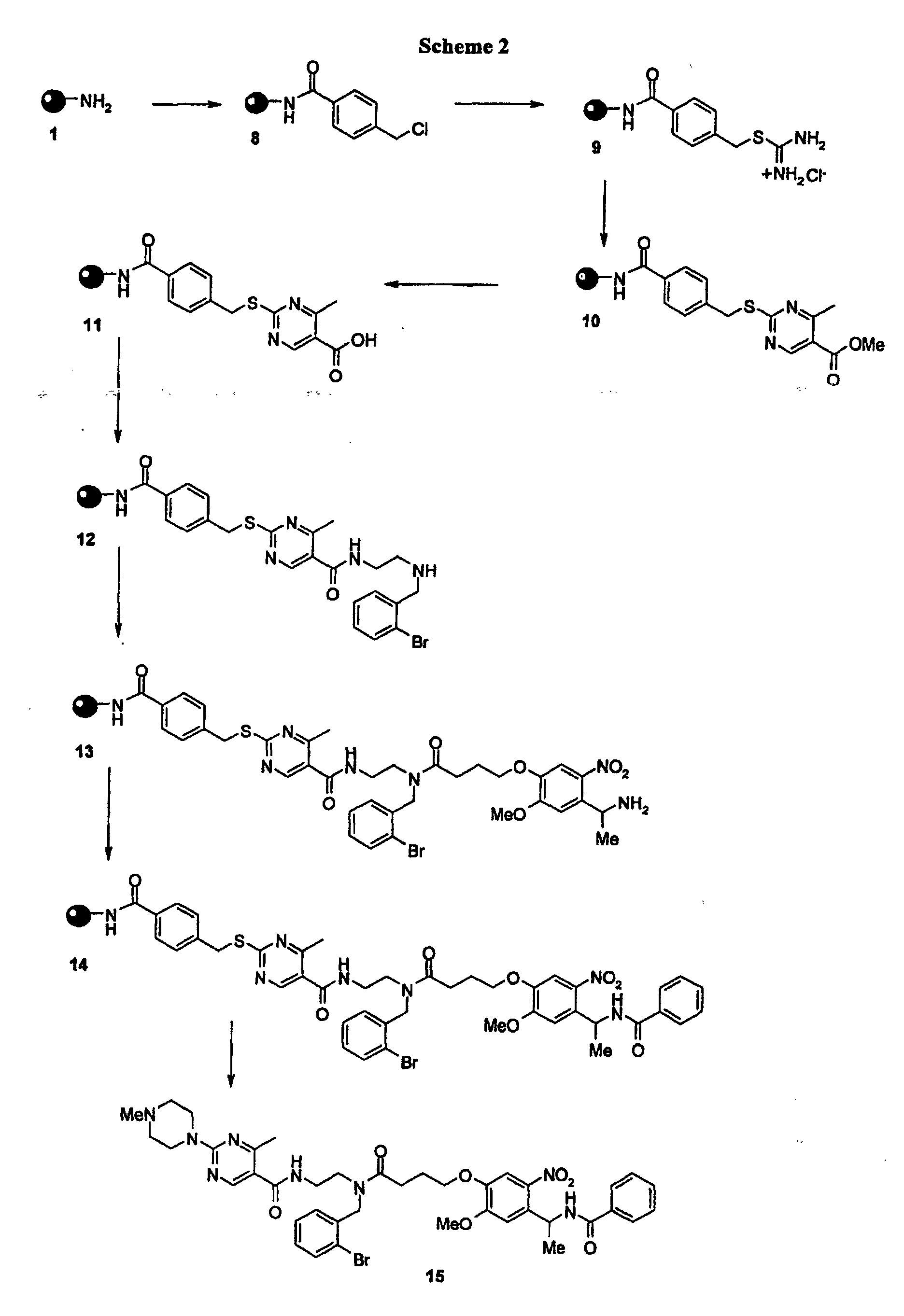

### Experimental for Scheme 2

A solution of 4-(chloromethyl)benzoic acid (0.27 g, 1.6 mmol), PyBOP® (0.83 g, 1.6 mmol) and DIPEA (0.56 ml, 3.2 mmol) in DMF (8 ml) was shaken for 20 min then this solution was added to TentaGel NH2 (Rapp Polymere) resin **1** (1 g, 0.32 mmol)), having a bead diameter in the range 130 µm to 160µm and a loading of 0.32 mmol g⁻¹, and the whole shaken for 5h. The resin was then drained and washed with DMF, DCM, Et₂O, DCM and finally Et₂O. The resin (**8**) was then dried *in vacuo*.

A slurry of thiourea (0.58 g, 7.7 mmol) in dioxane (4 ml) and ethanol (1 ml) was added to Resin **8** (1 g, 0.32 mmol) and the whole heated at 80 °C for 16h. The resin was drained and washed with DMF (x 5), DCM (x 5), Et₂O, DCM and finally Et₂O to give resin **9.** The resin was then dried *in vacuo.*

A solution of methyl-2-(dimethylaminomethylene)-3-oxobutanoate (0.17 g, 0.96 mmol) and triethylamine (65 µl, 0.48 mmol) in DMF (10 ml) was added to resin **9** (1 g, 0.31 mmol) and the whole heated at 80 °C for 16h. The resin was drained and washed with DMF, DCM, Et₂O, DCM, and finally Et₂O. The resin (**10**) was then dried *in vacuo.*

Resin **10** (0.5 g, 0.15 mmol) was then treated with a 1:1 mixture of THF and 2N aq.NaOH (4 ml) and shaken for 2h. The resin was then drained and washed with THF/H₂O, 10% AcOH/THF, THF/H₂O, THF, DMF, DCM, Et₂O, DCM, Et₂O and then dried *in vacuo* to give resin **11**.

A solution of PyBOP® (24 mg, 0.045 mmol) and DIPEA (16 µl, 0.09 mmol) in DMF (1 ml) was added to resin **11** (50 mg, 0.015 mmol) followed by a solution of 1*-tert*-butoxycarbonyl-1-(*o*-bromobenzyl)-diaminoethane (**18**) (15 mg, 0.045 mmol) in DMF (1 ml) and the whole shaken for 3 days. The resin was drained and washed with DMF, DCM, Et₂O, DCM, and finally Et₂O. A solution of 95% aq.TFA in DCM (20%, 1 ml) was then added to the resin and shaken for 2h. The resin was drained and washed with DMF, DCM, Et₂O, DCM, and then shaken with 10% DIPEA in DMF for 10 min. The resin was drained and washed with DMF, DCM, Et₂O, DCM, and finally Et₂O. The resin (**12**) was then dried *in vacuo*.

A solution of 4-[4-(1-(Fmoc-amino)ethyl)-2-methoxy-5-nitrophenoxy)butanoic acid (23 mg, 0.045 mmol), DIPEA (16 µl, 0.09 mmol) and HATU (17 mg, 0.045 mmol) in DMF (2 ml) was added to resin **12** (50 mg, 0.015 mmol) and the whole shaken for 3h. The resin was drained and washed with DMF, DCM, Et₂O, DCM, and finally Et₂O. The resin was then treated with 20% piperidine in DMF (3 ml) and the whole shaken for 1h. The resin (**13**) was then drained and washed with DMF, DCM, Et₂O, DCM, and finally Et₂O and dried *in vacuo*.

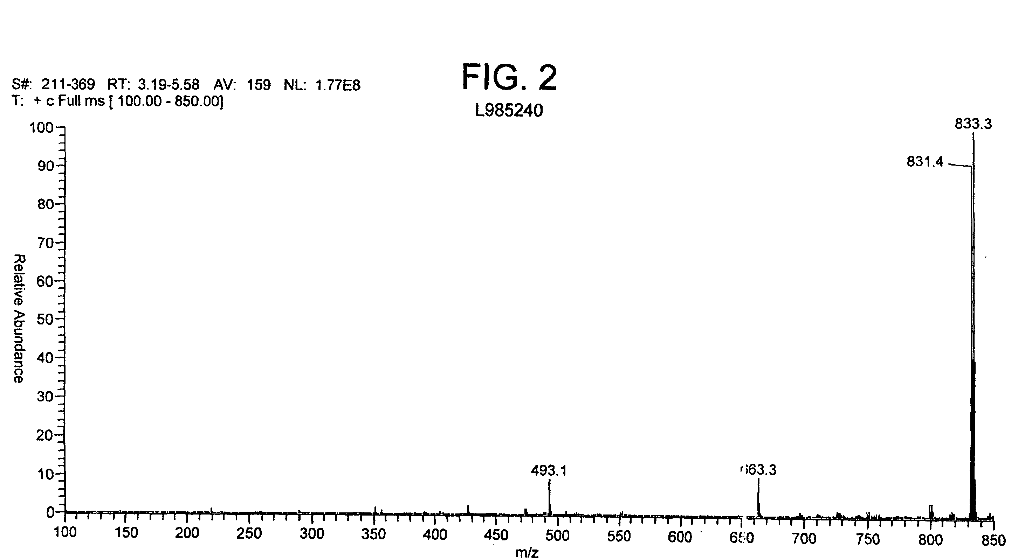

A solution of benzoic acid (11 mg, 0.09 mmol), DIPEA (16 µl, 0.09 mmol) and HATU (17 mg, 0.045 mmol) in DMF (2 ml) was added to resin **13** and the whole shaken for 2h. The resin (**14**) was then drained and washed with DMF, DCM, Et₂O, DCM, and finally Et₂O. Construct analysis of this resin by pyrimidine cleavage conditions (described below) followed by MS analysis is shown in figure **2**.

### General method of Construct Analysis of Resins via the Pyrimidine Linker.

A small sample of resin (ca. 0.5 mg) is treated with 0.01M m-chloroperbenzoic acid (0.5 ml) for 2h. The resin is then drained and washed with DCM, Et₂O and DCM. The resin is then treated with 0.02 M N-methylpiperazine in DMF (50 µl) is then added and shaken for 12h. The solution is then removed and analysed by mass spectrometry.

### EXAMPLE 3

### Preparation of Constructs Containing Different Peak Splitting Groups

A number of different mass spectrometric peak splitting groups have been prepared, and these are illustrated in Table 1 below.

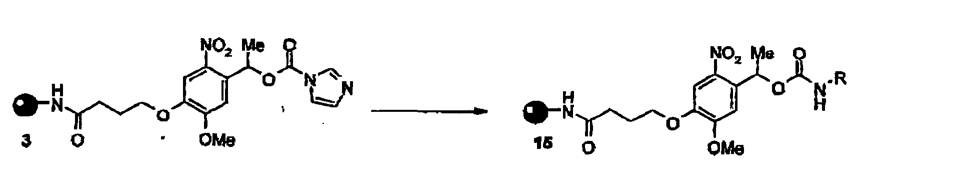

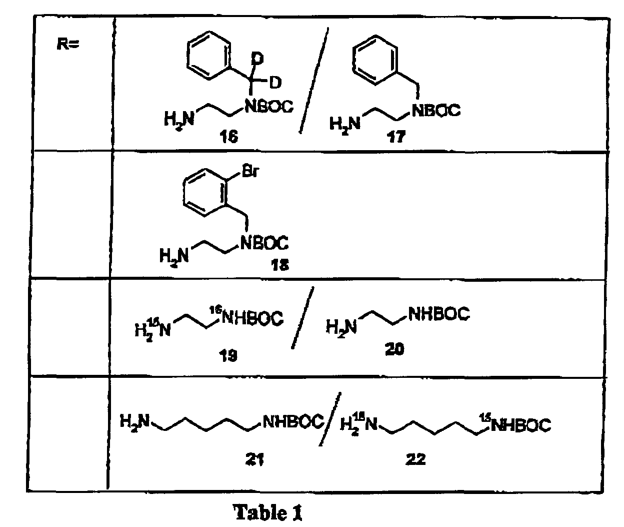

In Example 1, the peak splitting group was introduced by means of the mixture of deuterium labelled and unlabelled compounds **16/17** in Table 1 (see reference to resin **4**) whereas in Example 2, the peak splitting group was introduced by means of the bromo-benzyl compound **18** (see reference to resin **12**).

Resins containing alternative peak-splitting groups were prepared using the compounds **19/20** and **21/22** as described below.

### Use of amines 19/20

A solution of 1-*tert*-butoxycarbonyl-1,2-diaminoethane (**20**) (110 mg, 0.68 mmol) and doubly ¹⁵N labelled 1-*tert*-butoxycarbonyl-1,2-di(¹⁵N)aminoethane (**19**) (112 mg, 0.68 mmol) in DMF was added to resin **3** (0.6 g, 0.18 mmol) and the whole heated at 50 °C for 3h. The resin was then drained and washed with DMF, DCM, Et₂O, DCM, and finally Et₂O. The resin was then dried *in vacuo*.

### Use of amines 21/22

A solution of 1-*tert*-butoxycarbonyl-1,5-diaminopentane (**21**) (295 mg, 1.45 mmol) and doubly ¹⁵N labelled 1-*tert*-butoxycarbonyl-1,2-di(¹⁵N)aminopentane) (**22**) (295 mg, 1.45 mmol) in DMF was added to resin **3** (1.66 g, 0.58 mmol) and the whole heated at 50 °C for 4h. The resin was then drained and washed with DMF, DCM, Et₂O, DCM, and finally Et₂O. A solution of 95% aq.TFA in DCM (20%, 1 ml) was then added to the resin and shaken for 2h. The resin was drained and washed with DMF, DCM, Et₂O, DCM, and then shaken with 10% DIPEA in DMF for 10 min. The resin (**23**) was then dried *in vacuo.*

### EXAMPLE 4

### Preparation of Constructs Containing Dipeptide Substrates

In Examples 1 and 2, the substrate or target compound in each case is the model compound benzamide. In the following Examples, constructs were prepared containing dipeptides, as the substrate, bound to either a Rink linker or a Wang linker as the second linker group. In each case, a photocleavable first linker group was used. The reaction schemes used to prepare the constructs are shown in Schemes 3 and 4 below.

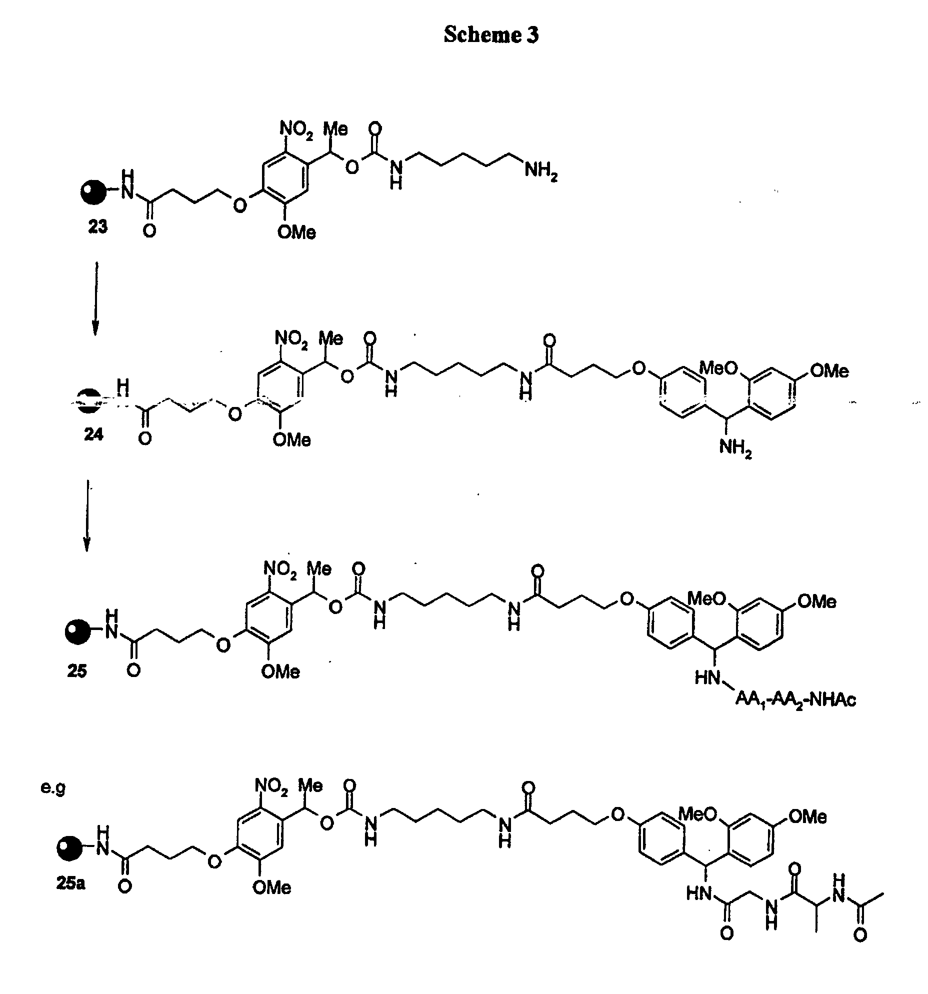

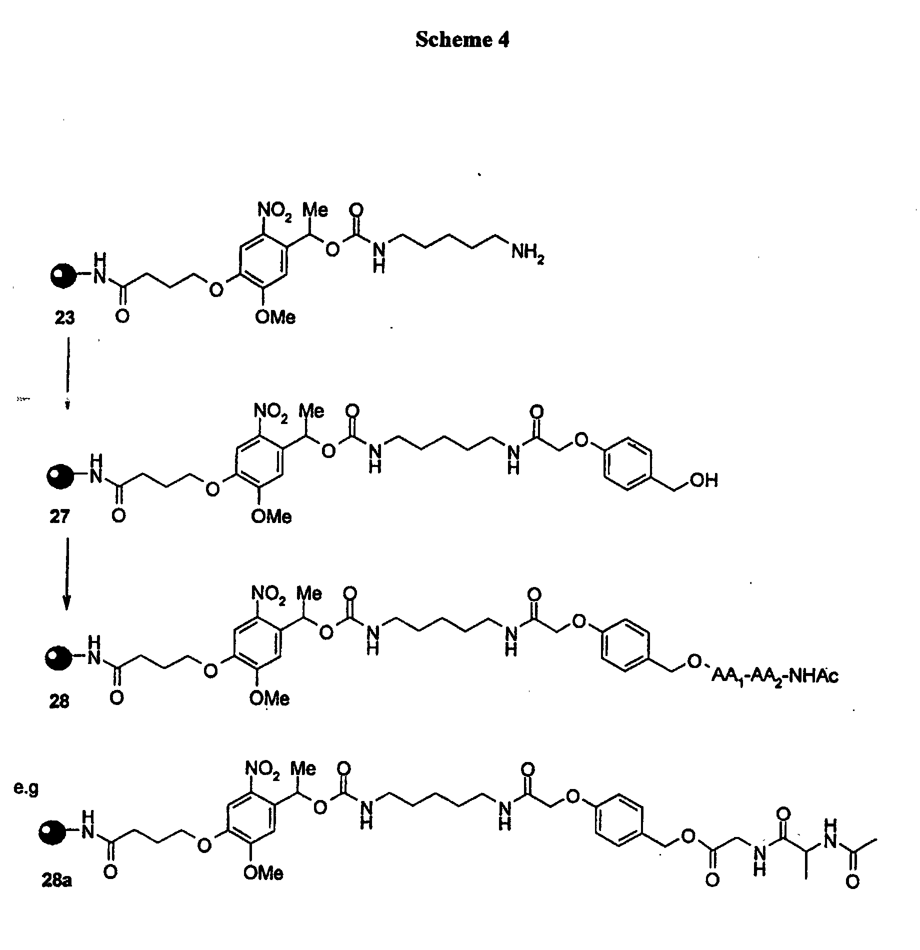

### Experimental for Amino acid examples

### Preparation of Rink resin 24

A solution of p-[(R,S)-α-[1-(9H-fluoren-9-yl)-methoxyformamido]-2,4-dimethoxy-benzyl]-phenoxybutanoic acid (240 mg, 0.42 mmol), DIC (65 µl, 0.42 mmol) and HOBT (57 mg, 0.42 mmol) in DMF (1 ml) was added to resin **23** (100 mg, 0.042 mmol) and the whole shaken for 4h. The resin was then drained and washed with DMF, DCM, Et₂O, DCM, and finally Et₂O. The resin was then treated with 20% piperidine in DMF (2 ml) and shaken for 30 min. The resin (**24**) was then drained and washed with DMF, DCM, Et₂O, DCM, and finally Et₂O. The resin was then dried *in vacuo*.

### Preparation of Wang resin 27.

A solution of 4-formylphenoxyacetic acid (23 mg, 0.13 mmol), DIC (20 µl, 0.13 mmol) and HOBT (17 mg, 0.13 mmol) in DMF (1 ml) was added to resin **23** (100 mg, 0.042 mmol) and the whole shaken for 4h. The resin was then drained and washed with DMF, DCM, Et₂O, DCM, and finally Et₂O. The resin was then dried *in vacuo.* The resin was then treated with a solution of tetrabutylammonium borohydride (50 mg, 0.2 mmol) in DCM and shaken for 16h. The resin **(27)** was then drained and washed with DCM, DMF, DCM, Et₂O, DCM, and finally Et₂O. The resin was then dried *in vacuo*.

### Preparation of acylated dipeptide sequence on resin.

**Table 2:**

| AA1 and AA2 on Rink and Wang linkers | | | | | |
|---|---|---|---|---|---|
| | **RINK (25)** | | | **WANG (28)** | |
| | AA1 | AA2 | | AA1 | AA2 |
| | | | | | |
| **Example 25a** | Gly | Ala | **Example 28a** | Gly | Ala |
| **Example 25b** | Val | Gly | **Example 28b** | Val | Gly |
| **Example 25c** | Ala | Val | **Example 28c** | Ala | Val |

The resins **25** and **28** were prepared by reacting the resins **24** and **27** respectively with the desired Fmoc protected amino acids followed by deprotection and then repeated coupling to the second aminoacid and deprotection (see general method). The terminal amino groups were then acylated using acetic anhydride (see method below).

Construct analysis of these resins by photocleavage followed by MS analysis gave the mass spectra shown in the figures according to table 3.

**Table 3:**

| Mass spectra after construct analysis | |
|---|---|
| Analysed Resin | Figure |
| | |
| **25a** | 3 |
| **25b** | 4 |
| **25c** | 5 |
| **28a** | 6 |
| **28b** | 7 |
| **28c** | 8 |

### General/Typical Method for coupling the amino acids to the linkers

A solution of the Fmoc protected amino acid (0.14 mmol, 10 Eq), DIC (22 µl, 0.14 mmol, 10 Eq) and HOBT (19 mg, 0.14 mmol, 10 Eq) in DMF (1ml) was added to required resins **24** and **27** (30 mg, ca. 0.014 mmol). The whole was then shaken for 5h. The resin was then drained and washed with DCM, DMF, DCM, Et₂O, DCM, and finally Et₂O. The Fmoc protecting groups were then be removed by the treatment of the resins with 20% piperidine solution in DMF (2 ml) and shaking for 30 min. The resin was then drained and washed with DCM, DMF, DCM, Et₂O, DCM, and finally Et₂O.The resin was then dried *in vacuo*.

### Acetylation of the dipeptides

A solution of acetic anhydride (15 ml, 0,14 mmol) in DCM (1 ml) was added to the resins (30 mg, ca. 0.014 mmol) followed by DMAP (1 mg, 0.008 mmol) and the whole shaken for 2h. The resin was then drained and washed with DCM, DMF, DCM, Et₂O, DCM, and finally Et₂O. The resin was then dried *in vacuo*.

As can be seen from the above examples, the constructs of the invention enable small molecules formed by solid phase methods to be analysed readily and easily by mass spectrometry. By providing an ionisable group as a mass spectral sensitiser, and a peak splitting isotope, the result in each case has been a readily interpreted mass spectrum in which the molecular ion appears as a characteristic doublet, and in which peaks due to fragments or extraneous materials are negligible.

### EXAMPLE 5

### Preparation of a Construct Containing a Sulphonamide Linker at the First Cleavage Site

A construct containing benzoic acid as a model substrate linked via an acid labile "Rink" type linker to an ethylene diamine mass spectral peak splitting group and in turn via a sulphonamido linker to a resin was prepared as shown in Scheme 5 below. In this Example, cleavage of the sulphonamide with mercaptoethanol in the presence of strong base releases an analytical fragment having a mass spectrometric sensitising amino group

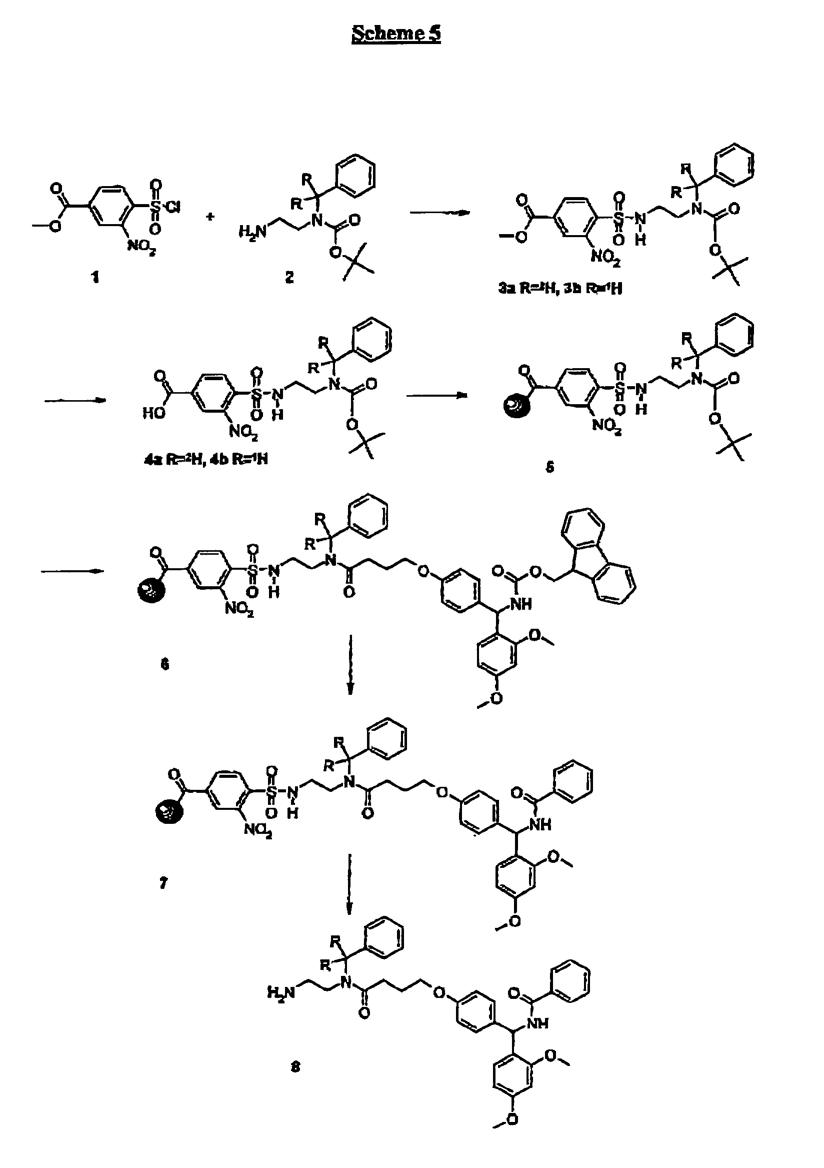

### Preparation of Compound 3a

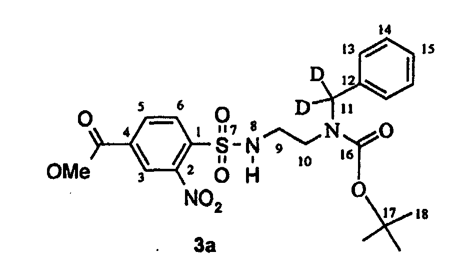

To a stirred solution of 2-nitro-4-methylbenzoatesulfonyl chloride **1** (1.10 g, 3.90 mmol) and triethylamine (0.39 g, 3.90 mmol) in dry dichloromethane (40 ml), was added at 0°C the amine **2a** (1.00 g, 3.9 mmol) in dry dichloromethane (20 ml) over 30 min. The reaction mixture was stirred for a further 2 h at room temperature then concentrated *in vacuo* to give a yellow oil. Purification by chromatography with [MTBE:hexane (1:1)] as eluant followed by recrystallisation from MTBE (5 ml) and hexane (2 ml) furnished ***the sulfonamide 3a*** (1.53 g, 79%) as white flakes; mp 129-130°C; R_{f} 0.31 [MTBE:hexane (1:1)]; (Found: C, 53.2; H, 5.6; N, 8.5; S, 6.4. C₂₂H₂₅D₂N₃O₈S requires C, 53.3; H, 5.5; N, 8.5; S, 6.5%); δ_{H} (DMSO-d⁶) 1.40 (9H, s, H-18), 3.05 (2H, m, H-9), 3.20 (2H, dt, *J*3 and 13, H-10), 3.9 (3H, s, O-Me), 7.10-7.35 (5H, m, H-13, 14 and 15), 8.09 (1H, d,*J*8, H-6), 8.35 (1H, d, *J* 8, H-5), 8.40 (1H, m, H-8), 8.45 (1H, s, H-3),; δ_{C} (DMSO-d⁶) 27.8 (C-18), 40.9 (C-9 and 10), 45.9 (C-11), 53.0 (O-Me), 79.1 (C-17), 124.8 (C-13), 127.0 (C-14 and 15), 128.3 (C-6), 130.0 (C-13), 132.9 (C-5), 134.2 (C-12), 136.4 (C-1), 147.5 (C-4), 154.7 (C-2), 163.5 (C-16 and C=O); MS (Electrospray +ve) m/z 496 (MH)⁺; HPLC, Rₜ 6.07 min.

### Preparation of Compound 4a

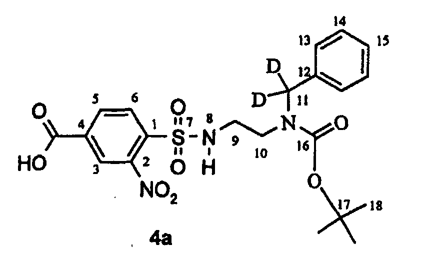

The ester **3a** (0.40 g, 0.80 mmol) in methanol (2 ml) was treated with 1M aqueous sodium hydroxide (1.60 ml, 1.60 mmol) and stirred at room temperature for 1 hour. The reaction was then concentrated *in vacuo* and the residue dissolved in water (10 ml) then acidified with 1M hydrochloric acid (1.80 ml, 1.80 mmol) at 0°C. The resulting white precipitate was extracted with dichloromethane (3 x 10 ml). The combined organic extracts were washed with brine, dried (Na₂SO₄) and evaporated under reduced pressure to ***the acid 4a*** (0.34 g, 88%) as a white solid; mp 143-145°C; R_{F} 0.58 [MTBE:hexane (1:1)]; δ_{H} (DMSO-d⁶) 1.29 (9H, s, H-18), 3.02 (2H, m, H-9), 3.20 (2H, dt, *J* 3 and 13, H-10), 7.10-7.40 (5H, m, H-13, 14 and 15), 8.06 (1H, d, *J* 8, H-6), 8.28 (1H, s, H-3), 8.30 (1H, d, *J* 8, H-3); δ_{C} (DMSO-d⁶) 27.9 (C-18), 40.9 (C-9 and 10), 45.9 (C-11), 79.1 (C-17), 124.5 (C-13), 127.1 (C-14 and 15), 128.4 (C-6), 129.5 (C-13), 132.5 (C-5), 139.2 (C-12), 147.3 (C-1), 155.1 (C-4), 164.8 (C-16 and C=O); (Found: MH⁺, 482.156364 C₂₁H₂₃D₂N₃O₈S requires MH+, 482.156615); HPLC, Rₜ 5.65 min.

### Preparation of Compound 3b

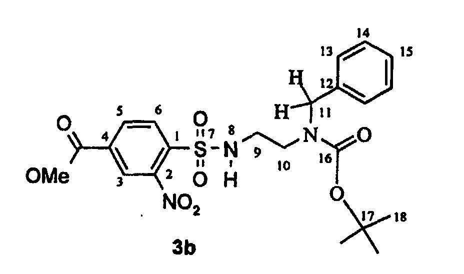

To a stirred solution of 2-nitro-4-methylbenzoatesulfonyl chloride **1** (1.10 g, 3.90 mmol) and triethylamine (0.39 g, 3.90 mmol) in dry dichloromethane (40 ml), was added at 0°C the amine **2b** (0.95 g, 3.9 mmol) in dry dichloromethane (20 ml) over 30 min. The reaction mixture was stirred for a further 3 h at room temperature then concentrated *in vacuo* to give a yellow oil. Purification by chromatography with [MTBE:hexane (1:1)] as eluant followed by recrystallisation from MTBE (5 ml) and hexane (2 ml) furnished ***the sulfonamide 3b*** (1.51 g, 79%) as white flakes; mp 131-132°C; R_{f} 0.28 [MTBE:hexane (1:1)]; (Found: C, 53.7; H, 5.4; N, 8.4; S, 6.5. C₂₂H₂₇N₃O₈S requires C, 53.5; H, 5.5; N, 8.5; S, 6.5%); δ_{H} (DMSO-d⁶) 1.38 (9H, s, H-18), 3.10 (2H, t, *J* 8, H-9), 3.24 (2H, t, *J* 8, H-10), 3.95 (3H, s, O-Me), 4.32 (2H, s, H11), 7.13-7.32 (5H, m, H-13, 14 and 15), 8.09 (1H, d, *J* 8, H-6), 8.29 (1H, s, H-3), 8.32 (1H, d, *J* 8, H-3); δ_{C} (DMSO-d⁶) 28.3 (C-18), 40.2 (C-9 and 10), 46.2 (C-11), 53.2 (O-Me), 79.3 (C-17), 125.1 (C-13), 127.2 (C-14 and 15), 128.6 (C-6), 130.3 (C-13), 133.1 (C-5), 134.4 (C-12), 136.6 (C-1), 147.6 (C-4), 163.7 (C-16 and C=O); MS (Electrospray -ve) m/z 492 (M-H)⁻; HPLC, Rₜ 5.99 min.

### Preparation of Compound 4b

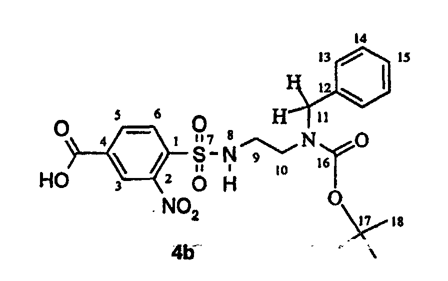

The ester **3b** (0.40 g, 0.80 mmol) in methanol (2 ml) was treated with 1M aqueous sodium hydroxide (1.60 ml, 1.60 mmol) and stirred at room temperature for 1 hour. The reaction was then concentrated *in vacuo* and the residue dissolved in water (10 ml) then acidified with 1M hydrochloric acid (1.80 ml, 1.80 mmol) at 0°C. The resulting white precipitate was extracted with dichloromethane (3 x 10 ml). The combined organic extracts were washed with brine, dried (Na₂SO₄) and evaporated under reduced pressure to ***the acid 4b*** (0.36 g, 93%) as a white solid; mp 123.7-124.8°C; R_{F} 0.59 [MTBE:hexane (1:1)]; δ_{H} (DMSO-d⁶) 1.42 (9H, s, H-18), 3.12 (2H, m, H-9), 3.22 (2H, m, H-10), 4.38 (2H, s, H11), 7.18-7.42 (5H, m, H-13, 14 and 15), 8.11 (1H, d, *J* 8, H-6), 8.39 (1H, d, *J* 8, H-3); 8.45 (1H, s, H-3); δ_{C} (DMSO-d⁶) 27.8 (C-18), 40.1 (C-9 and 10), 45.9 (C-11), 79.1 (C-17), 124.8 (C-13), 126.9 (C-14 and 15), 128.4 (C-6), 129.9 (C-13), 132.9 (C-5), 135.8 (C-12), 137.9 (C-1), 147.5 (C-4), 153.9 (C-2), 163.7 (C-16 and C=O) MS (Electrospray -ve) m/z 478 (M-H)⁻; HPLC, Rₜ 5.56 min.

### Preparation of Compound 5

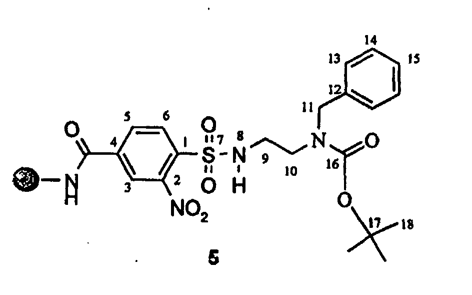

To a suspension of amine resin (Argogel-NH₂) (0.37 g, 0.15 mmol) ), having an average bead diameter of 150 µm and a loading of 0.4 mmol g⁻¹, in DMF (4 ml) and dichloromethane (2 ml) was added the benzoic acid (**4a**) (0.22 g, 0.45 mmol), and the benzoic acid (4b) (0.22 g, 0.45 mmol), followed by PyBOP (0.47 g, 0.9 mmol) and HOBT (012. g, 0.90 mmol) followed, 4 min. later by Hünig's base (0.21 g, 1.80 mmol). The reaction was agitated with nitrogen for 18 h and the resin then washed with dichloromethane (6 x 10 ml), DMF (6 x 10 ml), dichloromethane (6 x 10 ml), ether (2 x 10 ml) and dried *in vacuo* to give the ***sulfonamide resin 5*** (0.43 g, 97%) as a yellow solid; Kaiser test was negative; Bromophenol Blue test was negative.

### Preparation of Compound 6

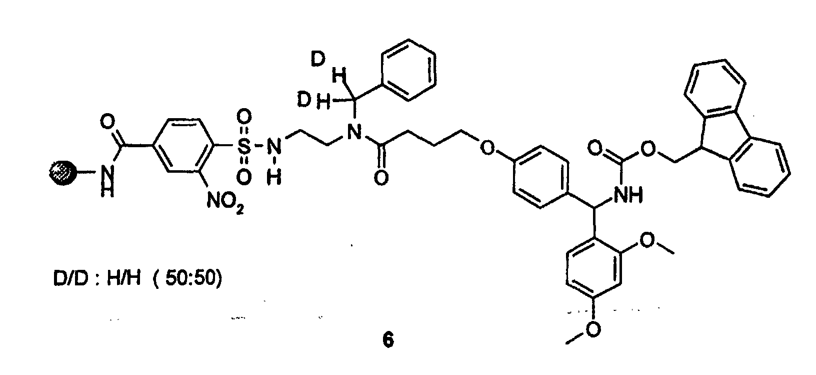

The protected resin (0.39 g, 0.13 mmol) was suspended in dichloromethane (2 ml) and treated with trifluoroacetic acid (2 x 4 ml) for 5 min. Solvents were removed by filtration and the resin washed with 10% DIPEA solution in DMF (3 x 5 ml), dichloromethane (6 x 5 ml), DMF (6 x 5 ml), dichloromethane (6 x 5 ml), ether (2 x 5 ml). The amine resin was then suspended in DMF (4 ml) and dichloromethane (2 ml) the Rink acid (0.51 g, 0.90 mmol) was added, followed by PyBOP (0.47 g, 0.9 mmol) and HOBT (012. g, 0.90 mmol). After 4 min. Hünig's base (0.21 g, 1.80 mmol) was added. The reaction was agitated with nitrogen for 18 h and the resin then washed with dichloromethane (6 x 10 ml), DMF (6 x 10 ml), dichloromethane (6 x 10 ml), ether (2 x 10 ml) and dried *in vacuo* to give the ***sulfonamide resin 6*** (0.43 g, 100%) as a yellow solid; Kaiser test was negative; Bromophenol Blue test was negative; Resin loading 98% as estimated by quantitative Fmoc group cleavage.

### Preparation of Compound 7

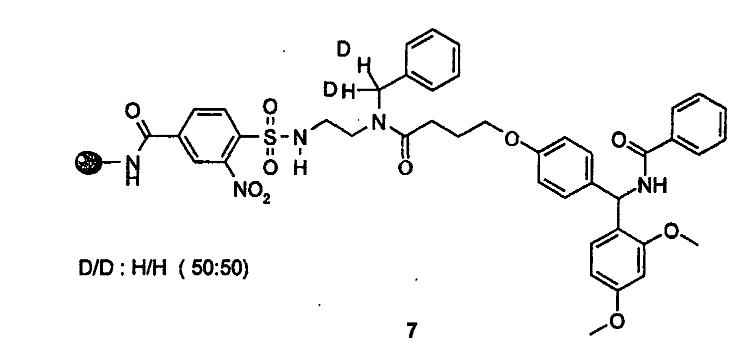

Resin (**6**) (0.20 g, 0.06 mmol) was treated with 20% Piperidine in DMF solution (2 x 3 ml) for 10 min. Solvents were removed by filtration and the resin washed with DMF (6 x 3 ml), dichloromethane (6 x 3 ml), ether (2 x 3 ml). The resin was suspended in DMF (1 ml) and dichloromethane (1 ml) and benzoic acid (12 mg, 0.01 mmol) was added, followed by PyBOP (52 mg, 0.1 mmol) and HOBT (13 mg, 0.1 mmol). After 4 min. Hünig's base (26 mg, 0.2 mmol) was added. The reaction was agitated with nitrogen for 3 h and the resin then washed with dichloromethane (6 x 1 ml), DMF (6 x 1 ml), dichloromethane (6 x 1 ml), ether (2 x 1 ml) and dried *in vacuo* to give the ***sulfonamide resin 7*** (51 mg, 100%) as a yellow solid; Kaiser test was negative; Bromophenol Blue test was negative;

### Preparation of Compound 8

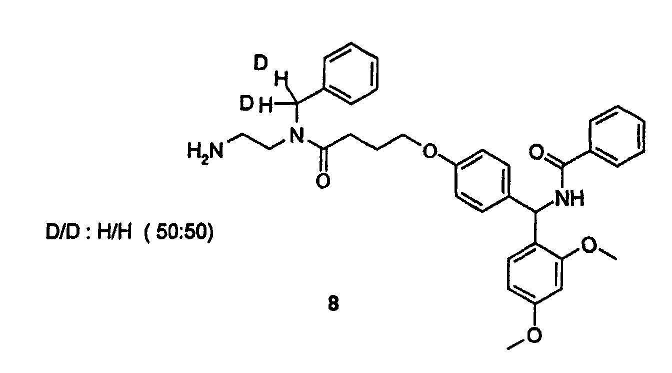

A small sample of resin (**7**) (ca. 0.5 mg) was treated with deprotecting solution A (0.5 ml) for 1 hr. The resin was filtered and the resulting solution was then analysed by electrospray mass spectrometry; MS (Electrospray -ve) m/z 582/584 (M-H)⁻; HPLC, Rₜ 4.49 min.
Solution A: Mercaptoethanol (0.23 ml, 3.0 mmol) and DBU (0.67 ml, 4.5 mmol) in MeCN (3 ml).

### EXAMPLE 5

### Preparation of a Construct Containing a dde-linker at the first Cleavage site

A construct containing a benzoic acid model substrate linked via a "Rink" type linker to an ethylene diamine mass spectral peak splitter and thence via a "dde" linker to a resin was prepared according to the reaction scheme set out in Scheme 6 below. Cleavage of the construct at the first cleavage site defined by the dde group using hydrazine generates an analytical fragment having a free amino group as a mass spectrometric sensitising group.

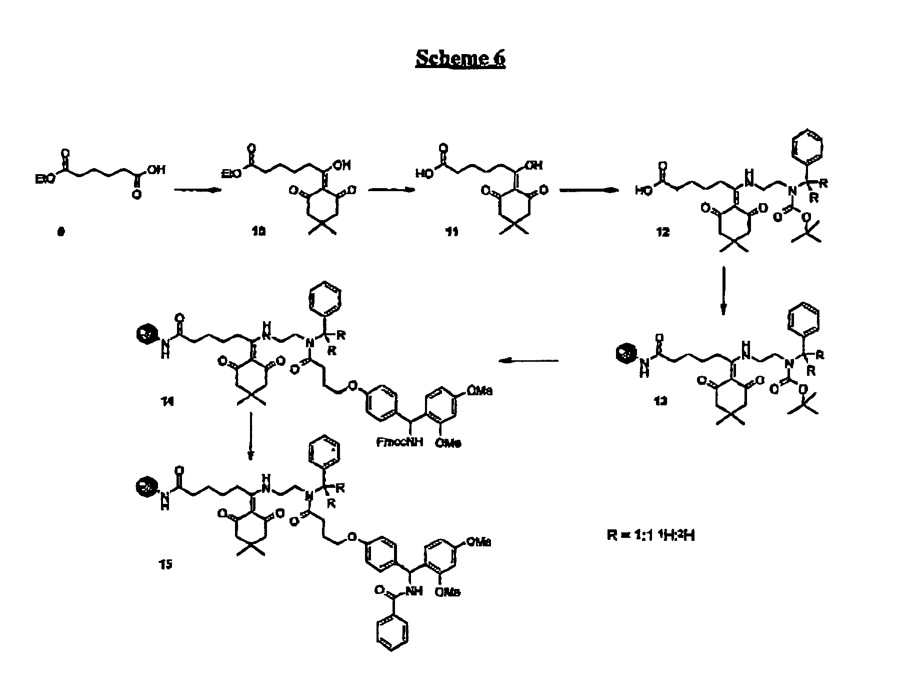

### General

HPLC was run on a Hewlett Packard 1050 instrument using a C₁₈ reverse phase column (Supelco, Supelcosil ABZ+, 3.3 mm, 4.6 mm φ). Method A: 10 to 95% solvent B gradient (1 ml/min) as the mobile phase. [Solvent A: 1% TFA in water; solvent B; 0.5% TFA in MeCN:water (10:1), 8 min gradient time], or method B: C₁₈ reverse phase column (Dynamax 60A, 25 mm, 6 mm φ) with 10 to 95% solvent B gradient (1 ml/min) as the mobile phase. [Solvent A: 1% TFA in water; Solvent B; 0.5% TFA in MeCN:water (10:1), 10 min gradient time]. Melting points were performed on a Mettler FP5 automatic melting point apparatus in open tubes heating from 50°C at 2°C min⁻¹ and are uncorrected.

### Preparation of Compound 10

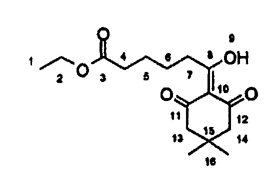

4-Dimethylaminopyridine (4.03 g, 33 mmol) was added to a solution of adipic acid monoethylester (5.33 ml, 30 mmol) in dimethylformamide (50 ml) followed by diisopropylcarbodiimide (5.17 ml, 33 mmol). After 25 min, a solution of dimedone (4.63 g, 33 mmol) in dimethylformamide (25 ml) was added and stirring continued for 48 h. The reaction mixture was diluted with ethyl acetate (300 ml) and washed with 0.5M KHSO₄ (2 x 100 ml), water (100 ml) and saturated brine (50 ml), then dried (MgSO₄), filtered and evaporated. The resulting slurry was triturated with ethyl acetate (250 ml), filtered and the residue washed with ethyl acetate. Evaporation of the combined filtrates gave a yellow gum which was purified by solid phase extraction (1.5" x 4" column, eluant: dichloromethane) to give ***compound 10*** as a yellow oil (7.43 g, 84%). δ_{H} (CDCl₃, 400MHz): 4.04 (q, 2H, H₂, 7 Hz), 2.97 (t, 2H, H₄, 7.5 Hz), 2.46 (s, 2H, H₁₃), 2.28 (s, 2H, H₁₄), 2.26 (d, 2H, H₇, 7.5 Hz), 1.60 (m, 4H, H_{5,6}), 1.00 (s, 6H, H₁₆). δ_{C} (CDCl₃, 400 MHz): 205.15 (C-12), 197.58 (C-11), 195.08 (C-3), 173.47 (C-8), 112.02 (C-10), 60.27 (C-2), 52.67 (C-4), 46.80 (C-13), 40.04 (C-14), 34.15 (C-7), 30.72 (C-15), 28.23 (C-16), 24.64 (C-5), 24.01 (C-6), 14.31 (C-1). νₘₐₓ (cm⁻¹): 2960 (OH), 1734 (C=O ester), 1665 (C=O ketone), 1564, 1445, 1143. MS: (electrospray +ve) m/z 297 (MH)⁺ (electrospray +ve), 295 (M-H)⁻. HPLC method A: 5.31 min, 97%. 254nm.

### Preparation of Compound 11

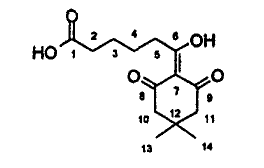

2M sodium hydroxide solution (2 ml, 4 mmol) was added to a solution of compound **10** in tetrahydrofuran (5 ml). After 3 h, the reaction mixture was poured onto 10% aq. hydrochloric acid (50 ml) and extracted with ethyl acetate (2 x 50 ml). The combined organic phases were dried (MgSO₄), filtered and evaporated to give ***compound 11*** as an off white solid (498.5 mg, 95%). δ_{H} (CDCl₃, 400MHz): 2.99 (dd, 2H, H₂, 5 Hz), 2.47 (s, 2H, H₁₀), 2.34 (dd, 2H, H₅, 5Hz), 2.29 (s, 2H, H₁₁), 1.63 (m, 4H, H_{3,4}), 1.00 (s, 6H, H_{13,14}). δ_{C} (CDCl₃, 400 MHz): 205.15 (C-9), 197.58 (C-8), 195.08 (C-1), 173.47 (C-6), 112.02 (C-7), 52.96 (C-2), 47.11 (C-10), 40.40 (C-11), 33.92 (C-5), 30.72 (C-12), 28.23 (C-13,14), 24.64 (C-3), 24.01 (C-4). νₘₐₓ (cm⁻¹): 3030 (acid OH), 1702 (acid C=O), 1646 (ketone C=O). Melting point: 58.2°C. MS: (electrospray +ve) m/z 269 (MH)⁺ (electrospray -ve), 267 (M-H)⁻. HPLC method A: 4.15 min, 97%. 254nm.

### Preparation of Compound 12

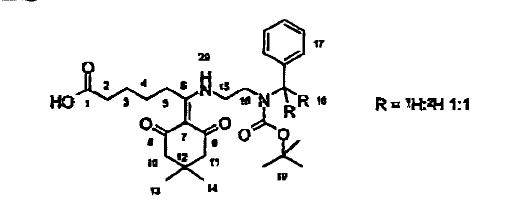

A solution of *N*-(*tert*-butoxycarbonyl)*-N*-benzyldiaminoethane (187.3 mg, 0.75 mmol) and *N*-(*tert*-butoxycarbonyl)-*N*-(α-dideutero)benzyldiaminoethane (189.3 mg, 0.75 mmol) in dichloromethane (3 ml) followed by diisopropylethylamine (348.4 µl, 2 mmol) was added to a solution of compound **11** (268.4 mg, 1 mmol) in dichloromethane (2 ml). After stirring for 20 h, the reaction mixture was evaporated, redissolved in ethyl acetate (25 ml) and washed with 10% citric acid solution (3 x 10 ml), water (10 ml) and saturated brine (10 ml). The organic phase was dried (MgSO₄), filtered and evaporated then purified by flash chromatography (1 to 3% methanol in chloroform) to give ***compound 12*** as a yellow gum (474.5mg, 95%). δ_{H} (CDCl₃, 400MHz): 13.50 (bs, 1H, H₂₀), 7.30 (m, 5H, H₁₇), 4.45 (bd, 1H, H₁₈), 3.47 (bd, 4H, H_{15,16}), 2.90 (m, 2H, H₂), 2.40 (t, 2H, H₅, 7.4Hz), 2.30 (s, 4H, H_{10,11}), 1.75 (m, 2H, H₄), 1.45 (m, 11H, H_{3,19}). νₘₐₓ (cm⁻¹): 3030 (acid OH), 1730 (C=O, Boc, acid), 1690 (ketone C=O), 1570 (conj. enamine). MS: (electrospray +ve) m/z 503, 501 (MH)⁺ (electrospray -ve), 502, 499 (M-H)⁻. HPLC method A: 5.35 min, >97%. 230, 254nm.

### Preparation of Compound 13

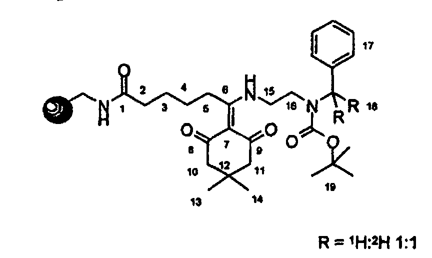

To a solution of compound **12** (310 mg, 0.62 mmol) in dichloromethane (6 ml) was added 1-hydroxybenzotriazole (110.8 mg, 0.82 mmol) and PyBOP (426.6 mg, 0.82 mmol). After 5 min, the solution was added to a suspension of aminomethyl Argogel® (500 mg, 0.205 mmol, 0.41 mmol/g loading) having an average bead size of 150µm, in dichloromethane (1 ml). The reaction mixture was agitated for 5 min then diisopropylethylamine (285.7µl, 1.64 mmol) was added and agitation continued for 16 h. The resin was washed with DMF (5 x) and DCM (5 x) then dried *in vacuo* to give ***resin 13*** (573.5 mg). The resin gave a negative bromophenol blue test.

### Preparation of Compound 14

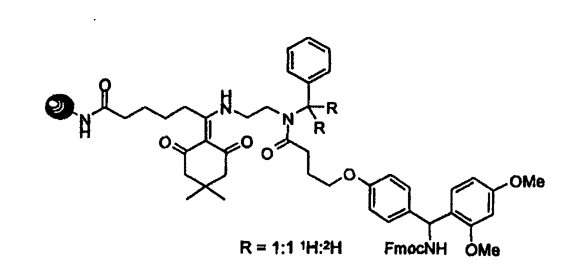

Resin **13** (475.5 mg) was swollen with dichloromethane then treated with a solution of phenol (60 mg) in dichloromethane (1ml) followed by trifluoroacetic acid (3ml). The reaction mixture was agitated for 10 min then drained and washed with dichloromethane (2 x). The reaction was repeated then the resin washed with dichloromethane (1 x), 20% diisopropylethylamine in dimethylformamide (3 x), dimethylformamide (5 x) and dichloromethane (5 x) to give the amino resin intermediate which gave a positive bromophenol blue. The resin was suspended in dichloromethane (2 ml) and treated with a preformed solution of the C-4 Rink acid (351.5 mg, 0.62 mmol), 1-hydroxybenzotriazole (110.8 mg, 0.82 mmol) and PyBOP (426.6 mg, 0.82 mmol) in dimethylformamide (2 ml). After 5 min, diisopropylethylamine (285.7µl, 1.64 mmol) was added and the reaction mixture agitated for 3 h after which the resin gave a negative bromophenol blue test. The resin was washed with DMF (5 x) and DCM (5 x) then dried *in vacuo* to give ***resin 14*** (500.9 mg).

A small sample of resin 14 (ca. 0.5 mg) was treated with 2% hydrazine in dimethyl formamide for 30 minutes. The solution was then analysed by electrospray mass spectrometry to give an ion 700/702.

### Preparation of Compound 15

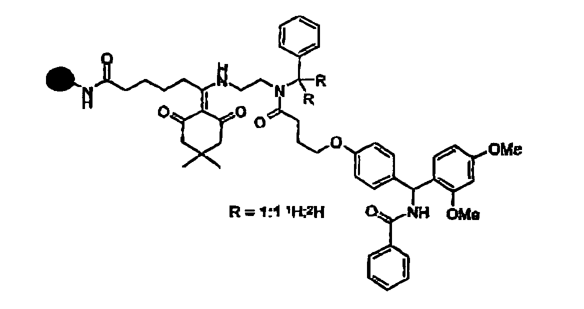

Resin **14** (65 mg) was treated with 20% piperidine in dimethylformamide (2 x 3 ml x 10 min) then washed with dimethylformamide (5 x) and dichloromethane (5 x). The resin gave a positive Kaiser test and was suspended in dichloromethane (1 ml) then treated with a preformed solution of benzoic acid (127 mg, 1.04 mmol), 1-hydroxybenzotriazole (140 mg, 1.04 mmol) and PyBOP (541 mg, 1.04 mmol) in dimethylformamide (2 ml). After 5 min, diisopropylethylamine (541 mgµl, 2.08 mmol) was added and the reaction mixture agitated for 72 h after which the resin gave a negative Kaiser test. The resin was washed with DMF (5 x) and DCM (5 x) then dried *in vacuo* to give ***resin 15*** (60 mg).

A small sample of the resin (ca. 0.5mg) was treated with 2% hydrazine in dimethylformamide for 30 minutes. The solution was then analysed by electrospray mass spectrometry to an ion 582/584.

### EXAMPLE 6

### Preparation of a 64-Membered Library By a 4x4x4 Combinatorial Synthesis

The usefulness of the constructs of the invention in creating combinatorial libraries was demonstrated by preparing a 64 membered library of constructs containing different diamide substrates using a 4x4x4 combinatorial synthetic procedure. In order to provide a means of determining the synthesis history of the beads, and to enable sorting and mixing of the beads to be positively controlled (rather than being determined merely by simple statistical distribution) so that all 64 members of the library were formed, quantities of the resin beads were loaded into 64 radio frequency labelled Iron MicroKan ™ containers. The MicroKan ™ containers, available from Irori, of La Jolla, California, USA, are polypropylene containers having a polypropylene mesh side wall, a resin capacity of up to 30 mg and an internal volume of 330 µl. The use of the MicroKan™ containers provides the benefits, in terms of numbers of compounds synthesised, of a split and pool combinatorial synthesis, but avoids the need to build up coding constructs on the resin beads since the MicroKan ™ containers are provided with radio-frequency tags which enable each container to be identified and therefore monitored through the reaction sequence. Consequently, since each container contains a unique substrate at the end of the reaction sequence, and the synthesis history of the container has been tracked, the identity of the substrate in each container can be determined, assuming of course that each reaction step has proceeded according to plan.

The diamide substrates were built up on a scaffold containing a thiopyrimidine safety catch linker at the first cleavage site, a diamino ethane connecting group carrying an N-benzyl peak splitting group, and a photolabile linker at the second cleavage site joining the substrate to the diaminoethane connecting group.

An intermediate construct containing the N-benzyl diamino ethane peak splitter group and photolabile pyrimidine linker group was prepared by the reaction sequence shown in Scheme 7 below, the subsequent synthetic steps and preparation of the combinatorial library being shown in Scheme 8. The experimental details for each scheme are set out below and the compound or construct numbers used in the experimentals refer to the construct/compound numbers in the two schemes.

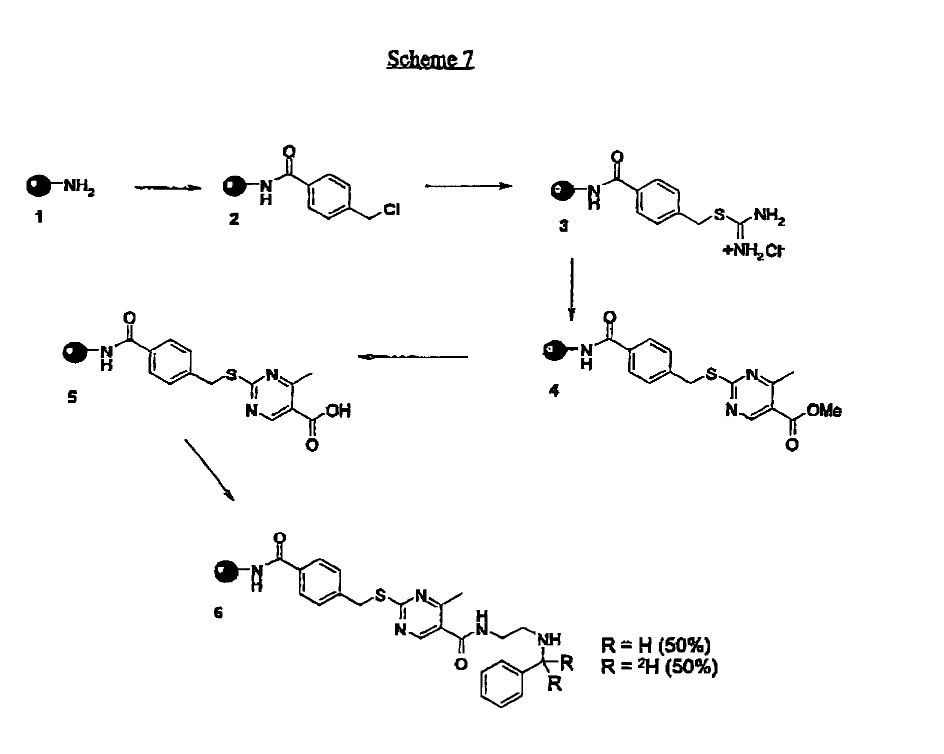

### Experimental for pyrimidine construct

A solution of 4-(chloromethyl)benzoic acid (3.91 g, 23 mmol), HATU (8.74 g, 23 mmol) and DIPEA (7.95 ml, 46 mmol) in DMF (40 ml) was shaken for 20 min then this solution was added to Argogel resin **1** (1 g, 0.46 mmol) ), having an average bead diameter of 150 µm and a loading of 0.46 mmol g⁻¹, and the whole was shaken for 5h. The resin was then drained and washed with DMF, DCM, Et₂O, DCM and finally Et₂O. The resin **(2)** was then dried *in vacuo.*

A slurry of thiourea (8.4 g, 110 mmol) in dioxane (80 ml) and ethanol (20 ml) was added to Resin **2** and the whole heated at 80 °C for 16h. The resin was drained and washed with DMF (x 5), DCM (x 5), Et₂O, DCM and finally Et₂O to give resin **3.** The resin was then dried *in vacuo.*

A solution of methyl-2-(dimethylaminomethylene)-3-oxobutanoate (2.4 g, 13.8 mmol) and triethylamine (0.96 ml, 6.9 mmol) in DMF (100 ml) was added to resin **3** and the whole heated at 80 °C for 16h. The resin was drained hot and washed with DMF, DCM, Et₂O, DCM, and finally Et₂O. The resin (**4**) was then dried *in vacuo*. Resin **4** was then treated with a 1:1 mixture of THF and 2N aq.NaOH (100 ml) and shaken for 2h. The resin was then drained and washed with THF/H₂O, 10% AcOH/THF, THF/H₂O, THF, DMF, DCM, Et₂O, DCM, Et₂O and then dried *in vacuo* to give resin **5**.

A solution of PyBOP® (3.6 g, 6.9 mmol) and DIPEA (2.4 ml, 25 mmol) in DMF (30 ml) was added to resin **5** (5 g, 2.3 mmol) followed by a solution of 1-benzyl-1-*tert*-butoxycarbonyl-1,2-diaminoethane (0.86 g, 3.5 mmol) and 1-(dideuterobenzyl)-1-*tert*-butoxycarbonyl-1,2-diaminoethane (0.86 g, 3.5 mmol) in DMF (1 ml) and the whole shaken for 4 h. The resin was drained and washed with DMF, DCM, Et₂O, DCM, and finally Et₂O. A solution of 20% aq.TFA in DCM (20%, 30 ml) was then added to the resin and shaken for 2h. The resin was drained and washed with DMF, DCM, Et₂O, DCM, and then shaken with 10% DIPEA in DMF for 10 min. The resin was drained and washed with DMF, DCM, Et₂O, DCM, and finally Et₂O. The resin (**6**) was then dried *in vacuo*.

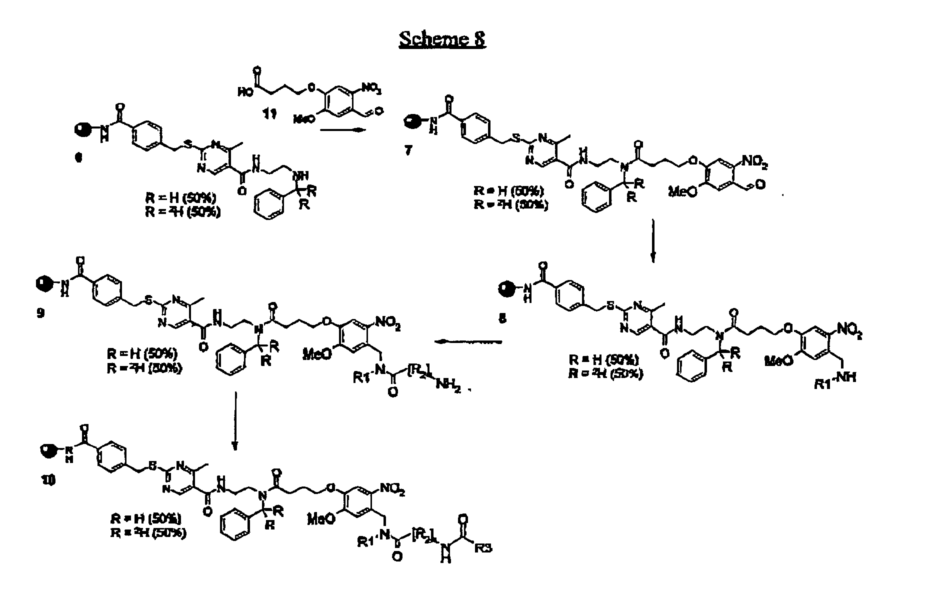

### Experimental for Scheme 8

The photolinker acid **11** (0.416 g, 1.47mmol), DIPEA (0.51 ml, 2.94 mmol) and PyBOP (0.765 g, 1.47 mmol) were dissolved in DMF (10ml) and left to stand for 10 mins. This was then added to resin **6** (0.7 g, 0.294 mmol) and shaken for 4 h. The resin was drained and washed with DMF, DCM, Et₂O, DCM, and then shaken with 10% DIPEA in DMF for 10 min. The resin was drained and washed with DMF, DCM, Et₂O, DCM, and finally Et₂O. The resin (**7**) was then dried *in vacuo*.

### General Procedure for the Reductive Amination Step

To a vessel containing 16 Irori MicroKan™ containers (each containing 10 mg, 0.004 mmol resin 7) in DCM (30 ml) was added a pre-mixed solution of the amine (for amounts see the table below) and acetic acid (46 µl, 0.768 mmol) in DCM (2 ml) and shaken for 20 min. To the vessel was then added a mixture of tetramethylammonium borohydride (0.404 mg, 1.54 mmol) and acetic acid (46 µl, 0.768 mmol) in DCM (5 ml) and the vessel shaken for 16 h. The vessels were then drained and the MicroKan (TM) containers washed with DCM, DMF, DCM, and Et₂O (each 3 times).

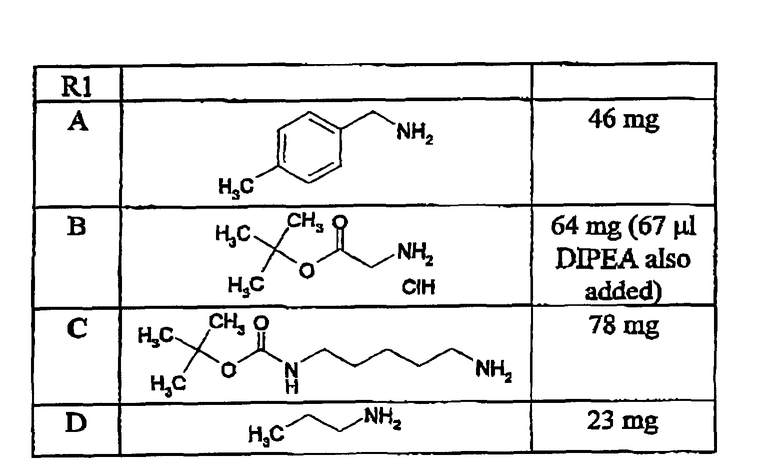

### General Procedure for the Acylation Step with amino acids

To each of 4 vessels containing 16 Irori MicroKan™ containers (each can containing 10 mg, 0.004 mmol resin **8**) in DMF (30 ml) was added a solution of PyBOP (333 mg, 0.64 mmol), DIPEA (224 µl, 1.28 mmol) and one of the amino acids (see the table below for amounts) in DMF (5 ml) and the mixture shaken for 16 h. The vessels were then drained and the MicroKan™ containers washed with DMF, DCM, DMF, DCM, and Et2O (each 3 times). To a vessel containing all 64 MicroKan™ containers was then added a solution of piperidine (20%) in DMF and shaken for 3 h. The vessels were then drained and the MicroKan™ containers washed with DMF, DCM, DMF, DCM, and Et2O (each 3 times).

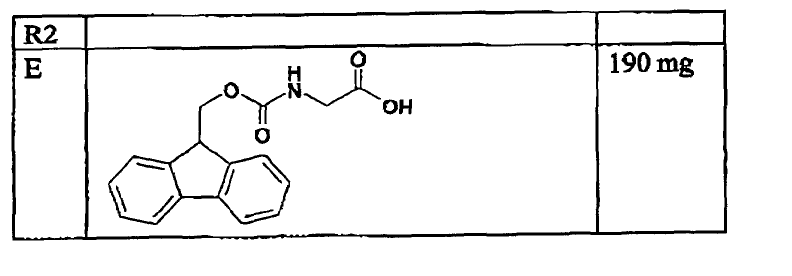

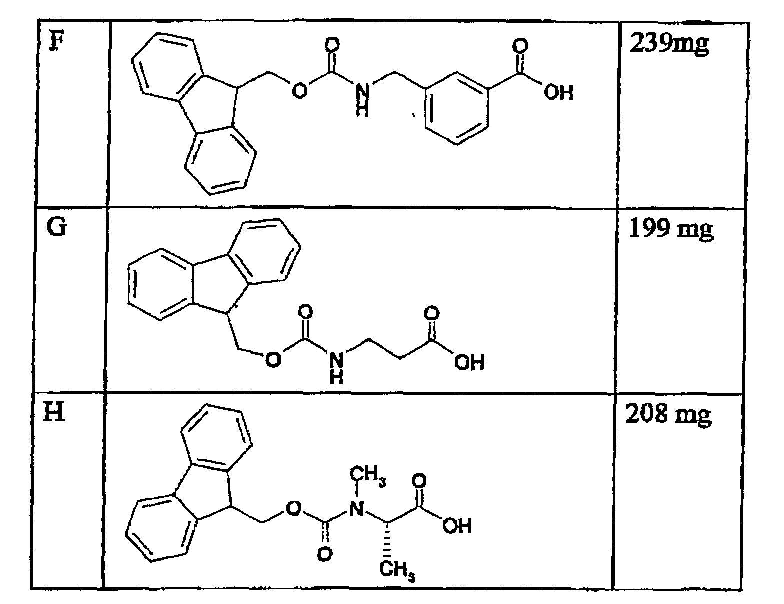

### General Procedure for the Acylation Step with acids

To each of 4 vessels containing 16 Irori MicroKan™ containers (each container containing 10 mg, 0.004 mmol resin 9) in DMF (30 ml) was added a solution of PyBOP (333 mg, 0.64 mmol), DIPEA (224 µl, 1.28 mmol) and one of the acids (see table 3 for amounts) in DMF (5 ml) and the mixture shaken for 16 h. The vessels were then drained and the MicroKan™ containers washed with DMF, DCM, DMF, DCM, and Et2O (each 3 times).

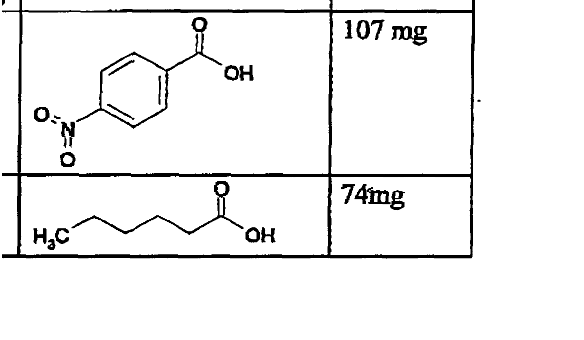

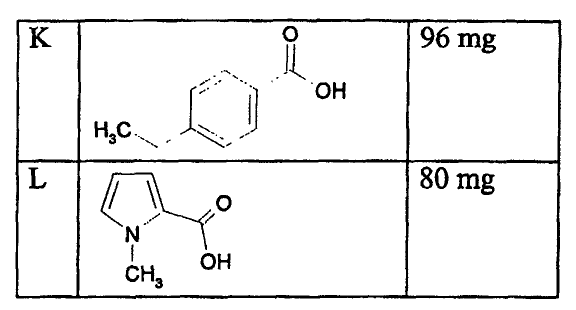

### General Procedure for the analytical cleavage of resin 10

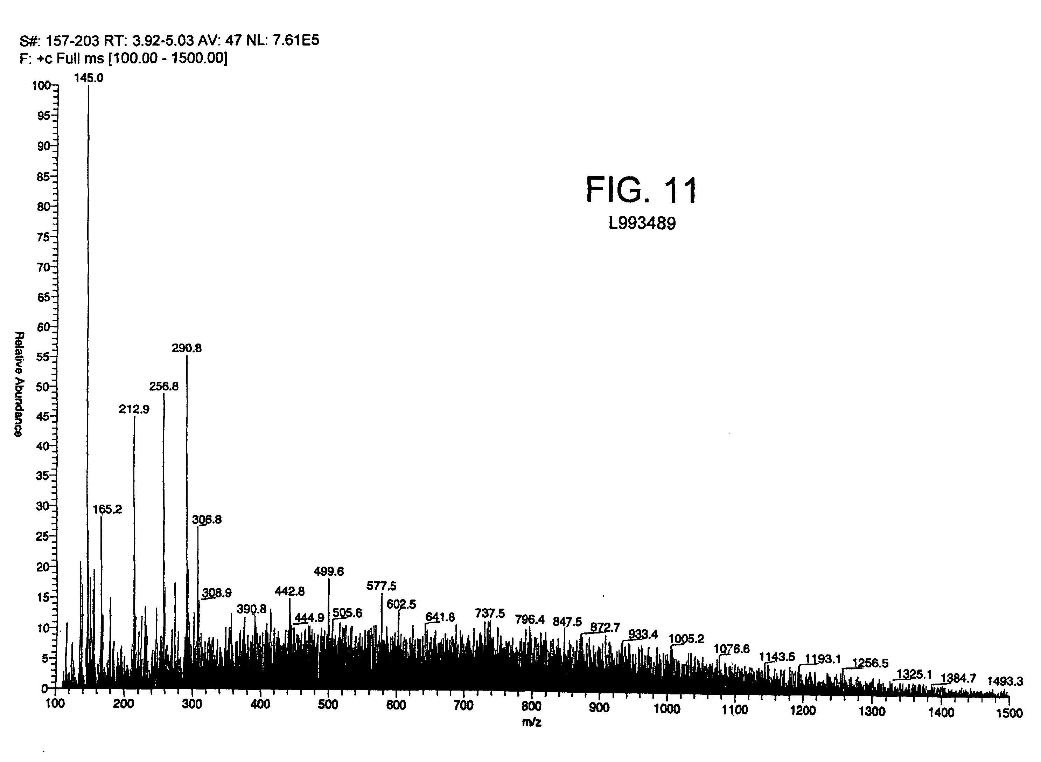

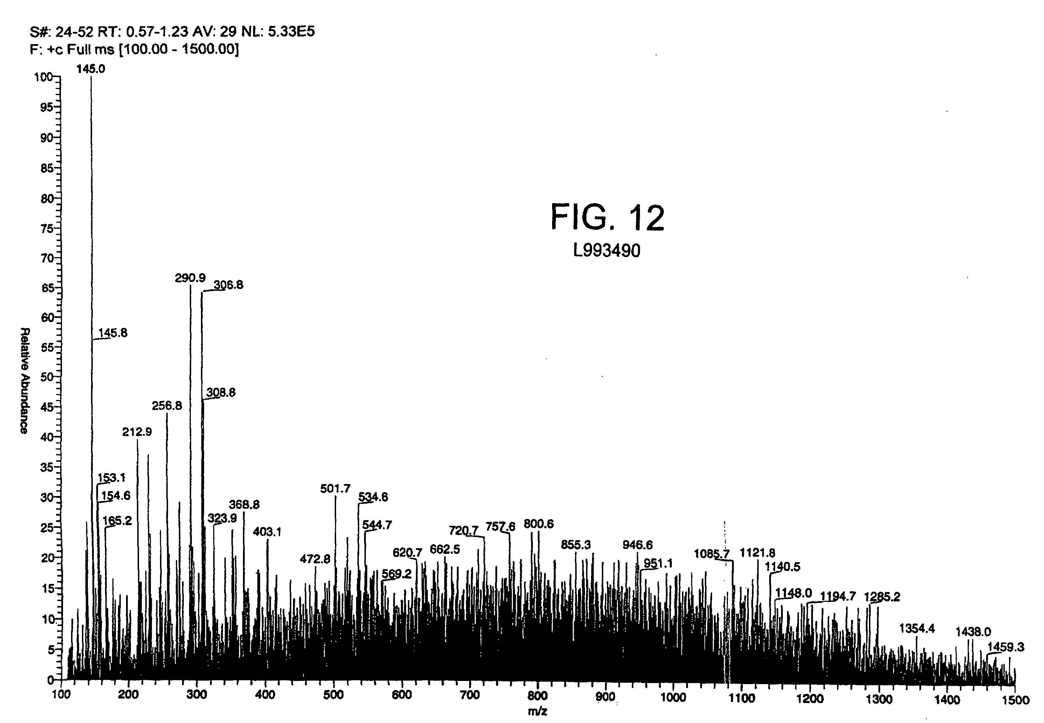

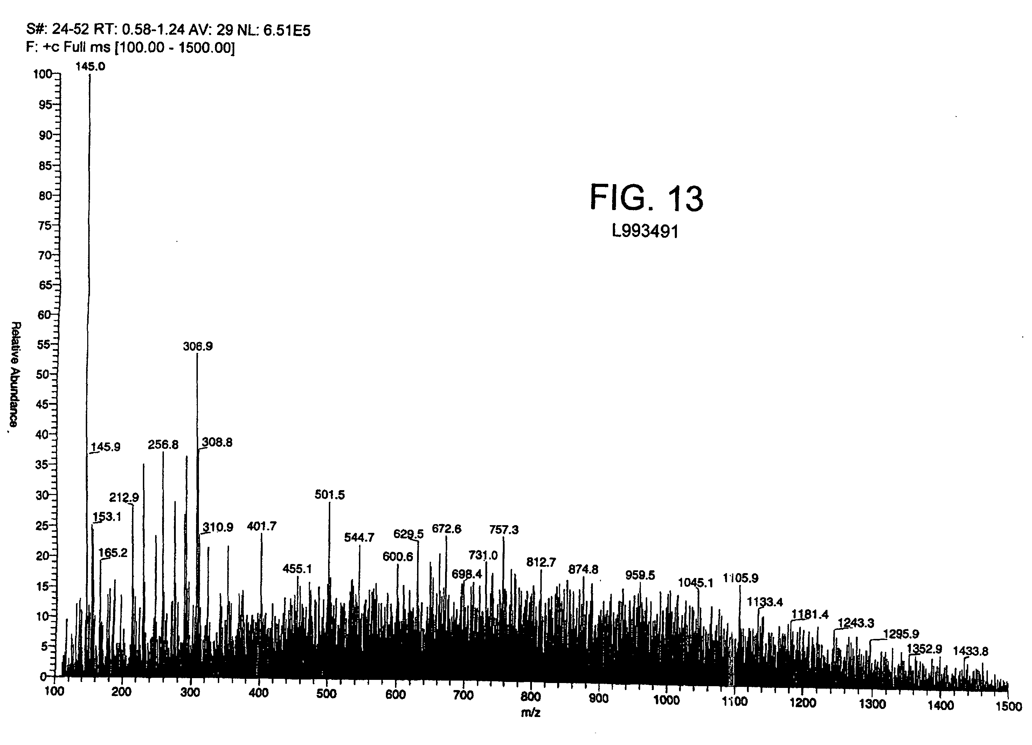

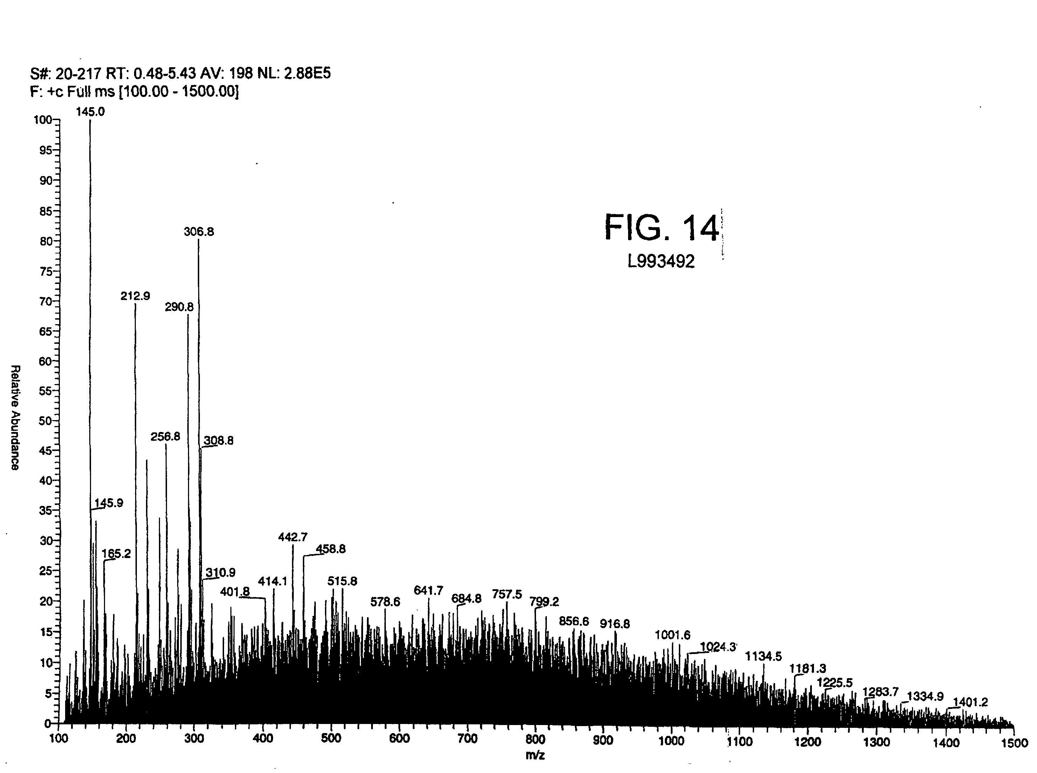

The resin 10 is treated with a 10% solution of *meta*-chloro-perbenzoic acid (mcpba) in DCM for 4 h. The solution is then removed by filtration and the beads washed with DCM. A single bead from each 64 MicroKan™ container is then treated with a 10% solution of 1-methylpiperazine in DMSO and left to stand for 16 h. The resulting cleaved solution is then subjected to analysis by electrospray mass spectrometry. The mass spectra for the cleaved analytical constructs are shown in the spectra identified in the column headed "Construct Cleavage" in the table below. The mass spectra for the first four monomer combinations are shown in Figures 11 to 14.

### General Procedure for the photocleavage of resin 10

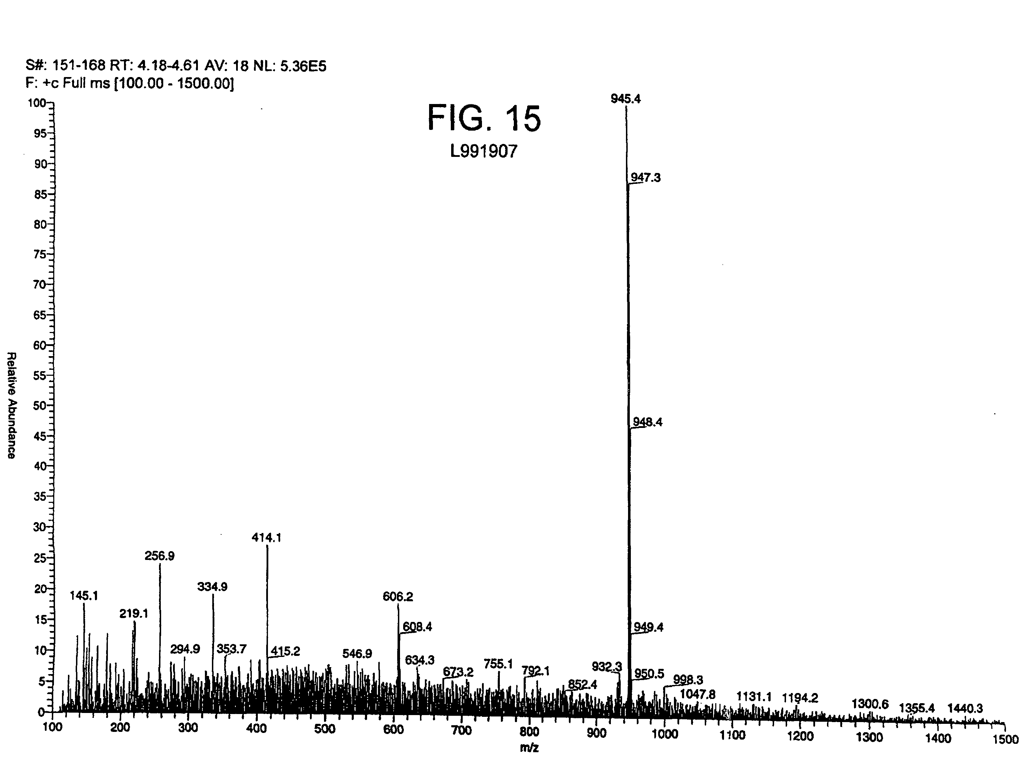

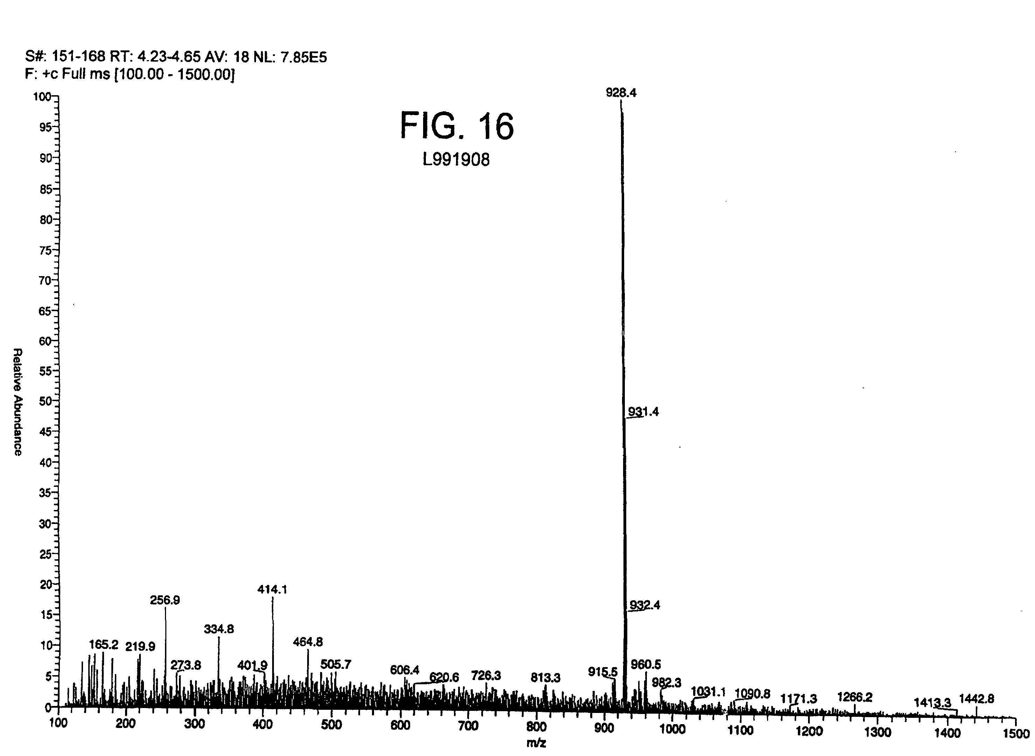

A single bead from the each 64 MicroKan™ container is swelled in DMSO (containing 0.2% H₂NNH₂) and then subjected to photolysis for 4 h and the solution subjected to analysis by electrospray mass spectrometry. The mass spectra of the photolysis cleavage products of the library are shown in the spectra identified in the column headed "UV Cleavage" in the table below. The mass spectra for the first four monomer combinations are shown in Figures 15 to 18.

### Results

| **Monomer Combinations** | | | **Mass Spec ID** | |
|---|---|---|---|---|
| | | | **UV Cleavage** | **Construct Cleavage** |
| A | I | E | L993489 | L991907 |
| A | I | F | L993490 | L991908 |
| A | I | G | L993491 | L991909 |
| A | I | H | L993492 | L991910 |
| A | J | E | L993493 | L991911 |
| A | J | F | L993494 | L991912 |
| A | J | G | L993495 | L991913 |
| A | J | H | L993496 | L991914 |
| A | K | E | L993497 | L991915 |
| A | K | F | L993498 | L991916 |
| A | K | G | L993499 | L991917 |
| A | K | H | L993500 | L991918 |
| A | L | E | L993501 | L991919 |
| A | L | F | L993502 | L991920 |
| A | L | G | L993503 | L991921 |
| A | L | H | L993504 | L991922 |
| B | I | E | L993505 | L991923 |
| B | I | F | L993506 | L991924 |
| B | I | G | L993507 | L991925 |
| B | I | H | L993508 | L991926 |
| B | J | E | L993509 | L991927 |
| B | J | F | L993510 | L991928 |
| B | J | G | L993511 | L991929 |
| B | J | H | L993512 | L991930 |
| B | K | E | L993513 | L991931 |
| B | K | F | L993514 | L991932 |
| B | K | G | L993515 | L991933 |
| B | K | H | L993516 | L991934 |
| B | L | E | L993517 | L991935 |
| B | L | F | L993518 | L991936 |
| B | L | G | L993519 | L991937 |
| B | L | H | L993520 | L991938 |
| C | I | E | L993521 | L991939 |
| C | I | F | L993522 | L991940 |
| C | I | G | L993523 | L991941 |
| C | I | H | L993524 | L991942 |
| C | J | E | L993525 | L991943 |
| C | J | F | L993526 | L991944 |
| C | J | G | L993527 | L991945 |
| C | J | H | L993528 | L991946 |
| C | K | E | L993529 | L991947 |
| C | K | F | L993530 | L991948 |
| C | K | G | L993531 | L991949 |
| C | K | H | L993532 | L991950 |
| C | L | E | L993533 | L991951 |
| C | L | F | L993534 | L991952 |
| C | L | G | L993535 | L991953 |
| C | L | H | L993536 | L991954 |
| D | I | E | L993537 | L991955 |
| D | I | F | L993538 | L991956 |
| D | I | G | L993539 | L991957 |
| D | I | H | L993540 | L991958 |
| D | J | E | L993541 | L991959 |
| D | J | F | L993542 | L991960 |
| D | J | G | L993543 | L991961 |
| D | J | H | L993544 | L991962 |
| D | K | E | L993545 | L991963 |
| D | K | F | L993546 | L991964 |
| D | K | G | L993547 | L991965 |
| D | K | H | L993548 | L991966 |
| D | L | E | L993549 | L991967 |
| D | L | F | L993550 | L991968 |
| D | L | G | L993551 | L991969 |
| D | L | H | L993552 | L991970 |

As can be seen from the mass spectra shown in Figures, when the constructs are cleaved photolytically at the second cleavage site to release substrate, the absence of any readily ionisable group on the substrate means that the mass spectrum does not show a strong molecular ion and hence identification of the substrate is difficult. However, when the constructs to the bead are cleaved at the first cleavage site to release an analytical fragment containing the substrate and sensitising and peak splitting groups, a strong characteristic and readily identifiable split molecular ion is produced, thereby enabling the substrate to be identified. In most cases, the identity of the substrate from the molecular ion in the mass spectrum correlated with the identity predicted from the known synthesis history of the resin beads in each MicroKan™. However, in certain of the reactions, the substrates formed oxidation products under the cleavage conditions used to unlock the safety catch linker but such oxidation products could be readily identified as M+16 ions in the mass spectrum. Thus a further advantage of the constructs of the invention is that it provides a means of determining when a particular reaction has not proceeded according to plan.

The dual linker constructs of the invention allow cleavage to take place either to release an analytical construct containing a mass spectrometric sensitising group, or to release substrate. Thus, the constructs enable QC procedures to be carried out at the single bead level in a manner that would not have been possible if the constructs had contained only a single linker and no mass spectrometric sensitising group.

### EXAMPLE 7

### Preparation of a Construct Containing an Alloc Linker at the First Cleavage site

An example of a construct containing an allyloxycarbonyl ("Alloc") linker at the first cleavage site and an acid labile Rink linker at the second cleavage site was prepared according to the reaction scheme shown in Scheme 9. In this example, palladium (0) is used to cleave the allyloxy carbonyl group from the amino group of the diamino ethane group so as to release a free amino group that can then function as a mass spectrometric sensitising group.

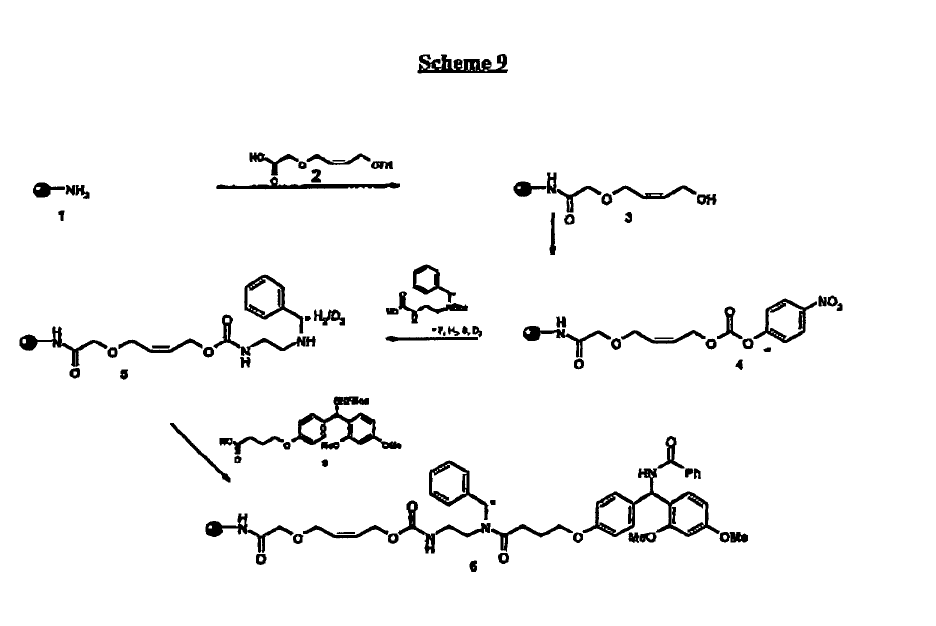

### Experimental for Scheme 9

### General

In this section, the compound numbers refer to the compound numbers in Scheme 9.

All washings were carried out fivefold with the quoted solvents. Standard washing procedure A: DMF, DCM, DMF, DCM, ET2O, DCM and Et₂O, all five fold each.

Commercially available acid **2** (65 mg, 0.115 mmol) (Neosystems) was dissolved in DMF (0.5ml) and to this added DIPEA (40 µl, 0.23 mmol) followed by HATU (44 mg, 0.115 mmol). This mixture was allowed to stand for 5 mins and then added to commercially available Argogel NH2 (Resin 1) (50 mg, 0.023mmol), having an average bead diameter of 150 µm and a loading of 0.46 mmol g⁻¹, and shaken for 2 h. The resin was then drained and washed according to standard washing procedure A and then dried *in vacuo*. The resin was then treated with a solution of DCM, TFA and TES (1 ml, 95:2:5 respectively) for 2h and then washed and drained as above and then this treatment with DCM, TFA and TES was repeated for 1 h. The resin was washed and drained as according to standard washing procedure A to give resin **3**.

Resin **3** (50 mg, 0.023 mmol) was then treated with a solution of 4-nitrophenylchloroformate (23mg, 0.115 mmol) in DCM (1 ml) and shaken for 16h. The resin was then quickly drained and washed with DCM and Et2O to give resin **4.** To this was added directly a solution of amines **7** (50 mg, 0.2mmol) and **8** (50 mg, 0.2mmol) and DIPEA (70 µl, 0.4 mmol) in DCM (1ml) and shaken for 16 h. The resin was then drained and washed according to standard washing procedure A and then dried in vacuo. This resin was then treated with a solution of 20% TFA in DCM (1 ml) and shaken for 2h. The resin was drained, washed with a solution of 10% DIPEA in DMF (2 ml) and then drained and washed according to standard washing procedure A and then dried in vacuo to give resin 5.

Rink acid **9** (65 mg, 0.115 mmol) was dissolved in DMF (0.5 ml) and DIPEA (40 µl, 0.23 mmol) and HATU (44mg, 0.115 mmol) added and this mixture allowed to stand for 5 min. This solution was then added to resin 5 (25 mg, 0.012 mmol) and shaken for 2h.. The resin was washed and drained as according to standard washing procedure A and dried in vacuo. This resin was then treated with a 20% solution of piperidine in DMF (1 ml) for 1 h. The resin was washed and drained as according to standard washing procedure A and dried in vacuo. Benzoic acid (14 mg, 0.115 mmol) was dissolved in DMF (0.5 ml) and DIPEA (40 µl, 0.23 mmol) and HATU (44mg, 0.115 mmol) added and this mixture allowed to stand for 5 min. This solution was then added to the resin and shaken for 2h. The resin was washed and drained as according to standard washing procedure A and dried in vacuo to give resin 6

### Cleavage of Resin 6: Method 1

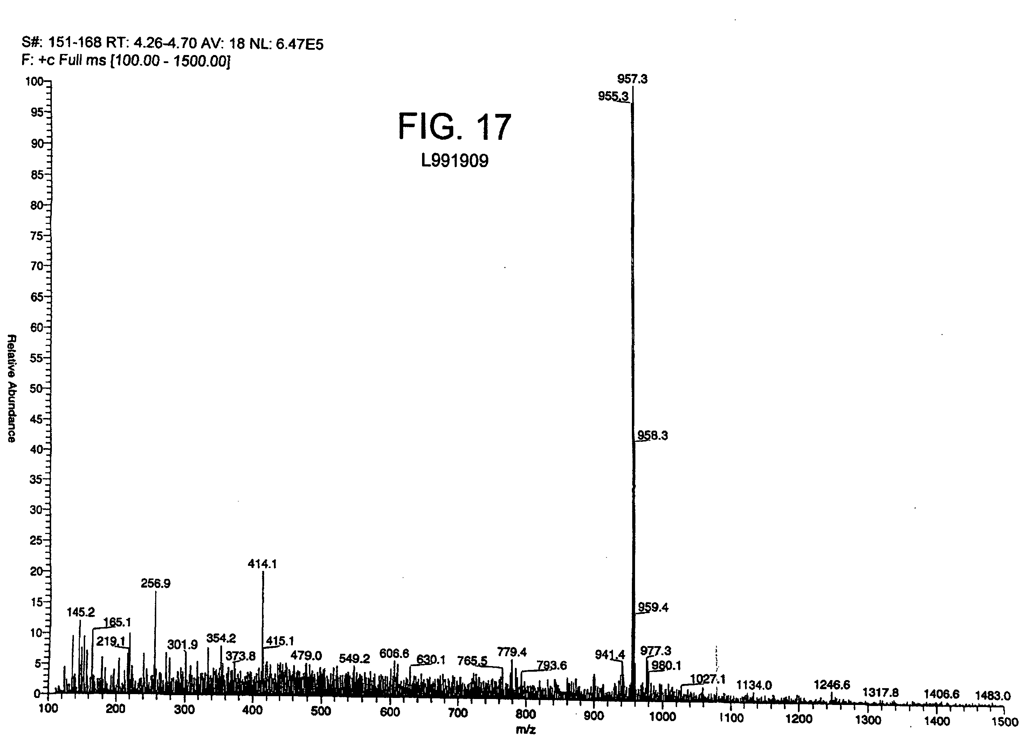

Resin 6 (0.5 mg) was swelled in DCM and then (Ph₃P)₄Pd(0) (2.7 mg, 2.3E⁻³ mmol) added followed by morpholine (10ml, 0.11 mmol) and the mixture shaken for 1 h. A sample of the liquid was removed and analysed by electrospray mass spectrometry to give an analysis as shown in figure 17. As with the previous examples, the presence of a mass spectrometric sensitising group and a peak splitting group enables a strong molecular ion to be formed and to be readily identified.

### Cleavage of Resin 6: Method 2

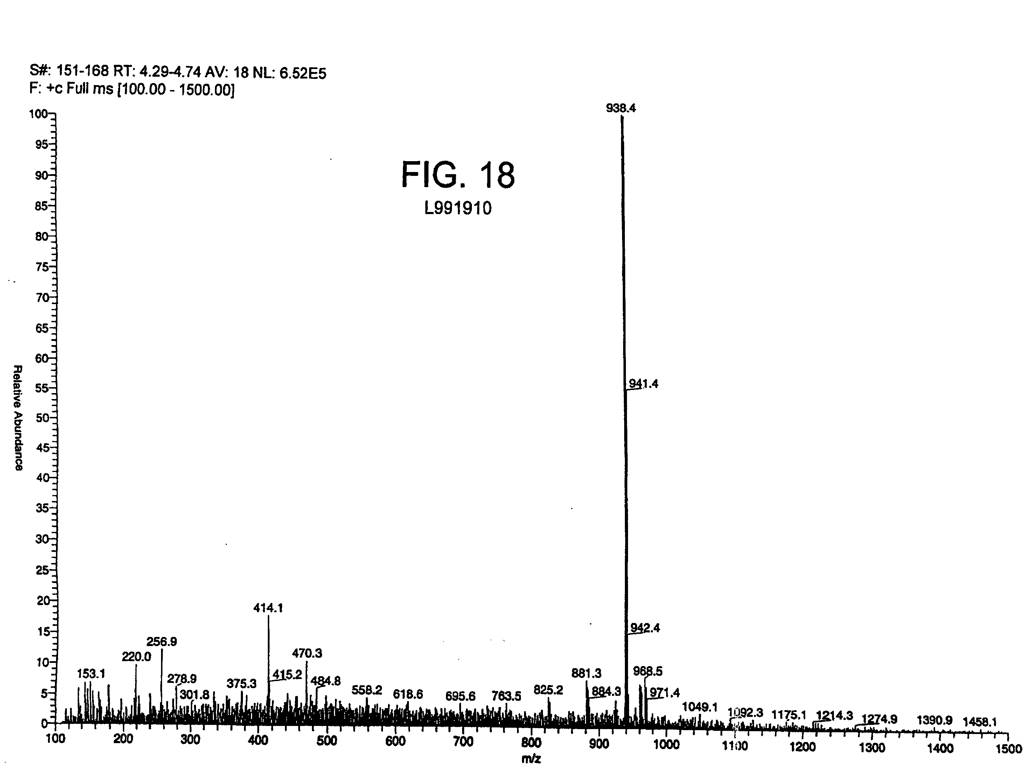

Resin 6 (0.5 mg) was swelled in a mixture of DMSO, THF and 0.5M HCI (0.5 ml, 2:2:1 respectively) and then (Ph₃P)₄Pd(0) (2.7 mg, 2.3E⁻³ mmol) added followed by morpholine (10ml, 0.11 mmol) and the mixture shaken for 1 h. A sample of the liquid was removed and analysed by electrospray mass spectrometry to give an analysis as shown in figure 18.

### EXAMPLE 8

### Preparation of a Construct Containing an Anthracenyl UV Chromophore

In order to demonstrate that UV spectrometry can be used to provide accurate quantitative information about the products of solid phase reactions, a construct containing an anthracenyl UV chromophore was prepared according to the synthetic route shown in Schemes 10, 11 and 12 below. The construct was provided with a sulphonamide linker at the first cleavage site and a Rink linker at the second cleavage site, an ethylene diamine chain connecting the two linkers and the two nitrogen atoms of the diamine chain serving as the points of attachment of the UV chromophore and either a peak splitting deuterium labelled benzyl group or an unlabelled benzyl group.

Scheme 10 below illustrates the synthesis of a non-resin bound intermediate construct, Scheme 11 illustrates the attachment of the construct of Scheme 10 to a resin support and subsequent reactions on the resin, and Scheme 12 illustrates the attachment of different substrate groups to the constructs and subsequent cleavage experiments. In the experimental section below, the compound numbers used relate to the compounds identified in Schemes 10, 11 and 12.

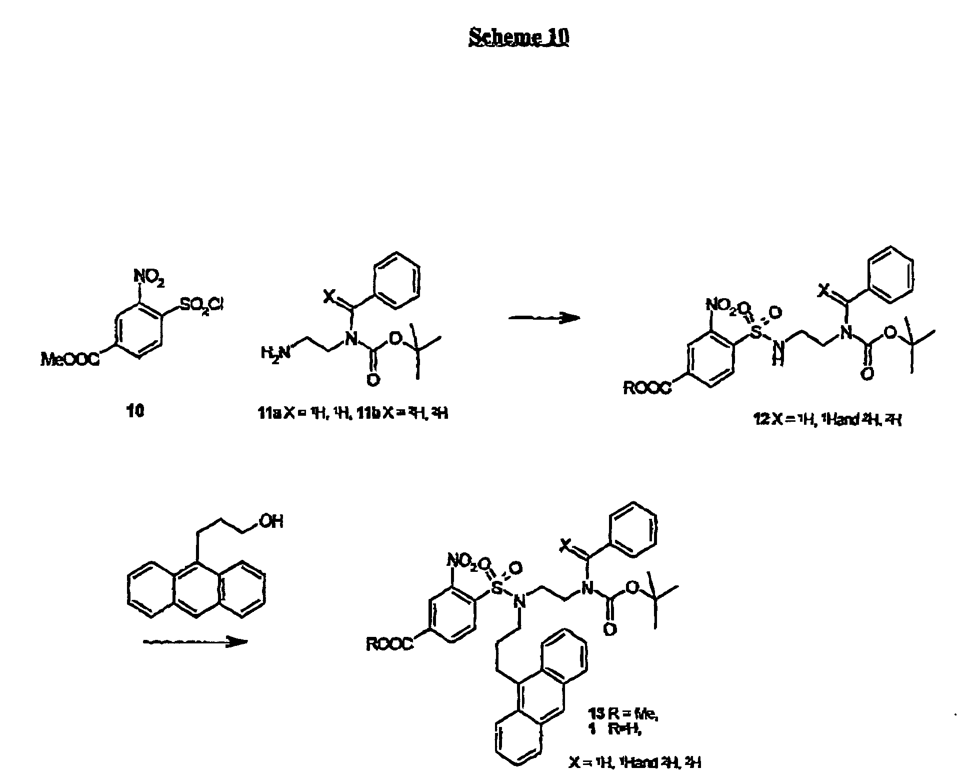

### Experimental for Scheme 10

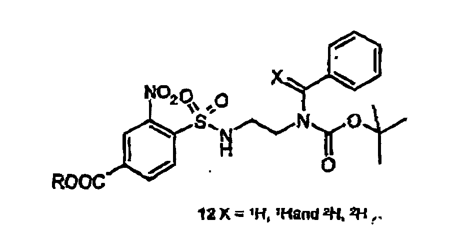

### Preparation of Compound 12 (X = ¹H, ¹H)

Dry triethylamine (25 µl, 0.2 mmol) was added to a solution of the amine (X=H, H) (**11**) (0.05 mmol) in dry dichloromethane (1 ml). A solution of 2-nitro-4-methylbenzoatesulfonyl chloride **(10)** (25 mg, 0.1 mmol) in dry dichloromethane (0.5 ml) was then added dropwise and the mixture stirred for 15 minutes. The solvent was evaporated *in vacuo* and the residue purified by flash chromatography using 2:1 hexane/ethyl acetate as eluent to afford the sulphonamide **(12)** (23.3 mg, 95%) as a colourless oil, R_{f} 17/50 [hexane-ethyl acetate (2:1)]; (Found: C, 53.7; H, 5.4; N, 8.4; S, 6.5. C₂₂H₂₇N₃O₈S requires C, 53.5; H, 5.5; N, 8.5; S, 6.5%); δ_{H} (DMSO-d⁶) 1.38 (9H, s, H-18), 3.10 (2H, t, *J* 8, H-9), 3.24 (2H, t, *J* 8, H-10), 3.95 (3H, s, O-Me), 4.32 (2H, s, H11), 7.13-7.32 (5H, m, H-13, 14 and 15), 8.09 (1H, d, *J* 8, H-6), 8.29 (1H, s, H-3), 8.32 (1H, d, *J* 8, H-3); δ_{C} (DMSO-d⁶) 28.3 (C-18), 40.2 (C-9 and 10), 46.2 (C-11), 53.2 (O-Me), 79.3 (C-17), 125.1 (C-13), 127.2 (C-14 and 15), 128.6 (C-6), 130.3 (C-13), 133.1 (C-5), 134.4 (C-12), 136.6 (C-1), 147.6 (C-4), 163.7 (C-16 and C=O); MS (Electrospray -ve) m/z 494 (M-H)⁻; HPLC, Rₜ 6.8 min.

### Preparation of Compound 12 ( X = ²H, ²H)

Dry triethylamine (25 µl, 0.2 mmol) was added to a solution of the amine (X=²H, ²H) (**11**) (0.05 mmol) in dry dichloromethane (1 ml). A solution of 2-nitro-4-methylbenzoatesulfonyl chloride (10) (25 mg, 0.1 mmol) in dry dichloromethane (0.5 ml) was then added dropwise and the mixture stirred for 15 minutes. The solvent was evaporated in *vacuo* and the residue purified by flash chromatography using 2:1 hexane/ethyl acetate as eluent to afford the sulphonamide (**12**) (23.5 mg, 94%) as a colourless oil, R_{f} 17/50 [hexane-ethyl acetate (2:1)]; (Found: C, 53.2; H, 5.6; N, 8.5; S, 6.4. C₂₂H₂₅D₂N₃O₈S requires C, 53.3; H, 5.5; N, 8.5; S, 6.5%); δ_{H} (DMSO-d⁶) 1.40 (9H, s, H-18), 3.05 (2H, m, H-9), 3.20 (2H, dt, *J*3 and 13, H-10), 3.9 (3H, s, O-Me), 7.10-7.35 (5H, m, H-13, 14 and 15), 8.09 (1H, d, *J* 8, H-6), 8.35 (1H, d, *J* 8, H-5), 8.40 (1H, m, H-8), 8.45 (1H, s, H-3),; δ_{C} (DMSO-d⁶) 27.8 (C-18), 40.9 (C-9 and 10), 45.9 (C-11), 53.0 (O-Me), 79.1 (C-17), 124.8 (C-13), 127.0 (C-14 and 15), 128.3 (C-6), 130.0 (C-13), 132.9 (C-5), 134.2 (C-12), 136.4 (C-1), 147.5 (C-4), 154.7 (C-2), 163.5 (C-16 and C=O); MS (Electrospray +ve) m/z 496 (MH)⁺; HPLC, Rₜ 6.8 min.

### Preparation of Compounds 13 and 1

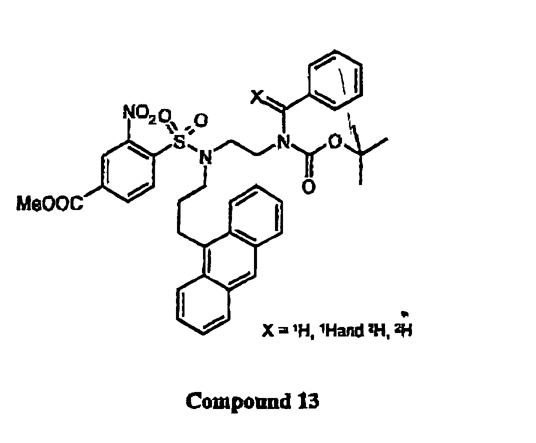

Di-*tert*-butylazodicarboxylate (32.6 mg, 142 µmol) was dissolved in dry tetrahydrofuran (0.5 ml) under nitrogen and added dropwise to a stirred solution of triphenylphosphine (37.1 mg, 142 µmol), 3-(9-anthracene)propanol (17.6 mg, 74 µmol) and sulphonamide **12** (X=H, H and ²H, ²H) (35 mg, 71 µmol), dissolved in dry tetrahydrofuran (1 ml) under nitrogen. After one hour the reaction was deemed complete by tlc and the solution was evaporated to near-dryness. The residue was purified by flash chromatography 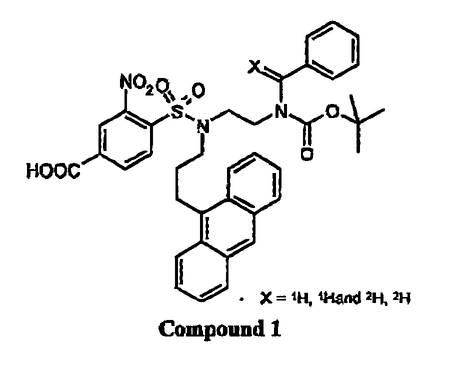 using 2:1 hexane/ethyl acetate containing 10% triethylamine to afford the sulphonamide **13** (45 mg, 90%) as a colourless oil, R_{f} 15/38 (hexane-ethyl acetate (2:1)); MS (Electrospray +ve) m/z 612, 614 (MH-Boc)⁺, MS (Electrospray -ve) m/z 756, 758 (M-H+formate) HPLC, Rₜ 6.9 min. 2M aqueous sodium hydroxide (0.35 ml, 0.7 mmol) was added dropwise to a solution of the ester **13** (0.25 g, 0.35 mmol) in methanol-1,4-dioxane (4:1) (2 ml). After stirring at room temperature for 1 hour (further methanol-dioxane (ca. 1 ml) was added as necessary to ensure a single phase) sufficient 2M HCl added to render the solution mildly acidic. The solution transferred to a separating flask containing ethyl acetate (50 ml) and water (10 ml) and after partition the aqueous layer was extracted again with ethyl acetate (25 ml). The combined organic extracts were dried (MgSO₄) and evaporated under reduced pressure to afford the acid **1** (0.23 g, 94%) as a colourless oil; R_{F} 2/40 [hexane-ethyl acetate (2:1)]; δ_{H} (CDCl₃) 1.40 (9H, br. d, *t*Bu), 1.90 (2H, br. m, CH₂), 3.3-3.7 (8H, br. m, 4xCH₂), 7.10-8.30 (17H, br. m, Arom. H); MS (electrospray +ve) m/z 697, 699 HPLC, R_{f} 7.2 min.

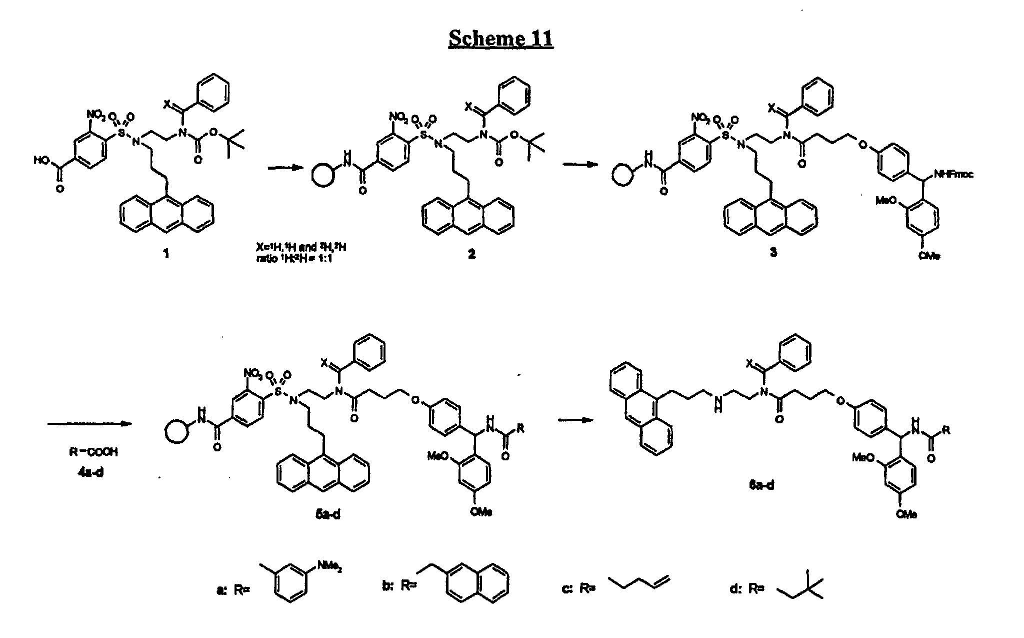

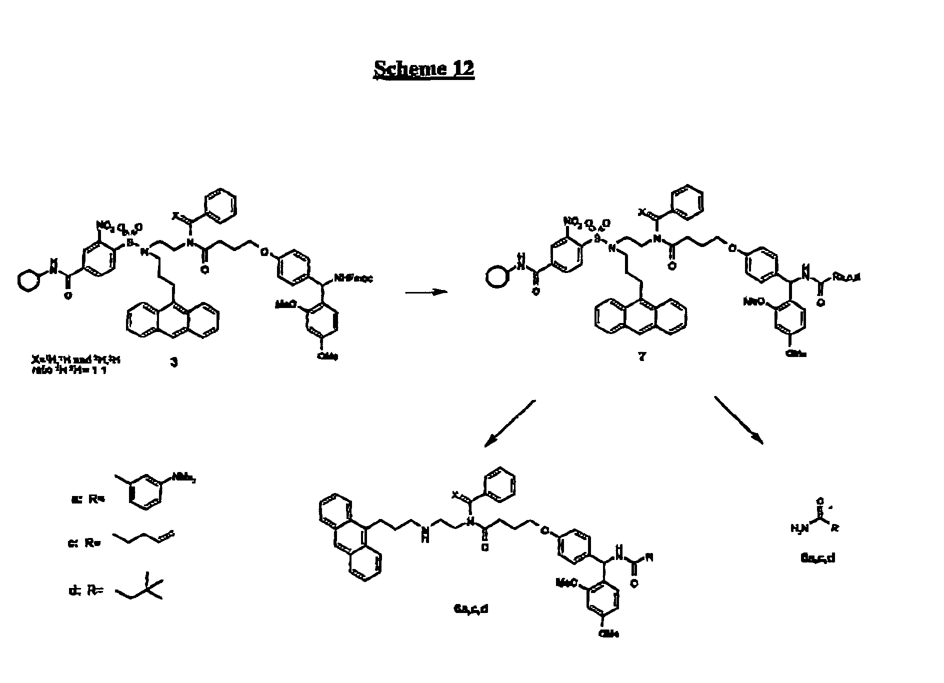

### Experimental for Schemes 11 and 12

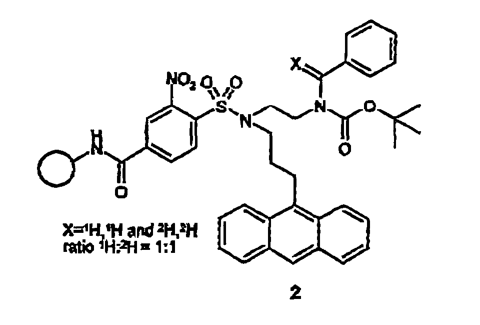

Dry aminomethyl Argogel® (0.87 g, 0.35 mmol)), having an average bead diameter of 150 µm and a loading of 0.4 mmol g⁻¹, in a reaction vessel flushed with dry nitrogen was swollen with dry dichloromethane (5 ml). In a separate flask flushed with nitrogen, the benzoic acid (**1**) (0.67 g, 0.93 mmol), PyBOP (0.728 g, 1.4 mmol) and 1-hydroxy-benzotriazole (0.19 g, 1.4 mmol) were dissolved in dry dimethylformamide (2 ml)and the resulting solution stirred two minutes before being transferred to the suspension of resin *via cannula*. A slow stream of nitrogen was aspirated through the solution for five minutes to ensure mixing and diisopropylethylamine (0.49 ml, 2.8 mmol) then added. Agitation was continued for 18 h. whereupon the resin (**2**) was drained, washed (dichloromethane (3x7 ml), dimethylformamide (3x7 ml) and dichloromethane (3x7 ml)) and dried *in vacuo*. An analytical sample of **2** gave a negative Kaiser test.

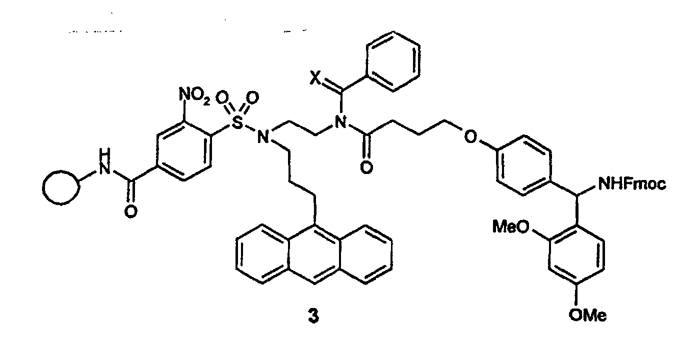

Resin 2 (0.35 mmol) was swollen in dichloromethane (3 ml) and phenol (66 mg, 0.7 mmol) added, followed by trifluoroacetic acid (4.5 ml). The resin was gently agitated for 5 minutes using a stream of nitrogen then drained and washed with dichloromethane (2x5 ml). The process was repeated and, after draining, the resin washed (dichloromethane (3x8 ml), 10% diisopropylamine in dichloromethane (2x6 ml) and dichloromethane (4x8 ml)) and dried *in vacuo*. An analytical sample gave a positive Kaiser test.

The resin, in a reaction vessel flushed with nitrogen, was swollen with dry dichloromethane (5 ml). In a separate flask flushed with nitrogen C-4 RINK acid (0.795 g, 1.4 mmol), PyBOP (1.46 g, 2.8 mmol) and 1-hydroxybenzotriazole (0.378 g, 2.8 mmol) were dissolved in dry dimethylformamide (3 ml) and the resulting solution transferred to the suspension of resin *via cannula*. This mixture was agitated gently by aspirating a stream of nitrogen through the solution and after five minutes dry diisopropylethylamine (0.98 ml, 5.6 mmol) was added. Agitation was continued on a mechanical shaker for 18 h. whereupon the resin (**3**) was drained and washed (dichloromethane (3x8 ml), dimethylformamide (3x5 ml) and dichloromethane (3x5 ml)) and dried *in vacuo*. An analytical sample gave a negative Kaiser test.

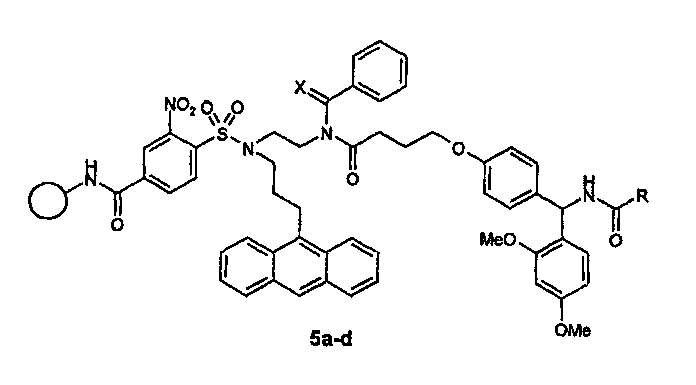

A sample of resin **3** (50 mg, 14 µmol) was treated with 20% v/v piperidine in 1:1 dichloromethane/dimethylformamide (2 ml) for 15 minutes. The resin was drained and washed (dichloromethane (3x2 ml), dimethylformamide (3x2 ml) and dichloromethane (3x2 ml)) then dried *in vacuo*, an analytical sample giving a positive bromophenol blue test. The reaction vessel containing the resin was flushed with nitrogen and dry 1:1 dichloromethane /dimethylformamide (0.5 ml) added. In a separate flask flushed with nitrogen PyBOP (57 mg, 110 µmol), 1-hydroxybenzotriazole (15 mg, 110 µmol) and the appropriate acid **(4a-d)** (55 µmol) were dissolved in dry 1:1 dichloromethane-dimethylformamide (0.5 ml) and this solution added in one portion to the suspension of resin, through which a stream of nitrogen was aspirated to ensure uniform mixing. After agitating for 18 h. the resin was drained, washed (dichloromethane (3x2 ml), dimethylformamide (4x2 ml) and dichloromethane (3x2 ml)) and dried *in vacuo* to give resins **5a-d.** An analytical sample of each gave a negative Kaiser test.

A small sample of each of the four resins was treated with DBU/ mercaptoethanol in acetonitrile to release **6a-d** which were analysed by HPLC and low-resolution mass spectrometry:
**6a** (R = 3-dimethylaminobenzoyl) HPLC Rₜ 4.51 min., MS (electrospray +ve) *m*/*z* 843.7 and 845.7;
**6b** (R = 2-naphthylacetoyl) HPLC Rₜ 5.41 min., MS (electrospray +ve) *m*/*z* 863.4 and 865.4;
**6c** (R = 4-pentenoyl) HPLC Rₜ 4.98 min., MS (electrospray +ve) *m*/*z* 778.7 and 780.7;
**6d** (R = *tert*-Butylacetoyl) HPLC Rₜ 5.18 min., MS (electrospray +ve) *m*/*z* 795.0 and 797.0.

### Relative quantitation.

Approximately equal quantities (ca. 50 mg) of resins **5a** and **5b** were combined and treated with 4-mercaptobenzoic acid (43 mg, 20 µmol) and morpholine (49 µmol) in dichloromethane (2 ml). After one hour the solution was drained and the resin washed with dichloromethane (2x1 ml). These combined washings were diluted with ethyl acetate (10 ml) and washed firstly with 50% saturated aqueous sodium bicarbonate (3 ml), water (3 ml) and brine (2 ml) and dried over magnesium sulphate. The filtered solution was evaporated to afford approximately 5 mg of a mixture of **6a** and **6b**. The ratio of components in this mixture was assessed firstly by HPLC, measuring peak areas at 254 nm and 386 nm, which was correlated with the ratio determined from the magnitude of the integrations of characteristic resonances in the ¹H NMR spectrum.

| **HPLC analysis** | | |
|---|---|---|
| | **6a** | **6b** |
| Area ratio (254 nm) | **1.00** | **1.30** |
| Area ratio (386 nm) | **1.00** | **1.26** |

| ^{**1**}**H nmr analysis** | | |
|---|---|---|
| | **6a** | **6b** |
| Ratio | **1.00** | **1.33** |

In the same way a mixture of resins **5c** and **5d,** afforded a mixture of **6c** and **6d,** the ratio of which were again assessed by HPLC and correlated with the ratios measured using ¹H nmr spectroscopy.

| **HPLC analysis** | | |
|---|---|---|
| | **6c** | **6d** |
| Area ratio (254 nm) | **1.00** | **0.93** |
| Area ratio (386 nm) | **1.00** | **0.97** |

| ^{**1**}**H nmr analysis** | | |
|---|---|---|
| | **6c** | **6d** |
| Ratio | **1.00** | **0.96** |

Finally, **6a, 6b, 6c** and **6d** were combined in one solution, which was analysed using HPLC and ¹H nmr as before:

| **HPLC analysis** | | | | |
|---|---|---|---|---|
| | **6a** | **6b** | **6c** | **6d** |
| Area ratio (254 nm) | **0.87** | **1.35** | **1.00** | **1.00** |
| Area ratio (386 nm) | **0.84** | **1.30** | **1.00** | **0.99** |

| ^{**1**}**H nmr analysis** | | | | |
|---|---|---|---|---|
| | **6a** | **6b** | **6c** | **6d** |
| Ratio | **0.85** | **1.34** | **1.00** | **1.05** |

Resin **3** (214 mg, 59 µmol) was treated with 20% v/v piperidine in 1:1 dichloromethane/dimethylformamide (3 ml) for 60 minutes. The resin was drained, washed (dichloromethane (3x3 ml), dimethylformamide (3x3 ml) and dichloromethane 93x3 ml)) and dried *in vacuo*, an analytical sample giving a positive bromophenol blue test. The reaction vessel was then flushed with nitrogen and the resin swollen with dry 1:1 dichloromethane/dimethylformamide(2 ml).

In a separate flask flushed with nitrogen PyBOP (245 mg, 470 µmol), 1-hydroxybenzotriazole (64 mg, 470 µmol) and a mixture of 4-pentenoic acid (6.0 µl, 59 µmol), 3-dimethylaminobenzoic acid (19.5 mg, 120 µmol) and *tert*-butylacetic acid (23 µl, 180 µmol) were dissolved in dry 1:1 dichloromethane/dimethylformamide (1 ml) and the solution added in one portion to the suspension of resin. A gentle stream of nitrogen was passed through the mixture and after five minutes diisopropylethylamine (165 µl, 940 µmol) was added. After agitating for 18 h. the resin (**7**) was drained, washed (dichloromethane (3x4 ml), dimethylformamide (4x4 ml) and dichloromethane (3x4 ml)) and dried *in vacuo*. An analytical sample of **7** gave a negative Kaiser test.

A small sample of the resin **7** was treated with DBU/ mercaptoethanol in acetonitrile to release a mixture of **6a, 6c** and **6d** the relative quantities of these components being assessed by HPLC, measuring the peak areas at 254 and 386 nm (table).

A single bead of **7** was then selected and cleaved in the same way. The ratio of the components was again measured using HPLC.

Approximately 200 mg of resin **7** (0.056 µmol) was swollen with dichloromethane and the excess solvent drained away. Phenol (10.5 mg, 0.112 µmol) was added followed by 80% trifluoroacetic acid in dichloromethane such that this added volume constituted one third of the total. After agitating for two hours the pale red solvent was drained into a round-bottomed flask and the resin washed with distilled dichloromethane (3x3 ml). The combined organics were evaporated to dryness to afford a mixture of the primary amides **8a**, **8c** and **8d**. The ratio of these components was assessed by measuring the magnitude of the integration of characteristic resonances in the ¹H nmr spectrum.

| **HPLC analysis (bulk beads)** | | | |
|---|---|---|---|
| | **6a** | **6c** | **6d** |
| Area ratio (254 nm) | **0.71** | **1.00** | **0.44** |
| Area ratio (386 nm) | **0.71** | **1.00** | **0.46** |

| **HPLC analysis (single bead)** | | | |
|---|---|---|---|
| | **6a** | **6c** | **6d** |
| Area ratio (254 nm) | **0.71** | **1.00** | **0.42** |
| Area ratio (386 nm) | **0.69** | **1.00** | **0.41** |

| ^{**1**}**H nmr analysis** | | | |
|---|---|---|---|
| | **8a** | **8c** | **8d** |
| Ratio | **0.74** | **1.00** | **0.42** |

The results set out in the tables above illustrate that there is a remarkably good correlation between the ratios obtained by the UV method and the ratios obtained by the well established and proven method of nmr analysis. Thus, the data demonstrate that UV detection and analysis can be used to provide a very accurate means of giving quantitative information about the reaction products of solid phase syntheses. Of particular significance is the fact that UV analysis has been shown to provide an accurate means of providing quantitative data even at the single bead level where the amounts of compound available for analysis are of the order of only 1 nanomole.

### EXAMPLE 9

### Preparation of a Construct Containing a Dansyl Group as a UV Chromophore

A second UV chromophore-containing construct was prepared, but this time including a dansyl group as the chromophore rather than the anthracenyl group. The sequence of steps leading to the construct is shown in Scheme 13 below. As can be seen from Scheme 13, the construct is analogous to the construct of Example 8 in that a sulphonamide linker group provides a first cleavage site to enable release of analytical fragment, and a Rink linker provides a second cleavage site to enable release of substrate. However, in this example, the chromophore, rather than being linked to the sulphonamide nitrogen, is attached to a lysine group positioned between the ethylene diamine chain and the Rink linker .

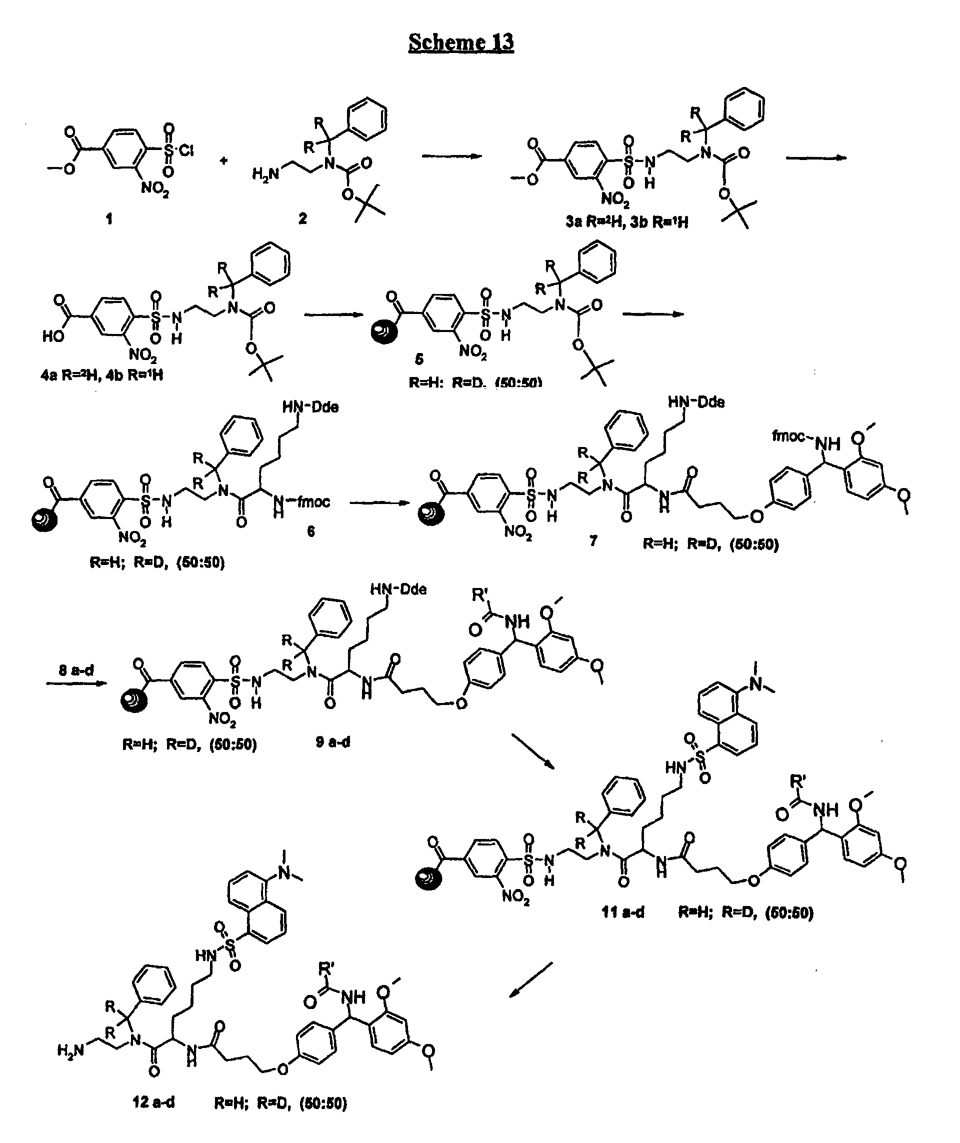

### Experimental for Scheme 13

In the following experimental details, the compound or construct numbers refer to the compounds and constructs identified in Scheme 13.

### Preparation of Compound 3a

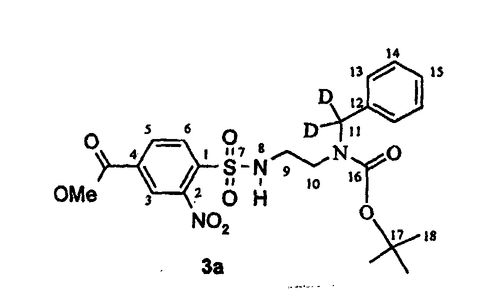

To a stirred solution of 2-nitro-4-methylbenzoatesulfonyl chloride **1** (1.10 g, 3.90 mmol) and triethylamine (0.39 g, 3.90 mmol) in dry dichloromethane (40 ml), was added at 0°C the amine **2a** (1.00 g, 3.9 mmol) in dry dichloromethane (20 ml) over 30 min. The reaction mixture was stirred for a further 2 h at room temperature then concentrated *in vacuo* to give a yellow oil. Purification by chromatography with [MTBE:hexane (1:1)] as eluant followed by recrystallisation from MTBE (5 ml) and hexane (2 ml) furnished ***the sulfonamide 3a*** (1.53 g, 79%) as white flakes; mp 129-130°C; R_{f} 0.31 [MTBE:hexane (1:1)]; (Found: C, 53.2; H, 5.6; N, 8.5; S, 6.4. C₂₂H₂₅D₂N₃O₈S requires C, 53.3; H, 5.5; N, 8.5; S, 6.5%); δ_{H} (DMSO-d⁶) 1.40 (9H, s, H-18), 3.05 (2H, m, H-9), 3.20 (2H, dt, *J* 3 and 13, H-10), 3.9 (3H, s, O-Me) 7.10-7.35 (5H, m, H-13, 14 and 15), 8.09 (1H, d, *J* 8, H-6), 8.35 (1H, d, *J* 8, H-5), 8.40 (1H, m, H-8), 8.45 (1H, s, H-3),; δ_{C} (DMSO-d⁶) 27.8 (C-18), 40.9 (C-9 and 10), 45.9 (C-11), 53.0 (O-Me), 79.1 (C-17), 124.8 (C-13), 127.0 (C-14 and 15), 128.3 (C-6), 130.0 (C-13), 132.9 (C-5), 134.2 (C-12), 136.4 (C-1), 147.5 (C-4), 154.7 (C-2), 163.5 (C-16 and C=O); MS (Electrospray +ve) m/z 496 (MH)⁺; HPLC (Method A), Rₜ 6.07 min.

### Preparation of Compound 4a

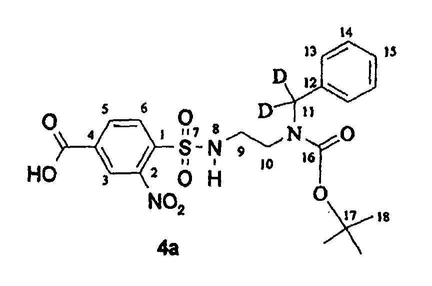

The ester **3a** (0.40 g, 0.80 mmol) in methanol (2 ml) was treated with 1M aqueous sodium hydroxide (1.60 ml, 1.60 mmol) and stirred at room temperature for 1 hour. The reaction was then concentrated *in vacuo* and the residue dissolved in water (10 ml) then acidified with 1M hydrochloric acid (1.80 ml, 1.80 mmol) at 0°C. The resulting white precipitate was extracted with dichloromethane (3 x 10 ml). The combined organic extracts were washed with brine, dried (Na₂SO₄) and evaporated under reduced pressure to ***the acid 4a*** (0.34 g, 88%) as a white solid; mp 143-145°C; R_{F} 0.58 [MTBE:hexane (1:1)]; δ_{H} (DMSO-d⁶) 1.29 (9H, s, H-18), 3.02 (2H, m, H-9), 3.20 (2H, dt, *J*3 and 13, H-10), 7.10-7.40 (5H, m, H-13, 14 and 15), 8.06 (1H, d, *J* 8, H-6), 8.28 (1H, s, H-3), 8.30 (1H, d, *J* 8, H-3); δ_{C} (DMSO-d⁶) 27.9 (C-18), 40.9 (C-9 and 10), 45.9 (C-11), 79.1 (C-17), 124.5 (C-13), 127.1 (C-14 and 15), 128.4 (C-6), 129.5 (C-13), 132.5 (C-5), 139.2 (C-12), 147.3 (C-1), 155.1 (C-4), 164.8 (C-16 and C=O); (Found: MH⁺, 482.156364 C₂₁H₂₃D₂N₃O₈S requires MH+, 482.156615); HPLC (Method A), Rₜ 5.65 min.

### Preparation of Compound 3b

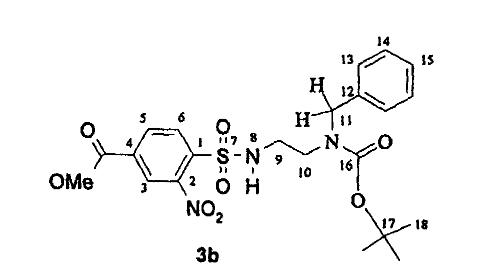

To a stirred solution of 2-nitro-4-methylbenzoatesulfonyl chloride **1** (1.10 g, 3.90 mmol) and triethylamine (0.39 g, 3.90 mmol) in dry dichloromethane (40 ml), was added at 0°C the amine **2b** (0.95 g, 3.9 mmol) in dry dichloromethane (20 ml) over 30 min. The reaction mixture was stirred for a further 3 h at room temperature then concentrated *in vacuo* to give a yellow oil. Purification by chromatography with [MTBE:hexane (1:1)] as eluant followed by recrystallisation from MTBE (5 ml) and hexane (2 ml) furnished ***the sulfonamide 3b*** (1.51 g, 79%) as white flakes; mp 131-132°C; R_{f} 0.28 [MTBE:hexane (1:1)]; (Found: C, 53.7; H, 5.4; N, 8.4; S, 6.5. C₂₂H₂₇N₃O₈S requires C, 53.5; H, 5.5; N, 8.5; S, 6.5%); δ_{H} (DMSO-d⁶) 1.38 (9H, s, H-18), 3.10 (2H, t, *J* 8, H-9), 3.24 (2H, t, *J* 8, H-10), 3.95 (3H, s, O-Me), 4.32 (2H, s, H11), 7.13-7.32 (5H, m, H-13, 14 and 15), 8.09 (1H, d, *J* 8, H-6), 8.29 (1H, s, H-3), 8.32 (1H, d, *J* 8, H-3); δ_{C} (DMSO-d⁶) 28.3 (C-18), 40.2 (C-9 and 10), 46.2 (C-11), 53.2 (O-Me), 79.3 (C-17), 125.1 (C-13), 127.2 (C-14 and 15), 128.6 (C-6), 130.3 (C-13), 133.1 (C-5), 134.4 (C-12), 136.6 (C-1), 147.6 (C-4), 163.7 (C-16 and C=O); MS (Electrospray -ve) m/z 492 (M-H)⁻; HPLC (Method A), Rₜ 5.99 min.

### Preparation of Compound 4b

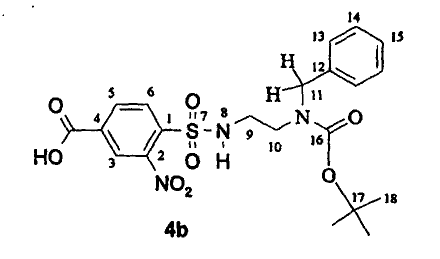

The ester **3b** (0.40 g, 0.80 mmol) in methanol (2 ml) was treated with 1M aqueous sodium hydroxide (1.60 ml, 1.60 mmol) and stirred at room temperature for 1 hour. The reaction was then concentrated *in vacuo* and the residue dissolved in water (10 ml) then acidified with 1M hydrochloric acid (1.80 ml, 1.80 mmol) at 0°C. The resulting white precipitate was extracted with dichloromethane (3 x 10 ml). The combined organic extracts were washed with brine, dried (Na₂SO₄) and evaporated under reduced pressure to ***the acid 4b*** (0.36 g, 93%) as a white solid; mp 123.7-124.8°C; R_{F} 0.59 [MTBE:hexane (1:1)]; δ_{H} (DMSO-d⁶) 1.42 (9H, s, H-18), 3.12 (2H, m, H-9), 3.22 (2H, m, H-10), 4.38 (2H, s, H11), 7.18-7.42 (5H, m, H-13, 14 and 15), 8.11 (1H, d, *J* 8, H-6), 8.39 (1H, d, *J* 8, H-3); 8.45 (1H, s, H-3); δ_{C} (DMSO-d⁶) 27.8 (C-18), 40.1 (C-9 and 10), 45.9 (C-11), 79.1 (C-17), 124.8 (C-13), 126.9 (C-14 and 15), 128.4 (C-6), 129.9 (C-13), 132.9 (C-5), 135.8 (C-12), 137.9 (C-1), 147.5 (C-4), 153.9 (C-2), 163.7 (C-16 and C=O); MS (Electrospray -ve) m/z 478 (M-H)⁻; HPLC (Method A), Rₜ 5.56 min.

### Preparation of Compound 5

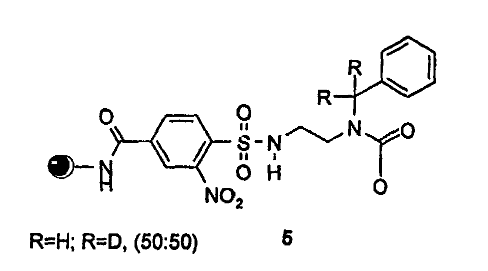

To a suspension of amine resin (Argogel-NH₂) (0.80 g, 0.32 mmol)), having a bead diameter of 150 µm and a loading of 0.4 mmol g⁻¹, in DMF (4 ml) and dichloromethane (4 ml) was added the benzoic acid (**4a**) (0.46 g, 0.96 mmol), and the benzoic acid (**4b**) (0.46 g, 0.96 mmol), followed by PyBOP (0.99 g, 1.92 mmol) and HOBT (0.26. g, 1.92 mmol) followed, 4 min. later by Hunig's base (0.49 g, 3.84 mmol). The reaction was agitated with nitrogen for 18 h and the resin then washed with dichloromethane (6 x 10 ml), DMF (6 x 10 ml), dichloromethane (6 x 10 ml), ether (2 x 10 ml) and dried *in vacuo* to give the ***sulfonamide resin 5*** (0.94 g, 97%) as a yellow solid; Kaiser test was negative; Bromophenol Blue test was negative.

### Preparation of Compound 6

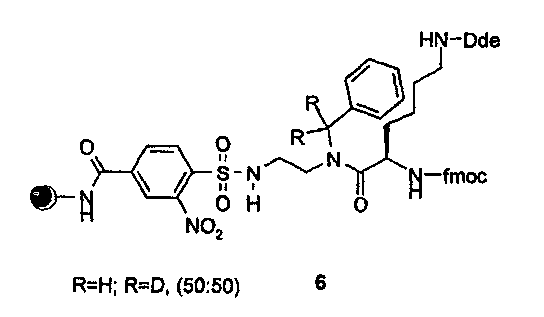

The protected resin 5 (0.85 g, 0.34 mmol) was suspended in dichloromethane (2 ml) and treated with trifluoroacetic acid (2 x 4 ml) for 5 min. Solvents were removed by filtration and the resin washed with 10% DIPEA solution in DMF (3 x 5 ml), dichloromethane (6 x 5 ml), DMF (6 x 5 ml), dichloromethane (6 x 5 ml), ether (2 x 5 ml). The amine resin was then suspended in DMF (4 ml) and dichloromethane (2 ml) FMoc-Lys(Dde)-OH (0.86 g, 1.62 mmol) was added, followed by PyBOP (0.84 g, 1.62 mmol) and HOBT (0.22. g, 1.62 mmol). After 4 min. Hünig's base (0.42 g, 3.24 mmol) was added. The reaction was agitated with nitrogen for 18 h and the resin then washed with dichloromethane (6 x 10 ml), DMF (6 x 10 ml), dichloromethane (6 x 10 ml), other (2 x 10 ml) and dried *in vacuo* to give the ***sulfonamide resin 6*** (0.96 g, 100%) as a yellow solid; Kaiser test was negative; Bromophenol Blue test was negative; Resin loading 99% as estimated by quantitative Fmoc group cleavage.

### Preparation of Compound 7

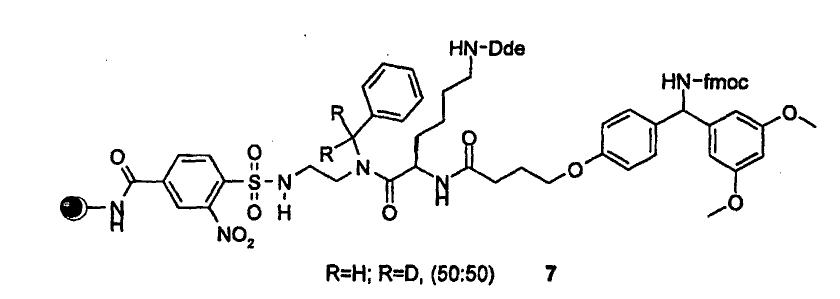

Resin (**6**) (0.93 g, 0.28 mmol) was treated with 20% Piperidine in DMF solution (2 x 5 ml) for 10 min. Solvents were removed by filtration and the resin washed with DMF (6 x 5 ml), dichloromethane (6 x 5 ml), and ether (2 x 4 ml). The resin was suspended in DMF (5 ml) and dichloromethane (5 ml) and the Rink acid **linker** (0.92 g, 1.62 mmol) was added, followed by PyBOP (0.84 g, 1.62 mmol) and HOBT (0.22 g, 1.62 mmol). After 4 min. Hünig's base (0.42g, 3.24 mmol) was added. The reaction was agitated with nitrogen for 3 h and the resin then washed with dichloromethane (6 x 5 ml), DMF (6 x 5 ml), dichloromethane (6 x 5 ml), ether (2 x 3 ml) and dried *in vacuo* to give the ***sulfonamide resin*** 7 (1.02 g, 98%) as a yellow solid; Kaiser test was negative; Bromophenol Blue test was negative; Resin loading 94% as estimated by quantitative Fmoc group cleavage.

### Preparation of Compounds 9a-d and 10a-d

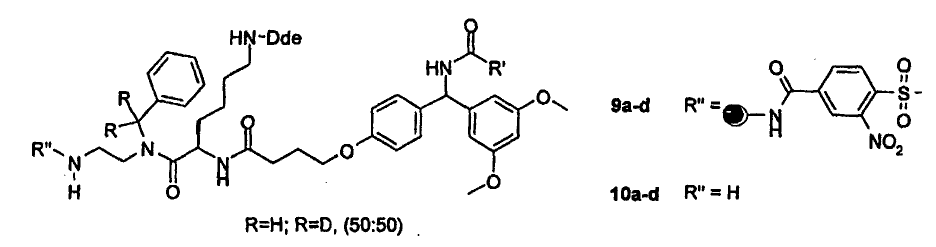

Resin (7) (70 mg, 0.02 mmol) was treated with 20% piperidine in DMF solution (2 x 1 ml) for 10 min. Solvents were removed by filtration and the resin washed with DMF (6 x 1 ml), dichloromethane (6 x 1 ml), ether (2 x 1 ml). The resin was suspended in DMF (0.5 ml) and dichloromethane (0.5 ml) and the appropriate acid (**8a-d**) (0.15 mmol) was added, followed by PyBOP (0.08 g, 0.15 mmol) and HOBT (0.02 g, 0.15 mmol). After 4 min. Hünig's base (0.04 g, 0.30 mmol) was added. The reaction was agitated with nitrogen for 3 h and the resin then washed with dichloromethane (6 x 1 ml), DMF (6 x 1 ml), dichloromethane (6 x 1 ml), ether (2 x 1 ml) and dried *in vacuo* to give the ***sulfonamide resins 9a-d*** as a yellow solid; Kaiser test and Bromophenol Blue test were negative in all four cases.

A small sample of each of the four resins was treated with DBU/Mercaptoethanol in acetonitrile to release ***10a-d*** which were analysed by low resolution mass spectrometry and RP HPLC (Method A):
**10a** (R'= Z-Gly-) MS (Electrospray +ve) m/z 961 and 963 (MH)⁺; HPLC, Rₜ 4.01 min.
**10b** (R'= Boc-Abu-) MS (Electrospray +ve) m/z 955 and 957 (MH)⁺; HPLC, R, 4.09 min.
**10c** (R'= 1-naphthylacetoyl-) MS (Electrospray +ve) m/z 938 and 939 (MH)⁺; HPLC, Rₜ 4.28 min.
**10d** (R'= Boc-Phe-) MS (Electrospray +ve) m/z 1017 and 1019 (MH)⁺; HPLC, Rₜ 4.42 min.

### Preparation of Compounds 11a-d and 12a-d

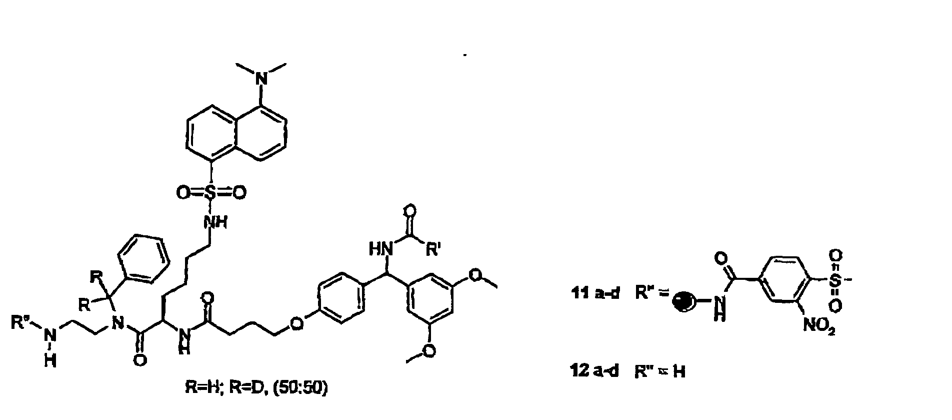

Resin (**9a-d**) (ca. 70 mg, 0.02 mmol) was treated with 10% hydrazine in DMF solution (3 x 1 ml) for 30 min. Solvents were removed by filtration and each resin washed with DMF (6 x 1 ml), dichloromethane (6 x 1 ml), ether (2 x 1 ml). To each resin portion was then added dansyl chloride (40 mg, 0.15 mmol) and triethylamine (15 mg, 0.15 mmol) in dichloromethane (1 ml). After 4 h reaction time the resins were washed with dichloromethane (6 x 1 ml), DMF (6 x 1 ml), dichloromethane (6 x 1 ml), ether (2 x 1 ml) and dried *in vacuo* to give the ***sulfonamide resins 11a-d*** as a yellow solid; Kaiser test and Bromophenol Blue test were positive in all four cases.

A small sample of each of the four resins was treated with DBU/Mercaptoethanol in acetonitrile to release ***12a-d*** which were analysed by low resolution mass spectrometry and RP HPLC (Method B):
**12a** (R'= Z-Gly-) MS (Electrospray +ve) m/z 1030 and 1032 (MH)⁺; HPLC, Rₜ 4.77 min.
**12b** (R'= Boc-Abu-) MS (Electrospray +ve) m/z 1024 and 1026 (MH)⁺; HPLC, Rₜ 4.94 min.
**12c** (R'= 1-naphthylacetoyl-) MS (Electrospray +ve) m/z 1007 and 1009 (MH)⁺; HPLC, Rₜ 5.47 min.
**12d** (R'= Boc-Phe-) MS (Electrospray +ve) m/z 1086 and 1089 (MH)⁺; HPLC, Rₜ 5.82 min.

It can be seen from the foregoing examples that the inclusion of a UV chromophore, and mass spectrometric sensitising group into the constructs enables rapid and simple qualitative and quantitative analysis of the products of a solid phase synthesis to be undertaken by the simple and well established procedures of HPLC in combination with UV detection and mass spectrometry.

## Claims

1. A chemical construct for use in solid phase synthesis comprising a solid support Q having linked thereto via a connecting group Y a substrate R; the cormecting group Y having first and second cleavage sites which are orthogonally and selectively cleavable; the second cleavage site being selectively cleavable to release the substrate; and the first cleavage site being located at a position between the second cleavage site and the solid support and being selectively cleavable to release a fragment Fr comprising the substrate and at least a portion of the connecting group Y; **characterised in that** cleavage of the skeleton of the construct at the first cleavage site forms or introduces on the chemical fragment Fr at the first cleavage site a moiety comprising a sensitising group G which sensitises the chemical fragment Fr to instrumental, e.g. mass spectroscopic, analysis.

2. A chemical construct according to claim 1 wherein the chemical fragment Fr contains a means for imparting a characteristic signature to the mass spectrum of the fragment.

3. A chemical construct according to claim 2 wherein the characteristic signature is provided by incorporating into the fragment Fr a peak splitting isotopic label.

4. A chemical construct according to claim 3 wherein the peak splitting isotopic label is defined one or more isotope pairs selected from ¹H/H² (D), ⁷⁹Br/⁸¹Br, ¹²C/¹³C, ¹⁴N/¹⁵N and ¹⁶O/¹⁸O.

5. A chemical construct according to any one of claims 2 to 4 wherein the means for imparting a characteristic signature to the mass spectrum of the fragment is located between the first and second cleavage sites.

6. A chemical construct according to any one of the preceding claims wherein the first and second cleavage sites cleavage sites are defined by first and second linker groups L¹ and L².

7. A chemical construct according to claim 6 wherein an spacer group A is interposed between the two linker groups L¹ and L², the spacer group A containing means for imparting a characteristic signature to the mass spectrum of the fragment Fr as defined in any one of claims 2 to 5.

8. A chemical construct according to claim 7 wherein the wherein the connecting group Y has the formula L¹-A-L².

9. A chemical intermediate construct according to claim 8 wherein the group A has the general formula NH-Alk-NH-X¹ wherein X¹ is hydrogen or an aralkyl group, and Alk is an alkylene group.

10. A chemical construct according to any one of the preceding claims wherein the sensitising group G is an ionisable group which is ionisable under mass spectrometric conditions.

11. A chemical construct according to claim 10 wherein the group G is ionisable to form a positive ion under mass spectrometric conditions, for example electrospray mass spectrometric conditions.

12. A chemical construct according to any one of the preceding claims wherein the group G is a basic amino group.

13. A chemical construct according to claim 12 wherein the basic amino group is a primary amino group.

14. A chemical construct according to claim 12 wherein the basic amino group is a tertiary amino group.

15. A chemical construct according to claim 14 wherein the tertiary amino group is a cyclic amino group.

16. A chemical construct according to claim 15 wherein the cyclic amino group is N-methylpiperazino.

17. A chemical construct according to claim 12 or claim 13 wherein the basic amino group is derived from the photochemical cleavage of a carbamate group.

18. A chemical construct according to any one of claims 3 to 17 wherein the peak splitting isotopic label is contained within a substituted or unsubstituted alkylene diamine group.

19. A chemical construct according to claim 18 wherein the alkylene diamine group is substituted by an N-benzyl group.

20. A chemical construct according to claim 19 wherein the N-benzyl group has a methylene group which is substituted with the peak splitting atom deuterium.

21. A chemical construct according to any one of the preceding claims wherein the first cleavage site is selectively cleavable by one type of chemistry selected from a group of chemistries consisting of cleavage under acid conditions, base catalysed cleavage, oxidative cleavage, reductive cleavage, nucleophilic displacement, cleavage by 1,2 *bis* nucleophiles, electrophilic displacement, and thermal, photochemical and enzymatic cleavage, and the second cleavage site is selectively cleavable by a different type of chemistry selected from the said group.

22. A chemical construct according to claim 21 wherein the first cleavage site is cleavable by one type of chemistry selected from:
(i) photochemical cleavage, e.g. photochemical cleavage of a nitrobenzylcarbamate group;
(ii) oxidation followed by cleavage through nucleophilic displacement, for example oxidation of a thiopyrimidine followed by nucleophilic displacement by an amine (e.g. a secondary amine such as N-methyl piperazine);
(iii) cleavage of a sulphonamide by nucleophilic displacement, for example by a thiolate nucleophile (e.g. mercaptoethanol I the presence of a strong base such as DBU);
(vi) cleavage of enamine groups (particularly those containing an enamine moiety conjugated to a carbonyl group; e.g. as 1-[4,4-dimethyl-2,6-dioxo-cyclohexylidene]ethyl amino) with a 1,2-*bis* nucleophile such as hydrazine or hydroxylamine or derivatives thereof ; and
(vii) transition metal catalysed cleavage of allyloxycarbonylamino groups, for example palladium (0) catalysed cleavage of allyloxycarbonylamino groups.

23. A chemical construct according to claim 22 wherein the second cleavage site is cleaved under acid conditions or by photolysis.

24. A chemical construct according to claim 21 or claim 22 wherein the first cleavage site is defined by a sulphonamide linker group, and the second cleavage site is optionally defined by a group, such as a Rink linker, which is cleavable under acidic conditions.

25. A chemical construct according to claim 21 or claim 22 wherein the first cleavage site is defined by a thiopyrimidine linker susceptible to cleavage by oxidation followed by nucleophilic displacement, and the second cleavage site is optionally defined by a group, such as a Rink linker, which is cleavable under acidic conditions.

26. A chemical construct according to claim 21 or claim 22 wherein the first cleavage site is defined by a dde group and the second cleavage site is optionally defined by a group, such as a Rink linker, which is cleavable under acidic conditions.

27. A chemical construct according to claim 21 or claim 22 wherein the first cleavage site is cleavable under photochemical conditions and the second cleavage site is defined by a group, such as a Rink linker, which is cleavable under acid conditions.

28. A chemical construct according to claim 21 or claim 22 wherein the first cleavage site is defined by a group such as allyloxycarbonylamino that can be cleaved by a transition metal such as palladium (0), and the second cleavage site is optionally defined by a group, such as a Rink linker, which is cleavable under acidic conditions

29. A chemical construct according to claim 21 or claim 22 wherein the first cleavage site is cleaved by oxidation followed by nucleophilic displacement.

30. A chemical construct according to claim 29 wherein the nucleophile is an amine.

31. A chemical construct according to claim 30 wherein the amine is a cyclic amine such as piperidine.

32. A chemical construct according to any one of the preceding claims wherein the fragment Fr contains a chromophore C^{u} that facilitates analysis of the fragment Fr by ultraviolet, visible or fluorescence spectrophotometry.

33. A chemical construct according to claim 32 wherein the chromophore C^{u} has a principal log Eₘₐₓ value of at least 2.5.

34. A chemical construct according to claim 33 wherein the principal log Eₘₐₓ value is at least 1.5 times greater than the principal log Eₘₐₓ of the substrate R.

35. A chemical construct according to any one of claims 1 to 34, the construct comprising a solid support Q having linked thereto via the connecting group Y the substrate R wherein the fragment Fr comprises the substrate and at least a portion of the connecting group Y, and the said portion contains a chromophore C^{u} which facilitates analysis of the fragment Fr^{u} by ultra violet, visible or fluorescence spectroscopy, the chromophore C^{u} having a principal log Eₘₐₓ value of at least 2.5 and wherein (i) the principal log Eₘₐₓ value is at least 1.5 times greater than the principal log Eₘₐₓ of the substrate R; or (ii), the chromophore C^{u} has an absorption peak at a wavelength remote from absorptions due to the substrate R.

36. A chemical construct according to any one of claims 1 to 35 comprising a solid support Q having linked thereto via the connecting group Y the substrate R wherein the fragment Fr comprises the substrate and at least a portion of the connecting group Y, and the said portion contains a chromophore C^{u} which facilitates analysis of the fragment Fr^{u} by ultra violet, visible or fluorescence spectroscopy, wherein the absorption characteristics of the chromophore C^{u} and the substrate R are such that at a given measurement wavelength, any errors in measurement of the quantity of substrate R (or any fragment or construct containing the fragment) arising from any overlap between absorption bands due to the chromophore and absorption bands due to the substrate R are less than 10%, preferably less than 5%.

37. A chemical construct according to any one of claims 32 to 36 wherein the chromophore is a group containing an aryl group.

38. A chemical construct according to claim 37 wherein the aryl group is a fused polycyclic aryl group, in which one or more ring carbon atoms are optionally replaced by a heteroatom.

39. A chemical construct according to claim 38 wherein the fused polycyclic aryl group is selected from the group consisting of naphthyl, phenanthrenyl and anthracenyl groups.

40. A chemical construct according to claim 39 wherein the fused polycyclic aryl group is an anthracenyl group.

41. A chemical construct according to claim 39 wherein the fused polycyclic aryl group is a dansyl (1-dimethylamino-5-naphthylsulphonyl) group.

42. A method of analysing the constructs of any one of the preceding claims; the method comprising cleaving the construct at the first cleavage site to release the chemical fragment Fr, the cleavage reaction generating on the chemical fragment Fr at the cleavage site a group comprising a mass spectrometric sensitising group G (e.g. a group which is ionisable under mass spectroscopic conditions), and then subjecting the chemical fragment to mass spectrometry, e.g. electrospray mass spectrometry.

43. An intermediate chemical construct for use preparing a chemical construct as defined in any one of the preceding claims, the intermediate construct having the formula Q-Y' wherein Y' is a reactive or protected form of the group Y, and Q and Y are as hereinbefore defined.

44. An intermediate construct of the formula Q-L¹-A^{p} wherein Q and L¹ are as defined in any one of the preceding claims and A^{p} is a reactive or protected form of the spacer group A containing a peak splitting isotopic label.

45. An intermediate construct according to claim 44 having the general formula Q-L¹-NH-Alk-NH-X¹ wherein X¹ is hydrogen or an aralkyl group, and Alk is an alkylene group.

46. A method of analysing the fragment Fr derived from a chemical construct according to any of claims 32 to 41 which comprises subjecting the fragment Fr to ultra violet, visible or fluorescence spectrophotometric analysis to quantify the substrate R.

47. A method of identifying a pharmaceutically useful substrate comprising preparing a library containing a plurality of chemical constructs as defined in any of the preceding claims, and subjecting the library to biological testing to identify biologically active substrates.

48. A method according to claim 47 that includes the further step of formulating a biologically active substrate thus identified with a pharmaceutically acceptable carrier to form a pharmaceutical composition.

## Patentansprüche

1. Chemisches Konstrukt zur Verwendung in der Festphasensynthese, umfassend einen festen Träger Q mit einem daran über eine Verbindungsgruppe Y gebundenen Substrat R; wobei die Verbindungsgruppe Y erste und zweite Spaltungsstellen aufweist, die orthogonal und selektiv abspaltbar sind; wobei die zweite Spaltungsstelle selektiv unter Freisetzung des Substrats abspaltbar ist; und wobei sich die erste Spaltungsstelle an einer Position zwischen der zweiten Spaltungsstelle und dem festen Träger befindet und selektiv unter Freisetzung eines Fragments Fr abspaltbar ist, das das Substrat und wenigstens einen Teil der Verbindungsgruppe Y umfasst; **dadurch gekennzeichnet, dass** die Abspaltung des Gerüsts des Konstrukts an der ersten Spaltungsstelle eine Einheit am chemischen Fragment Fr an der ersten Abspaltungsstelle bildet oder einführt, die eine sensibilisierende Gruppe G umfasst, die das chemische Fragment Fr für die instrumentelle, z.B. massenspektroskopische, Analyse sensibilisiert.

2. Chemisches Konstrukt gemäss Anspruch 1, worin das chemische Fragment Fr ein Mittel enthält, um dem Massenspektrum des Fragments eine charakteristische Signatur zu verleihen.

3. Chemisches Konstrukt gemäss Anspruch 2, worin die charakteristische Signatur bereitgestellt wird, indem eine Peak-spaltende Isotopenmarkierung in das Fragment Fr eingeführt wird.

4. Chemisches Konstrukt gemäss Anspruch 3, worin die Peak-spaltende Isotopenmarkierung durch ein oder mehrere Isotopenpaare definiert ist, die aus ¹H/²H (D), ⁷⁹Br/⁸¹Br, ¹²C/¹³C, ¹⁴N/¹⁵N und ¹⁶O/¹⁸O ausgewählt sind.

5. Chemisches Konstrukt gemäss einem der Ansprüche 2 bis 4, worin sich das Mittel, um dem Massenspektrum des Fragments eine charakteristische Signatur zu verleihen, zwischen der ersten und zweiten Spaltungsstelle befindet.

6. Chemisches Konstrukt gemäss einem der vorhergehenden Ansprüche, worin die ersten und zweiten Spaltungsstellen durch erste und zweite Linker-Gruppen L¹ und L² definiert sind.

7. Chemisches Konstrukt gemäss Anspruch 6, worin eine Spacer-Gruppe A zwischen den zwei Linker-Gruppen L¹ und L² zwischengeschaltet ist, wobei die Spacer-Gruppe A Mittel enthält, um dem Massenspektrum des Fragments Fr eine charakteristische Signatur zu verleihen, wie in einem der Ansprüche 2 bis 5 definiert.

8. Chemisches Konstrukt gemäss Anspruch 7, worin die Verbindungsgruppe Y die Formel L¹-A-L² hat.

9. Chemisches intermediäres Konstrukt gemäss Anspruch 8, worin die Gruppe A die allgemeine Formel NH-Alk-NH-X¹ hat, worin X¹ Wasserstoff oder eine Aralkylgruppe ist und Alk eine Alkylengruppe ist.

10. Chemisches Konstrukt gemäss einem der vorhergehenden Ansprüche, worin die sensibilisierende Gruppe G eine ionisierbare Gruppe ist, die unter massenspektrometrischen Bedingungen ionisierbar ist.

11. Chemisches Konstrukt gemäss Anspruch 10, worin die Gruppe G zur Bildung eines positiven Ions unter massenspektrometrischen Bedingungen ionisierbar ist, z.B. unter Elektrospray-massenspektrometrischen Bedingungen.

12. Chemisches Konstrukt gemäss einem der vorhergehenden Ansprüche, worin die Gruppe G eine basische Aminogruppe ist.

13. Chemisches Konstrukt gemäss Anspruch 12, worin die basische Aminogruppe eine primäre Aminogruppe ist.

14. Chemisches Konstrukt gemäss Anspruch 12, worin die basische Aminogruppe eine tertiäre Aminogruppe ist.

15. Chemisches Konstrukt gemäss Anspruch 14, worin die tertiäre Aminogruppe eine cyclische Aminogruppe ist.

16. Chemisches Konstrukt gemäss Anspruch 15, worin die cyclische Aminogruppe N-Methylpiperazino ist.

17. Chemisches Konstrukt gemäss Anspruch 12 oder 13, worin die basische Aminogruppe aus der photochemischen Spaltung einer Carbamatgruppe stammt.

18. Chemisches Konstrukt gemäss einem der Ansprüche 3 bis 17, worin die Peak-spaltende Isotopenmarkierung innerhalb einer substituierten oder unsubstituierten Alkylendiamingruppe enthalten ist.

19. Chemisches Konstrukt gemäss Anspruch 18, worin die Alkylendiamingruppe mit einer N-Benzylgruppe substituiert ist.

20. Chemisches Konstrukt gemäss Anspruch 19, worin die N-Benzylgruppe eine Methylengruppe hat, die mit dem Peak-spaltenden Atom-Deuterium substituiert ist.

21. Chemisches Konstrukt gemäss einem der vorhergehenden Ansprüche, worin die erste Spaltungsstelle selektiv spaltbar durch einen Typ von Chemie ist, der aus einer Chemiegruppe ausgewählt ist, die aus Spaltung unter sauren Bedingungen, basenkatalysierter Spaltung, oxidativer Spaltung, reduktiver Spaltung, nukleophiler Verdrängung, Spaltung durch 1,2-Bisnukleophile, elektrophiler Verdrängung und thermischer, photochemischer und enzymatischer Spaltung besteht, und worin die zweite Spaltungsstelle durch einen unterschiedlichen Typ von Chemie selektiv spaltbar ist, ausgewählt aus dieser Gruppe.

22. Chemisches Konstrukt gemäss Anspruch 21, worin die erste Spaltungsstelle durch einen Typ von Chemie spaltbar ist, ausgewählt aus:
(i) photochemischer Spaltung, z.B. photochemischer Spaltung einer Nitrobenzylcarbamatgruppe;
(ii) Oxidation, gefolgt von Spaltung durch nukleophile Verdrängung, z.B. Oxidation eines Thiopyrimidins, gefolgt von nukleophiler Verdrängung mit einem Amin (z.B. mit einem sekundären Amin wie N-Methylpiperazin) ;
(iii) Spaltung eines Sulfonamids durch nukleophile Verdrängung, z.B. durch ein Thiolatnukleophil (z.B. Mercaptoethanol in Gegenwart einer starken Base wie DBU) ;
(vi) Spaltung von Enamingruppen (insbesondere denjenigen, die eine Enamineinheit enthalten, die mit einer Carbonylgruppe konjugiert ist; wie z.B. 1-[4,4-Dimethyl-2,6-dioxocyclohexyliden] ethylamino) mit einem 1,2-Bis-nukleophil wie Hydrazin oder Hydroxylamin oder Derivaten davon; und
(vii) Übergangsmetall-katalysierte Spaltung von Allyloxycarbonylaminogruppen, z.B. Palladium(0)-katalysierte Spaltung von Allyloxycarbonylaminogruppen.

23. Chemisches Konstrukt gemäss Anspruch 22, worin die zweite Spaltungsstelle unter sauren Bedingungen oder durch Photolyse gespaltet wird.

24. Chemisches Konstrukt gemäss Anspruch 21 oder 22, worin die erste Spaltungsstelle durch eine Sulfonamid-Linker-Gruppe definiert ist und die zweite Spaltungsstelle gegebenenfalls durch eine Gruppe wie einen Ring-Linker definiert ist, die unter sauren Bedingungen spaltbar ist.

25. Chemisches Konstrukt gemäss Anspruch 21 oder 22, worin die erste Spaltungsstelle durch einen Thiopyrimidin-Linker definiert ist, der für Spaltung durch Oxidation, gefolgt von nukleophiler Verdrängung empfindlich ist, und worin die zweite Spaltungsstelle gegebenenfalls durch eine Gruppe wie einen Ring-Linker definiert ist, die unter sauren Bedingungen spaltbar ist.

26. Chemisches Konstrukt gemäss Anspruch 21 oder 22, worin die erste Spaltungsstelle durch eine dde-Gruppe definiert ist und die zweite Spaltungsstelle gegebenenfalls durch eine Gruppe wie einen Ring-Linker definiert ist, die unter sauren Bedingungen spaltbar ist.

27. Chemisches Konstrukt gemäss Anspruch 21 oder 22, worin die erste Spaltungsstelle unter photochemischen Bedingungen spaltbar ist und die zweite Spaltungsstelle durch eine Gruppe wie einen Ring-Linker definiert ist, die unter sauren Bedingungen spaltbar ist.

28. Chemisches Konstrukt gemäss Anspruch 21 oder 22, worin die erste Spaltungsstelle durch eine Gruppe, wie Allyloxycarbonylamino, definiert ist, die durch ein Übergangsmetall wie Palladium(0) gespalten werden kann, und worin die zweite Spaltungsstelle gegebenenfalls durch eine Gruppe wie einen Ring-Linker definiert ist, die unter sauren Bedingungen spaltbar ist.

29. Chemisches Konstrukt gemäss Anspruch 21 oder 22, worin die erste Spaltungsstelle durch Oxidation, gefolgt von nukleophiler Verdrängung gespalten wird.

30. Chemisches Konstrukt gemäss Anspruch 29, worin das Nukleophil ein Amin ist.

31. Chemisches Konstrukt gemäss Anspruch 30, worin das Amin ein cyclisches Amin wie Piperidin ist.

32. Chemisches Konstrukt gemäss einem der vorhergehenden Ansprüche, worin das Fragment Fr einen Chromophor C^{u} enthält, der die Analyse des Fragments Fr durch Ultraviolett-, sichtbare oder Fluoreszenz-Spektrophotometrie erleichtert.

33. Chemisches Konstrukt gemäss Anspruch 32, worin der Chromophor C^{u} einen log Eₘₐₓ-Hauptwert von wenigstens 2,5 hat.

34. Chemisches Konstrukt gemäss Anspruch 33, worin der log Eₘₐₓ-Hauptwert wenigstens 1,5 mal grösser als der log Eₘₐₓ-Hauptwert des Substrats R ist.

35. Chemisches Konstrukt gemäss einem der Ansprüche 1 bis 34, wobei das Konstrukt einen festen Träger Q mit dem daran über die Verbindungsgruppe Y gebundenen Substrat R umfasst, worin das Fragment Fr das Substrat und wenigstens einen Teil der Verbindungsgruppe Y umfasst, und worin der Teil einen Chromophor C^{u} enthält, der die Analyse des Fragments Fr durch Ultraviolett-, sichtbare oder Fluoreszenz-Spektroskopie erleichtert, wobei der Chromophor C^{u} einen log Eₘₐₓ-Hauptwert von wenigstens 2,5 hat und worin (i) der log Eₘₐₓ-Hauptwert wenigstens 1,5 mal grösser als der log Eₘₐₓ-Hauptwert des Substrats R ist; oder (ii) der Chromophor C^{u} einen Absorptionspeak bei einer von Absorptionen aufgrund des Substrats R entfernten Wellenlänge hat.

36. Chemisches Konstrukt gemäss einem der Ansprüche 1 bis 35, das einen festen Träger Q mit dem daran über die Verbindungsgruppe Y gebundenen Substrat R umfasst, worin das Fragment Fr das Substrat und wenigstens einen Teil der Verbindungsgruppe Y umfasst, und wobei der Teil einen Chromophor C^{u} enthält, der die Analyse des Fragments Fr durch Ultraviolett-, sichtbare oder Fluoreszenz-Spektroskopie erleichtert, worin die Absorptionscharakteristiken des Chromophors C^{u} und des Substrats R derart sind, dass bei einer gegebenen Messwellenlänge etwaige Fehler in der Messung der Menge des Substrats R (oder jedes Fragments oder Konstrukts, das das Fragment enthält), die aus einer Überlappung zwischen Absorptionsbanden aufgrund des Chromophors und Absorptionsbanden aufgrund des Substrats R entstehen, geringer als 10 % sind, bevorzugt geringer als 5 %.

37. Chemisches Konstrukt gemäss einem der Ansprüche 32 bis 36, worin der Chromophor eine Gruppe ist, die eine Arylgruppe enthält.

38. Chemisches Konstrukt gemäss Anspruch 37, worin die Arylgruppe eine anellierte polycyclische Arylgruppe ist, in der ein oder mehrere Ringkohlenstoffatome gegebenenfalls durch Heteroatome ersetzt sind.

39. Chemisches Konstrukt gemäss Anspruch 38, worin die anellierte polycyclische Arylgruppe aus der Gruppe ausgewählt ist, die aus Naphthyl-, Phenanthrenyl- und Anthracenylgruppen besteht.

40. Chemisches Konstrukt gemäss Anspruch 39, worin die anellierte polycyclische Arylgruppe eine Anthracenylgruppe ist.

41. Chemisches Konstrukt gemäss Anspruch 39, worin die anellierte polycyclische Arylgruppe eine Dansylgruppe (1-Dimethylamino-5-naphthylsulfonyl) ist.

42. Verfahren zur Analyse der Konstrukte gemäss einem der vorhergehenden Ansprüche, wobei das Verfahren das Spalten des Konstrukts an der ersten Spaltungsstelle zur Freisetzung des chemischen Fragments Fr, wobei die Spaltungsreaktion am chemischen Fragment Fr an der Spaltungsstelle eine Gruppe erzeugt, die eine massenspektrometrisch sensibilisierende Gruppe G umfasst (z.B. eine Gruppe, die unter massenspektroskopischen Bedingungen ionisierbar ist), und anschliessend das Unterwerfen des chemischen Fragments der Massenspektrometrie, z.B. Elektrospray-Massenspektrometrie, umfasst.

43. Intermediäres chemisches Konstrukt zur Verwendung in der Herstellung eines chemischen Konstrukts wie in einem der vorhergehenden Ansprüche definiert, worin das intermediäre Konstrukt die Formel Q-Y' hat, worin Y' eine reaktive oder geschützte Form der Gruppe Y ist und Q und Y wie hier zuvor definiert sind.

44. Intermediäres Konstrukt der Formel Q-L¹-AP, worin Q und L¹ wie in einem der vorhergehenden Ansprüche definiert sind und AP eine reaktive oder geschützte Form der Spacer-Gruppe A ist, die eine Peak-spaltende Isotopenmarkierung enthält.

45. Intermediäres Konstrukt gemäss Anspruch 44 mit der allgemeinen Formel Q-L¹-NH-Alk-NH-X¹, worin X¹ Wasserstoff oder eine Aralkylgruppe ist und Alk eine Alkylengruppe ist.

46. Verfahren zum Analysieren des Fragments Fr, das aus einem chemischen Konstrukt gemäss einem der Ansprüche 32 bis 41 stammt, welches das Unterwerfen des Fragments Fr der Ultraviolett-, sichtbaren und Fluoreszenz-spektrophotometrischen Analyse zur Quantifizierung des Substrats R umfasst.

47. Verfahren zur Identifizierung eines pharmazeutisch nützlichen Substrats, welches das Herstellen einer Bibliothek, die eine Mehrzahl von chemischen Konstrukten wie in einem der vorhergehenden Ansprüche definiert enthält, und das Unterwerfen der Bibliothek einer biologischen Untersuchung zur Identifizierung biologisch aktiver Substrate umfasst.

48. Verfahren gemäss Anspruch 47, welches den weiteren Schritt der Formulierung eines so identifizierten, biologisch aktiven Substrats mit einem pharmazeutisch akzeptablen Träger zur Bildung einer pharmazeutischen Zusammensetzung einschliesst.

## Revendications

1. Construction chimique utile dans une synthèse en phase solide comprenant un support solide Q auquel est lié un substrat R par l'intermédiaire d'un groupe de raccordement Y ; le groupe de raccordement Y ayant un premier site et un second site de clivage qui peuvent être clivés de façon orthogonale et sélective ; le second site de clivage pouvant être clivé de façon sélective pour libérer le substrat ; et le premier site de clivage étant situé en une position entre le second site de clivage et le support solide et pouvant être clivé de façon sélective pour libérer un fragment Fr comprenant le substrat et au moins une partie du groupe de raccordement Y, **caractérisé en ce que** le clivage du squelette de la construction au niveau du premier site de clivage forme ou introduit sur le fragment chimique Fr au niveau du premier site de clivage une partie comprenant un groupe sensibilisant G qui sensibilise le fragment chimique Fr à une analyse instrumentale, par exemple une spectroscopie de masse.

2. Construction chimique selon la revendication 1, dans laquelle le fragment chimique Fr contient un moyen pour conférer une signature caractéristique au spectre de masse du fragment.

3. Construction chimique selon la revendication 2, dans laquelle la signature caractéristique est fournie par incorporation dans le fragment Fr d'un marqueur isotopique de fractionnement des pics.

4. Construction chimique selon la revendication 3, dans laquelle le marqueur isotopique de fractionnement des pics est défini par une ou plusieurs paires d'isotopes choisies parmi ¹H/²H (D), ⁷⁹Br/⁸¹Br, ¹²C/¹³C, ¹⁴N/¹⁵N et ¹⁶O/¹⁸O.

5. Construction chimique selon l'une quelconque des revendications 2 à 4, dans laquelle le moyen pour conférer une signature caractéristique au spectre de masse du fragment est situé entre le premier site et le second site de clivage.

6. Construction chimique selon l'une quelconque des revendications précédentes, dans laquelle le premier site et le second site de clivage sont définis par les premier et second groupes de liaison L¹ et L².

7. Construction chimique selon la revendication 6, dans laquelle un groupe espaceur A est intercalé entre les deux groupes de liaison L¹ et L², le groupe espaceur A contenant un moyen pour conférer une signature caractéristique au spectre de masse du fragment Fr tel que défini dans l'une quelconque des revendications 2 à 5.

8. Construction chimique selon la revendication 7, dans laquelle le groupe de raccordement Y a la formule L¹-A-L².

9. Construction chimique selon la revendication 8, dans laquelle le groupe A a la formule générale NH-Alk-NH-X¹ dans laquelle X¹ est un atome d'hydrogène ou un groupe aralkyle et Alk est un groupe alkylène.

10. Construction chimique selon l'une quelconque des revendications précédentes, dans laquelle le groupe sensibilisant G est un groupe ionisable qui est ionisable dans des conditions de spectrométrie de masse.

11. Construction chimique selon la revendication 10, dans laquelle le groupe G est ionisable pour former un ion positif dans les conditions de spectrométrie de masse, par exemple, les conditions de spectrométrie de masse en mode électrospray.

12. Construction chimique selon l'une quelconque des revendications précédentes, dans laquelle le groupe G est un groupe amino basique.

13. Construction chimique selon la revendication 12, dans laquelle le groupe amino basique est un groupe amino primaire.

14. Construction chimique selon la revendication 12, dans laquelle le groupe amino basique est un groupe amino tertiaire.

15. Construction chimique selon la revendication 14, dans laquelle le groupe amino tertiaire est un groupe amino cyclique.

16. Construction chimique selon la revendication 15, dans laquelle le groupe amino cyclique est un groupe N-méthylpipérazino.

17. Construction chimique selon la revendication 12 ou la revendication 13, dans laquelle le groupe amino basique provient du clivage photochimique d'un groupe carbamate.

18. Construction chimique selon l'une quelconque des revendications 3 à 17, dans laquelle le marqueur isotopique de fractionnement des pics est contenu à l'interieur d'un groupe alkylène diamine substitué ou non substitué.

19. Construction chimique selon la revendication 18, dans laquelle le groupe alkylène diamine est substitué par un groupe N-benzyle.

20. Construction chimique selon la revendication 19, dans laquelle le groupe N-benzyle a un groupe méthylène qui est substitué par l'atome de deutérium de fractionnement des pics.

21. Construction chimique selon l'une quelconque des revendications précédentes, dans laquelle le premier site de clivage peut être clivé de façon sélective par un type de réaction chimique choisi dans un groupe de réactions chimiques consistant en clivage dans des conditions acides, clivage catalysé par une base, clivage oxydant, clivage réducteur, déplacement nucléophile, clivage par des 1,2-bis-nucléophiles, déplacement électrophile et clivage thermique, photochimique et enzymatique, et le second site de clivage peut être clivé de façon sélective par un type de réaction chimique différent choisi dans ledit groupe.

22. Construction chimique selon la revendication 21, dans laquelle le premier site de clivage peut être clivé par un type de réaction chimique choisi parmi :
(i) un clivage photochimique, par exemple le clivage photochimique d'un groupe nitrobenzylcarbamate ;
(ii) une oxydation suivie d'un clivage par déplacement nucléophile, par exemple, l'oxydation d'une thiopyrimidine suivie d'un déplacement nucléophile par une amine (par exemple, une amine secondaire comme la N-méthyl-pipérazine) ;
(iii) un clivage d'un sulfonamide par déplacement nucléophile, par exemple par un nucléophile thiolate (par exemple, le mercaptoéthanol en présence d'une base forte comme le DBU) ;
(iv) un clivage de groupes ènamine (en particulier ceux qui contiennent une partie ènamine conjuguée à un groupe carbonyle ; par exemple comme le 1-[4,4-diméthyl-2,6-dioxocyclohexylidène]-éthylamino) avec un 1,2-bis-nucléophile comme l'hydrazine ou l'hydroxylamine ou leurs dérivés ; et
(v) un clivage catalysé par un métal de transition de groupes allyloxycarbonylamino, par exemple, le clivage catalysé par du palladium (0) de groupes allyloxycarbonylamino.

23. Construction chimique selon la revendication 22, dans laquelle le second site de clivage est clivé dans des conditions acides ou par photolyse.

24. Construction chimique selon la revendication 21 ou la revendication 22, dans laquelle le premier site de clivage est défini par un groupe de liaison sulfonamide, et le second site de clivage est éventuellement défini par un groupe, comme un groupe de liaison Rink, qui peut être clivé dans des conditions acides.

25. Construction chimique selon la revendication 21 ou la revendication 22, dans laquelle le premier site de clivage est défini par un groupe de liaison thiopyrimidine sensible à un clivage par oxydation suivi d'un déplacement nucléophile, et le second site de clivage est éventuellement défini par un groupe, comme un groupe de liaison Rink, qui peut être clivé dans des conditions acides.

26. Construction chimique selon la revendication 21 ou la revendication 22, dans laquelle le premier site de clivage est défini par un groupe 1-[4,4-diméthyl-2,6-dioxocyclohexylidène]-éthyle (dde) et le second site de clivage est éventuellement défini par un groupe, comme un groupe de liaison Rink, qui peut être clivé dans des conditions acides.

27. Construction chimique selon la revendication 21 ou la revendication 22, dans laquelle le premier site de clivage peut être clivé dans des conditions photochimiques et le second site clivage est défini par un groupe, comme un groupe de liaison Rink, qui peut être clivé dans des conditions acides.

28. Construction chimique selon la revendication 21 ou la revendication 22, dans laquelle le premier site de clivage est défini par un groupe comme un groupe allyloxycarbonylamino qui peut être clivé par un métal de transition comme le palladium (0) et le second site de clivage est éventuellement défini par un groupe, comme un groupe de liaison Rink, qui peut être clivé dans des conditions acides.

29. Construction chimique selon la revendication 21 ou la revendication 22, dans laquelle le premier site de clivage est clivé par oxydation suivie d'un déplacement nucléophile.

30. Construction chimique suivant la revendication 29, dans laquelle le nucléophile est une amine.

31. Construction chimique selon la revendication 30, dans laquelle l'amine est une amine cyclique comme la pipéridine.

32. Construction chimique selon l'une quelconque des revendications précédentes, dans laquelle le fragment Fr contient un chromophore C^{u} qui facilite l'analyse du fragment Fr par spectrophotométrie ultraviolette, visible ou de fluorescence.

33. Construction chimique selon la revendication 32, dans laquelle le chromophore C^{u} a une valeur log Emax principal d'au moins 2,5.

34. Construction chimique selon la revendication 33, dans laquelle la valeur log Emax principal est au moins 1,5 fois supérieure au log Emax principal du substrat R.

35. Construction chimique selon l'une quelconque des revendications 1 à 34, la construction comprenant un support solide Q auquel est lié le substrat R par l'intermédiaire du groupe de raccordement Y dans laquelle le fragment Fr comprend le substrat et au moins une partie du groupe de raccordement Y, et ladite partie contient un chromophore C^{u} qui facilite l'analyse du fragment Fr^{u} par spectroscopie ultraviolette, visible ou de fluorescence, le chromophore C^{u} ayant une valeur de log Emax principal d'au moins 2,5 et dans laquelle (i) la valeur de log Emax principar est au moins 1,5 fois supérieure au log Emax principal du substrat R ; ou bien (ii) le chromophore C^{u} a un pic d'absorption à une longueur d'onde éloignée des absorptions dues au substrat R.

36. Construction chimique selon l'une quelconque des revendications 1 à 35, comprenant un support solide Q auquel est lié le substrat R par l'intermédiaire du groupe de raccordement Y, dans laquelle le fragment Fr comprend le substrat et au moins une partie du groupe de raccordement Y, et ladite partie contient un chromophore C^{u} qui facilite l'analyse du fragment Fr^{u} par spectroscopie ultraviolette, visible ou de fluorescence, où les caractéristiques d'absorption du chromophore C^{u} et du substrat R sont telles qu'à une longueur d'onde de mesure donnée, toutes erreurs dans la mesure de la quantité de substrat R (ou de tout fragment ou construction contenant le fragment) provenant d'un quelconque chevauchement entre les bandes d'absorption dues au chromophore et les bandes d'absorption dues au substrat R sont inférieures à 10%, de préférence inférieures à 5%.

37. Construction chimique selon l'une quelconque des revendications 32 à 36, dans laquelle le chromophore est un groupe contenant un groupe aryle.

38. Construction chimique selon la revendication 37, dans laquelle le groupe aryle est un groupe aryle polycyclique condensé, dans lequel un ou plusieurs atomes de carbone de cycle sont éventuellement remplacés par un hétéroatome.

39. Construction chimique selon la revendication 38, dans laquelle le groupe aryle polycyclique condensé est choisi dans le groupe consistant en groupes naphtyle, phénanthrényle et anthracényle.

40. Construction chimique selon la revendication 39, dans laquelle le groupe aryle polycyclique condensé est un groupe anthracényle.

41. Construction chimique selon la revendication 39, dans laquelle le groupe aryle poiycyclique condensé est un groupe dansyle (1-diméthylamino-5-naphtylsulfonyle)

42. Procédé pour l'analyse des constructions selon l'une quelconque des revendications précédentes, le procédé comprenant le clivage de la construction au premier site de clivage pour libérer le fragment chimique Fr, la réaction de clivage générant sur le fragment chimique Fr au niveau du site de clivage un groupe comprenant un groupe G sensibilisant pour spectrométrie de masse, (par exemple un groupe qui est ionisable dans des conditions de spectrométrie de masse) et ensuite la soumission du fragment chimique à une spectrométrie de masse, par exemple, une spectrométrie de masse en mode électrospray.

43. Construction chimique intermédiaire utile pour préparer une construction chimique selon l'une quelconque des revendications précédentes, la construction intermédiaire ayant la formule Q-Y' dans laquelle Y' est une forme réactive ou protégée du groupe Y, et Q et Y sont tels que définis plus haut.

44. Construction intermédiaire de formule Q-L¹-A^{p} dans laquelle Q et L¹ sont tels que définis dans l'une quelconque des revendications précédentes et A^{p} est une forme réactive ou protégée du groupe espaceur A contenant un marqueur isotopique de fractionnement des pics.

45. Construction intermédiaire selon la revendication 44 ayant la formule générale Q-L¹-NH-Alk-NH-X¹, dans laquelle X¹ est un atome d'hydrogène ou un groupe aralkyle, et Alk est un groupe alkylène.

46. Procédé pour analyser le fragment Fr provenant d'une construction chimique selon l'une quelconque des revendications 32 à 41, qui comprend la soumission du fragment Fr à une analyse spectrophotométrique ultraviolette, visible ou de fluorescence pour quantifier le substrat R.

47. Procédé pour identifier un substrat utile du point de vue pharmaceutique comprenant la préparation d'une librairie contenant une pluralité de constructions chimiques tel que défini dans l'une quelconque des revendications précédentes, et la soumission de la librairie à une évaluation biologique pour identifier des substrats actifs du point de vue biologique.

48. Procédé selon la revendication 47, qui comprend l'étape supplémentaire de formulation d'un substrat actif du point de vue biologique ainsi identifié avec un support acceptable du point de vue pharmaceutique pour former une composition pharmaceutique.
